# EUROPÄISCHE PATENTSCHRIFT

(11) **EP 2 563 847 B1**
(45) Veröffentlichungstag und Bekanntmachung des Hinweises auf die Patenterteilung: **13.04.2016**
(21) Anmeldenummer: 11717486.2
(22) Anmeldetag: 14.04.2011
(51) Int. Cl.: C07K 5/02, C07K 1/06, C07K 14/00, C07K 5/062, C08G 83/00

(54) **MCR-DENDRIMERE**
MCR DENDRIMERS
DENDRIMÈRES MCR

(30) Priorität: 30.04.2010 DE 102010018882
(43) Veröffentlichungstag der Anmeldung: 06.03.2013
(73) Patentinhaber: Leibniz-Institut für Pflanzenbiochemie (IPB), 06120 Halle (Saale) (DE)
(72) Erfinder: WESSJOHANN, Ludger, A., 06120 Halle (Saale) (DE); HENZE, Michael, 79115 Freiburg im Breisgau (DE); KREYE, Oliver, 75203 Königsbach (DE); RIVERA, Daniel, Garcia, Ciuadad Habana 10300 (CU)
(74) Vertreter: Müller-Boré & Partner Patentanwälte PartG mbB
(86) Internationale Anmeldenummer: PCT/EP2011/001905
(87) Internationale Veröffentlichungsnummer: WO 2011/134607

(56) Entgegenhaltungen:
- Oliver Kreye: "Zyklisierende und verzweigte mehrfache Ugi-Reaktionen", , 2009, XP002650047, Gefunden im Internet: URL:http://nbn-resolving.de/urn:nbn:de:gbv :3:4-2618 [gefunden am 2011-07-12]
- DIGHE ET AL.: "Convergent synthesis: A strategy to synthesize compounds of biological interest", PHARMACIA LETTRE, Bd. 2, Nr. 1, 2010, XP002650048, ISSN: 0974-248X
- OLEG LUKIN ET AL: "Designer Dendrimers: Branched Oligosulfonimides with Controllable Molecular Architectures", JOURNAL OF THE AMERICAN CHEMICAL SOCIETY, Bd. 128, Nr. 27, 1. Juli 2006 (2006-07-01) , Seiten 8964-8974, XP55002741, ISSN: 0002-7863, DOI: 10.1021/ja061606b
- IZABELA BURY ET AL: "Interfacial Behavior of a Series of Amphiphilic Block Co-dendrimers", LANGMUIR, Bd. 23, Nr. 2, 1. Januar 2007 (2007-01-01) , Seiten 619-625, XP55002743, ISSN: 0743-7463, DOI: 10.1021/la062066z
- DANIEL G. RIVERA ET AL: "Architectural Chemistry: Synthesis of Topologically Diverse Macromulticycles by Sequential Multiple Multicomponent Macrocyclizations", JOURNAL OF THE AMERICAN CHEMICAL SOCIETY, Bd. 131, Nr. 10, 18. März 2009 (2009-03-18), Seiten 3721-3732, XP55002746, ISSN: 0002-7863, DOI: 10.1021/ja809005k
- CHAO WANG ET AL: 'PEPTIDE DENDRIMERS', [Online] 28 Februar 2005, XP055159723 Gefunden im Internet: <URL:http://www.chemistry.illinois.edu/rese arch/organic/seminar_extracts/2004_2005/6_W ang_Abstract_SP05.pdf>
- DIAZ-MOCHON ET AL: "Peptoid dendrimers-microwave-assisted solid-phase synthesis and transfection agent evaluation", TETRAHEDRON LETTERS, PERGAMON, GB, vol. 49, no. 5, 21 December 2007 (2007-12-21), pages 923-926, XP022413177, ISSN: 0040-4039, DOI: 10.1016/J.TETLET.2007.11.122

## Beschreibung

Die vorliegende Erfindung betrifft ein Verfahren zur Herstellung von peptoidischen, peptidischen und chimären peptid-peptoidischen Dendrimeren durch multiple iterative Mehrkomponentenreaktionen (engl. multicomponent reaction = MCR), d.h. UGI- bzw. PASSERINI- Mehrkomponentenreaktionen.

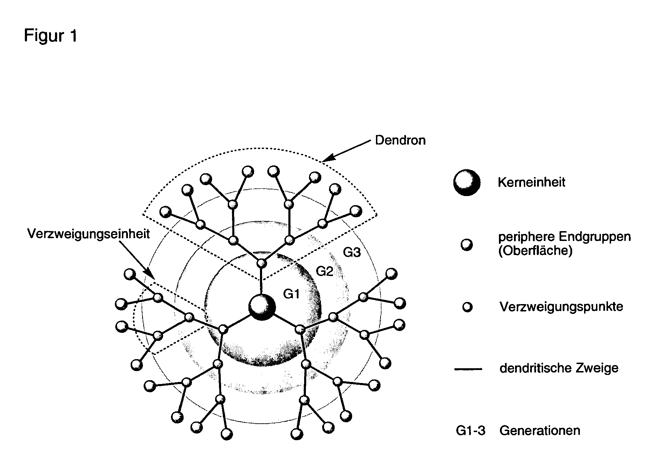

Unter Dendrimeren versteht man supramolekulare, verzweigte Architekturen mit wohl definiertem Molekulargewicht. Ausgehend von multifunktionellen Kerneinheiten verzweigen sich diese Moleküle von innen nach außen zu mehr oder weniger regelmäßigen dreidimensionalen Schalen, deren periphere Endgruppen die Oberfläche bilden (vgl. Figur 1). Mit einem großen Grad an molekularer Uniformität, niedriger Polydispersität und Eigenschaften, welche sie insbesondere zu attraktiven Materialien für die Entwicklung in der Nanomedizin machen, sind Dendrimere in vielerlei Hinsicht hochinteressant. Die hohe Vielfalt und Molekülgrößen, die mit dieser Methode erreicht werden können, übertreffen bisher bekannte Techniken und deren Möglichkeiten. Die Dendrimere zeigen Potential bezüglich des Einsatzes im Targeting z.B. als Kontrastmittel, Coating, Drug Display, Drug Delivery, Light-Harvesting bzw. Energietransfer, und zum Einsatz in Nanobiosystemen.

Die gebräuchlichste und einfachste Methode zum Aufbau von Dendrimeren erfolgt über sogenannte divergente Synthesemethoden. Man bezeichnet damit die Aufbaustrategie, die bei der Kerneinheit beginnt und das Dendrimer in einer "von innen nach außen" durchgeführten Synthese erzeugt. Divergente Synthesen sind in der Regel einfach durchzuführen und werden deshalb bevorzugt angewendet. Allerdings ist ein großer Nachteil, dass in höheren Generationen aufgrund sterischer Hinderungen oft keine vollständigen Reaktionen mehr möglich sind und es zur Bildung sogenannter "Fehlstellen" kommt. Die erhaltenen Produktgemische lassen sich dann chromatographisch schwer aufreinigen. Daneben sind konvergente Syntheseverfahren bekannt, bei der die Dendrimere "von außen nach innen" aufgebaut werden. Obwohl konvergente Synthesen weniger verbreitet sind als divergente, hat diese Strategie gewisse Vorteile. Das Entstehen von "Fehlstellen" wird minimiert und die erzeugten Dendrons lassen sich leichter aufreinigen. Ein Nachteil besteht allerdings in der Anbindung der Dendrons an Kerneinheiten, die in vielen Fällen aufgrund sterischer Hinderung erfolglos ist.

Neben divergenten und konvergenten Synthesen existieren auch neuere Methoden, wie die orthogonale Synthese, die konvergente Zweistufenmethode, die doppeltexponentielle Methode, die Festphasensynthese sowie die koordinationschemische Synthese.

Ein Nachteil aller genannter Synthesen ist die geringe Variationsmöglichkeit im Aufbau der Dendrimere, da die verwendeten Reagenzien, wie z. B. Acrylnitril, Acrylsäureester und Dihydroxybenzylalkohole, aufgrund ihres chemischen Reaktionsverhaltens nicht in der Grundstruktur verändert werden können.

Basierend auf diesen monotonen bzw. problematischen Aufbaustrategien liegt der vorliegenden Erfindung die Aufgabe zugrunde, designbare, hochvariable und flexible Dendrimere durch Mehrkomponentenreaktionen zu synthetisieren, die durch Erzeugung hoher Diversität erhebliche Vorteile gegenüber den erwähnten Standardmethoden aufweisen.

Diese Aufgabe wird durch die in den Ansprüchen gekennzeichneten Ausführungsformen gelöst.

Insbesondere wird gemäß der vorliegenden Erfindung ein Verfahren zur Herstellung von chimären peptid-peptoidischen Dendrimeren, einschließend peptoide, peptidische und depsipeptoidische Dendrimere, durch multiple iterative Mehrkomponentenreaktionen, d.h. UGI- bzw. PASSERINI-Mehrkomponentenreaktionen, gemäß Anspruch 1 bereitgestellt.

Das Verfahren gemäß der vorliegenden Erfindung umfasst das Umsetzen einer polyfunktionalisierten Kerneinheit mit zwei bis sechs weiteren Komponenten, die jeweils verschiedene Funktionalitäten mit unterschiedlichen Reaktivitäten bzw. Schutzgruppen aufweisen, wobei diese Mehrkomponentenreaktion über reaktive Verzweigungen der 3 bis 7 Komponenten zu einer verzweigten Verbindung führt, das Aktivieren der weniger reaktiven Funktionalitäten bzw. das Entschützen der Schutzgruppen, um eine funktionalisierte erste Generation eines verzweigten Dendrimeren zu generieren,
das Umsetzen der funktionalisierten ersten Generation des verzweigten Dendrimeren mit weiteren Komponenten, die jeweils verschiedene Funktionalitäten mit unterschiedlichen Reaktivitäten bzw. Schutzgruppen aufweisen, wobei diese Mehrkomponentenreaktion über reaktive Verzweigungen der 3 bis 7 Komponenten zu einer nächsten verzweigten Verbindung führt, und
das iterative Wiederholen der vorgenannten Schritte, um peptid-peptoidische Dendrimere höherer Generationen zu erhalten,
wobei UGI- und/oder PASSERINI-Mehrkomponentenreaktionen angewendet werden, worin die polyfunktionalisierte Kerneinheit UGI-reaktive Funktionalitäten aufweist und mit bifunktionellen Komponenten, die jeweils eine erste UGI-reaktive Funktionalität und eine zweite terminale UGI-reaktive Funktionalität, welche in geschützter Form vorliegt (PURG, engl. protected UGI reactive group), aufweisen, in einer UGI- und/oder PASSERINI-Mehrkomponentenreaktion umgesetzt wird, wobei sich die zweite geschützte UGI-reaktive Funktionalität nach erfolgter Umsetzung durch Entschützen der PURG's wieder zu UGI-reaktiven Gruppen aktivieren lässt.

UGI-Mehrkomponentenreaktionen (im Folgenden als UGI-4CR abgekürzt) basieren auf Vier-Komponentenreaktionen von Bausteinen, die jeweils eine UGI-reaktive Funktionalität (URG, engl. UGI reactive group) aus reaktiven Gruppierungen wie Isonitrilen, Carbonsäuren, primären Aminen oder Oxogruppen, wie Aldehydgruppen oder Ketogruppen, aufweisen und N-verzweigte Dipeptid (Peptid-Peptoid)-Einheiten generieren. Im Falle von PASSERINI-Reaktionen wird die Aminokomponente weggelassen, wobei in einer Drei-Komponentenreaktion eine Depsipeptid-Einheit entsteht. Im Sinne der vorliegenden Erfindung schließen UGI-4CR's PASSERINI-Reaktionen mit ein.

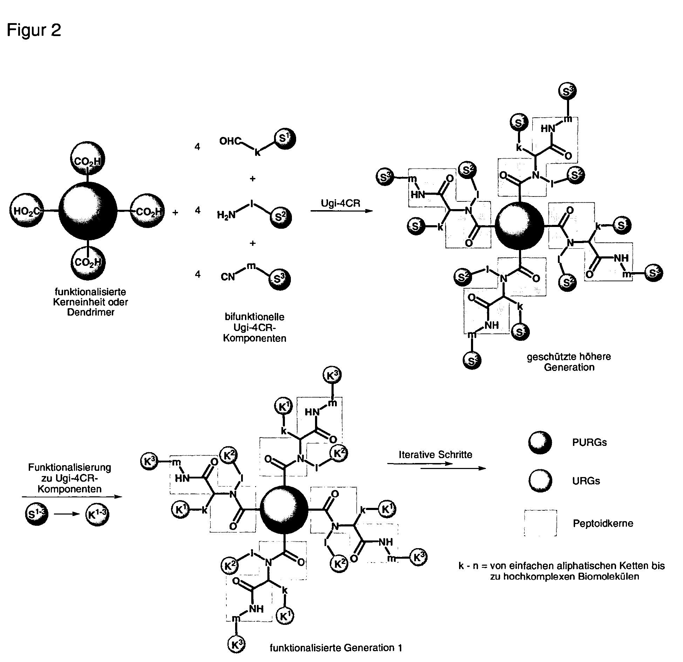

Das erfindungsgemäße Verfahren ist vereinfacht in Figur 2 dargestellt, worin exemplarisch eine Tetracarbonsäure als Kerneinheit eine vierfache UGI-4CR mit bifunktionellen Isonitrilen, primären Aminen und Aldehyden eingeht, um die Verzweigungen zu generieren. Durch anschließende Aktivierungen der geschützten Funktionalitäten erhält man schließlich die bis zu dreifache Anzahl an funktionellen Gruppen der ersten Generation, die wiederum in UGI-4CRs eine hochverzweigte zweite Generation bilden können.

Das erfindungsgemäße Verfahren ist jedoch nicht wie in Figur 2 gezeigt darauf beschränkt, dass die Kerneinheit Carbonsäuregruppen als URG's aufweist, sondern kann auch eine andere URG aufweisen.

Im Rahmen des erfindungsgemäßen Verfahrens werden vorzugsweise monogeschützte bifunktionelle Bausteine, die aus unterschiedlichen organischen Resten (k, I, m und n) aufgebaut sind, eingesetzt, um α-Aminoacylamid-Einheiten aufzubauen, wodurch eine unendliche Produktdiversität erzeugt werden kann. Es handelt sich hierbei um eine UGI-(4CR)-Vierkomponentenreaktion, wobei ein Aminderivat, eine Carbonylkomponente (Aldehyd oder Keton), eine Carbonsäure (oder andere Ugi-reaktive Säuren wie HN₃ oder saure Phenole) und ein Isocyanidderivat (auch Isonitrile genannt) zu α-Aminoacylamid-Derivaten reagieren. Bei den organischen Resten kann es sich sowohl um einfache aliphatische Ketten sowie funktionalisierte Ketten hin bis zu hochkomplexen Biomolekülen handeln. Entscheidend ist, dass keine weiteren reaktiven Gruppierungen vorhanden sind, die ebenfalls in UGI-4CR's reagieren können.

Das erfindungsgemäße Verfahren erlaubt die Verwendung von bifunktionellen Bausteinen, die PURG's umfassen, wobei als geschützte Funktion (PURG, S) sämtliche Gruppierungen eingesetzt werden, die sich in einfachen Reaktionen zu URG's umsetzen lassen. Mögliche Funktionalisierungen und deren Methoden sind in der nachstehenden Tabelle 1 wiedergegeben.

Die Reaktionsführung des erfindungsgemäßen Verfahrens kann derart gestaltet werden, dass die Synthese sowohl in Lösung als auch an fester Phase durchgeführt werden kann. Die dadurch zwangsläufig gegebene und für den Fachmann naheliegende Erweiterung auf einen hohen Automatisierungsgrad ist eine wesentliche Charakteristik des erfindungsgemäßen Verfahrens.

**Tabelle 1**

| 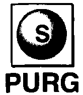 | 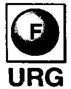 | **Möglichkeiten und Bedingungen** |
|---|---|---|
| -CO₂PG | -CO₂H | Spaltung von Esterfunktionen (CO₂Me, CO₂Et, CO₂*t*-Bu, CO₂Bn, CO₂All, etc.) nach unterschiedlichen Bedingungen (sauer, basisch, reduktiv, katalytisch, enzymatisch, etc.) |
| -CO-NH-PG | -CO₂H | Hydrolyse von Amiden, bevorzugt Indolylamiden unter schwach basischen Bedingungen |
| -CH₂OH oder -CH₂O-PG | -CO₂H oder-CHO | Oxidation von primären Alkoholen zu Aldehyden oder Carbonsäuren nach unterschiedlichen Varianten |
| -CH(OR)₂ | -CHO | Spaltung von Acetalen zu Aldehyden unter sauren Beding-ungen (Ketale/Ketone analog) |
| -NH-CHO | -NC | Umsetzung von Formamiden zu Isonitrilen mit wasser-entziehenden Reagenzien unter Baseneinfluß |
| -NH-PG | -NH₂ | Abspaltung von Aminoschutzgruppen (Boc, Cbz, Fmoc, Alloc, etc.) nach unterschiedlichen Bedingungen (sauer, basisch, reduktiv, katalytisch, etc.) |
| -N₃ oder -NO₂ | -NH₂ | Reduktion von Aziden oder Nitroverbindungen zu primären Aminen nach unterschiedlichen Verfahren |
| -CN | -CH₂NH₂ | Reduktion von Nitrilen zu primären Aminen |

Im folgenden sind beispielhaft im Rahmen der vorliegenden Erfindung einsetzbare funktionelle Gruppen an/in Ugi Dendrimeren aufgeführt (UBU = UGI-type branching unit; Peptoid-Peptid-Verzweigungselement, das aus einer UGI-Reaktion entsteht).

**Carbonsäuren/Carbonsäureester:**

| | |
|---|---|
| Methylester | 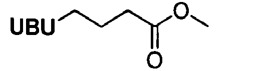 |
| | 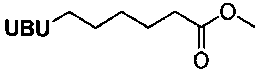 |
| Allylester | 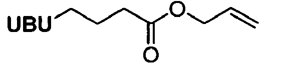 |
| Benzylester | 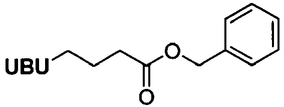 |
| Carboxylat | 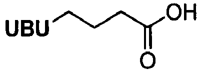 |

**Amine/geschützte Amine:**

| | |
|---|---|
| Cbz-Schutzgruppe | 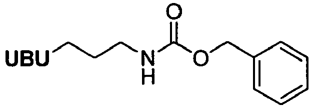 |
| Boc-Schutzgruppe | 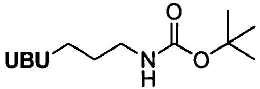 |
| Freies Amin | 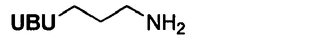 |

**Thiole:**

| | |
|---|---|
| *t*-Butyl-Thiol geschützt | 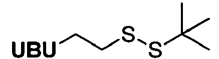 |

**Alkohole:**

| | |
|---|---|
| Hexanol | 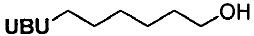 |

**Aromaten:**

| | |
|---|---|
| Methoxyphenyl | 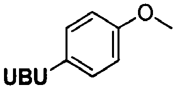 |
| Trifluormethylbenzol | 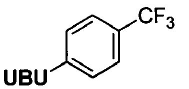 |

**Alkylketten:**

| | |
|---|---|
| C₈-Alkylkette | 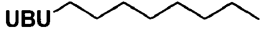 |
| Benzylgruppen | 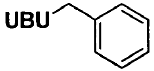 |
| *t*-Butyl-Gruppe | 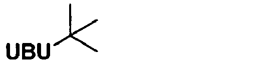 |
| Isopropyl-Gruppe | 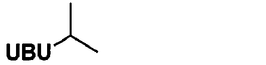 |
| Terminale Alkine | 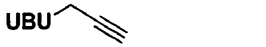 |

**Zuckerderivate:**

| | |
|---|---|
| 2-Acetamido-3,4,6-O-acetyl-2deoxy-β-D-glucose | 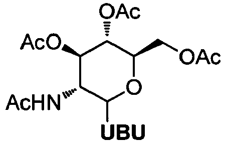 |
| Pentaacetyl-Mannose | 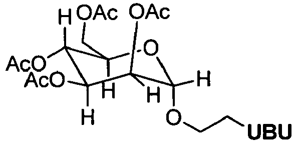 |

**PEG-Einheiten:**

| |
|---|
| 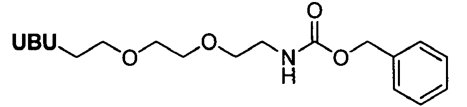 |

**Vorstufe konvertierbares Isocyanid:**

| |
|---|
| 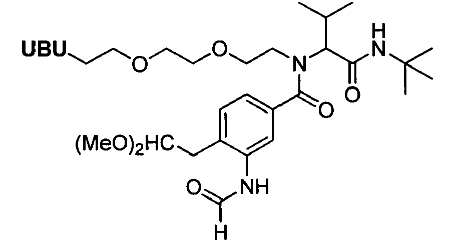 |

**Geschütze Aminosäuren:**

| | |
|---|---|
| Boc-Serin | 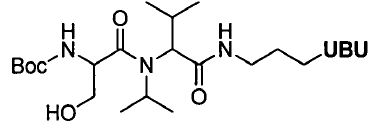 |

**Dopaminderivate:**

| | |
|---|---|
| Methoxy-geschützes Dopaminderivat | 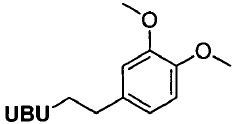 |

**Fluoreszenzfarbstoffe:**

| | |
|---|---|
| Fluorescein | 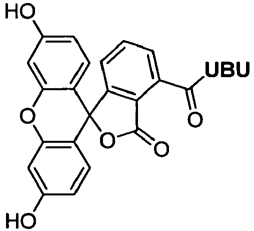 |
| Rhodamin B | 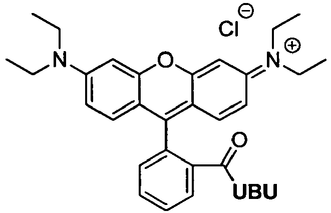 |
| Pyren | 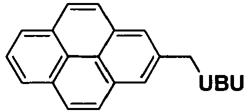 |

Beispielhafte Komponenten für die UGI-Dendrimerensynthese gemäß der vorliegenden Erfindung sind wie folgt:
Schutzgruppen (protective group = PG) / Vorläufergruppen für PURG (Analog auch Schutzgruppen für FG (functional unit)/NBU (nonbranching unit))

### Esterschutzaruppen für Carboxylfunktionen:

Methyl-, Ethyl-, n-Propyl-, iso-Propyl-, n-Butyl-, iso-Butyl-, Methoxymethyl-, Methylthiomethyl-, Tetrahydropyranyl-, Benzyloxymethyl-, Phenacyl-, N-Phthalimidomethyl-, 2,2,2-Trichlorethyl-, 2-Haloethyl-, 2-(p-Toluol-4-sulfonyl)ethyl-, tert.-Butyl-, Cinnamyl-, Benzyl-, Triphenylmethyl- (Trityl-), Bis(o-nitrophenyl)-methyl-, 9-Anthrylmethyl-, 2-(9,10-Di-oxo)anthrylmethyl-, Piperonyl-, Trimethyl-silyl-, 4-Nitrobenzyl-, 4-Methoxybenzyl-, Pyridyl-4-methyl- (4-Picolyl-), 4-Methoxyphenacyl-, Diphenylmethyl-(Benzhydryl-), Cyclohexyl-, 9-Fluorenylmethyl-, 1-Adamantyl-, 2-Adamantyl-, Allyl-, Dicyclopropylmethyl-, tert.-Butyldimethylsilylester.

### Amide und Hydrazide als Schutzaruppe für die Carboxylfunktion:

Indolylamide und deren Vorstufen, z.B. N-[2-(2',2'-dialkoxy-ethyl)phenyl-], Cyclohexenyl u.a., die nach (Synlett 2007, 3188-3192) aus sogenannten konvertierbaren Isonitrilen hervorgehen,
N,N-Dimethylamid, N-7-Nitroindoylamide, Hydrazid, N-Phenylhydrazid, N,N'-Diisopropylhydrazid.

### Aminoschutzgruppen vom Urethantyp:

Benzyloxycarbonyl- (Z oder Cbz-), tert.-Butyloxycarbonyl- (Boc-), Fluorenyl-9-methoxycarbonyl- (Fmoc-), 4-Methoxybenzyloxycarbonyl-, 2, 3 und 4-Nitrobenzyloxycarbonyl-, 2, 3 und 4-Chlorbenzyloxycarbonyl-, 3,5-Dimethoxybenzyloxycarbonyl-, α,α-Dimethyl-3,5-dimethoxybenzyloxycarbonyl-, 2-Nitro-4,5-dimethoxy-benzyloxycarbonyl- (6-Nitroveratryloxycarbonyl-), Furyl-2-methoxycarbonyl- (Furfuryloxycarbonyl-), 2-(4-Tolylsulfonyl)ethoxycarbonyl-, 4-Phenylazobenzyloxycarbonyl-, 2-lodethoxycarbonyl, 2-Cyan-tert.-butyloxycarbonyl-, 2,2,2-Trichlor-tert.-butyloxycarbonyl-, Isonicotinyloxycarbonyl-, tert.-Amyloxycarbonyl-, Adamantyl-1-oxycarbonyl-, 1-(1-Adamantyl)-1-methylethoxycarbonyl-, Isobornyloxycarbonyl-, 2-[Biphenyl-(4)]-propyl-2-oxycarbonyl-, Piperidinooxycarbonyl-, Cyclopentyloxycarbonyl-, α-Methyl-2,4,5-trimethylbenzyloxycarbonyl-, 5-Benzisoxazolylmethyloxycarbonyl-, (4-Phenylazophenyl)-isopropyloxycarbonyl-, Methyloxycarbonyl-, 2,2,2-Trichlorethyloxycarbonyl-, 2-Trimethylsilylethyloxycarbonyl-, 1,1-Dimethyl-propinyloxycarbonyl-, 1-Methyl-1-phenylethyloxycarbonyl-, 1-Methyl-1-(4-biphenyl)-ethyloxycarbonyl-, 1,1-Dimethyl-2-haloethyloxycarbonyl-, 1,1-dimethyl-2-cyanoethyloxycarbonyl-, Cyclobutyloxycarbonyl-, 1-Methylcyclobutyloxycarbonyl-, Vinyloxycarbonyl-, Allyloxycarbonyl-, Cinnamyloxycarbonyl-, 8-Chinolyloxycarbonyl-, N-Hydroxypiperidinyloxycarbonyl-, 2,4-Dichlorbenzyloxycarbonyl-, 9-Anthrylmethyloxycarbonyl-, 2-[4-(methylsulfonyl)phenylsulfonyl]ethyloxycarbonyl-, 2,2-Bis-(4'-nitrophenyl)ethyloxycarbonyl-, 2-(2,4-Dinitrophenyl)ethyloxycarbonyl-, Methylsulfonylethyloxycarbonyl-, Diphenylmethyloxycarbonyl-Schutzgruppen.

### Amide als Aminoschutzgruppen:

N-Formyl-, N-Acetyl-, N-Chloracetyl-, N-Trichloracetyl-, N-Trifluoacetyl-, N-o-Nitrophenylacetyl-, N-o-Nitrophenoxyacetyl-, N-Acetoacetyl-, N-3-Phenylpro-pionyl-, N-3-(p-Hydroxyphenyl)propionyl-, N-2-Methyl-(o-nitrophenoxy)propionyl-, N-2-Methyl-2-(o-phenylazophenoxy)propionyl-, N-4-Chlorobutyryl-, N-o-Nitrocinnamoyl-, N-Picolinoyl-, N-(N'-Acetylmethionyl)-, N-Benzoyl-, N-Phthaloyl-, N-Dithiasuccinoyl-Schutzgruppen.

### Spezielle Aminoschutzgruppen:

N-Allyl-, N-Phenacyl-, N-3-Acetoxypropyl-, N-Methoxymethyl-, N-Benzyloxyme-thyl-, N-Pivaloyloxymethyl-, N-Tetrahydropyranyl-, N-2,4-Dinitrophenyl-, N-Benzyl-, N-o-Nitrobenzyl-, N-Di(p-methoxyphenyl)methyl-, N-Triphenylmethyl- (N-Trityl-), N-(p-Methoxyphenyl)diphenylmethyl-, N-Diphenyl-4-pyridylmethyl-, N-2-Picolyl-N'-oxid-, N,N'-Isopropyliden-, N-Benzyliden-, N-p-Nitrobenzyliden, N-Salicyliden-, N-(5,5-Dimethyl-3-oxo-1-cyclohexenyl)-, N-Nitro-, N-Oxid-, N-Diphenylphosphinyl-, N-Dimethylthiophosphinyl-, N-Benzolsulfonyl-, N-o-Nitrobenzolsulfonyl-, N-Toluolsulfonyl- (N-Tosyl-), N-Benzylsulfonyl-, N-Trifluormethylsulfonyl-, N-Phenacylsulfonyl-Schutzgruppen und die Schützung von Aminogruppen als quarternäre Ammoniumsalze.

### Schutzgruppen für Carbonylfunktionen (Aldehyde und Ketone):

Dialkyl-, Bis(2,2,2-trichlorethyl)-, S,O- und S,S'-Dialkylacetale und -ketale, 1,3-Dioxan, 5-Methylen-1,3-dioxan, 5,5-Dibrom-1,3-dioxan, 1,3-Dioxolan, 4-Brommethyl-1,3-dioxolan, 4-o-Nitrophenyl-1,3-dioxolan, 1,3-Dithian, 1,3-Dithiolan, 1,3-Oxathiolan, O-Trimethylsiliylcyanohydrin, N,N-dimethylhydrazon, 2,4-Dinitrophenylhydrazon, O-Phenylthiomethyloxim, substituierte Methylenderivate, Bismethylendioxoderivate.

### Gruppen die in Isonitril umgewandelt werden können:

N-Formamid, N-Formamid-Orthoester

### Gruppen, die mit CN-Reagenzien in ein Isonitril überführt werden können:

-Cl, -Br, -I, OTs, O-Ms, -O-TFA, Epoxid

### Weitere Schutzgruppen (PG)

Viele sonstige funktionelle Gruppen müssen bei UGI-Reaktionen im allgemeinen selten geschützt werden. Sollen aber spezielle Reaktionen zur Funktionalisierung durchgeführt werden, kann eine Schützung, z.B. der Hydroxylfunktion oder Sulfhydrylgruppe, notwendig sein.

### Schützen von Hydroxylgruppen als Ether:

Methyl-, Methoxymethyl- (MOM-), Methylthiomethyl- (MTM-), 2-Methoxyethoxymethyl-(MEM-), Bis(2-Chlorethoxy)methyl-, Tetrahydropyranyl- (THP-), Tetrahydrothiopyranyl-, 4-Methoxytetrahydropyranyl-, 4-Methoxytetrahydrothiopyranyl-, Tetrahydrofuranyl-, Tetrahydrothiofuranyl-, 1-Ethoxyethyl-, 1-Methyl-1-methoxyethyl-, 2-(Phenylselenyl)ethyl-, tert.-Butyl-, Allyl-, Benzyl-, o-Nitrobenz-yl-, Triphenylmethyl-(Trityl-), α-Naphthyldiphenylmethyl-, p-Methoxyphenyldiphenylmethyl-, 9-(9-Phenyl-10-oxo)anthryl-, Trimethylsilyl- (TMS-), Isopropyldimethylsilyl-, tert.-Butyldimethylsilyl-(TBDMS-), tert.-Butyldiphenylsilyl-, Tribenzylsilyl-, Triisopropylsilyl-Ether.

### Schützen von Hydroxylgruppen als Ester:

Formiat, Acetat, Trichloracetat, Phenoxyacetat, Isobutyrat, Pivaloat, Adamantoat, Benzoat, 2,4,6-Trimethylbenzoat (Mesitoat), Methylcarbonat, 2,2,2-Trichlorethylcarbonat, Allylcarbonat, p-Nitrophenylcarbonat, Benzylcarbonat, p-Nitrobenzylcarbonat, S-Benzylthiocarbonat, N-Phenylcarbonat, Nitrat, 2,4-Dinitrophenylsulfenat.

**Beispiele für FGs, NBUs, die für die Eigenschaften der Polymere relevant sind:**

Auch Biomoleküle und Naturstoffe wie Peptide, Lipide, Saccharide, Steroide, Nucleotide, Terpene und Alkaloide können Bausteine für die Dendrimerensynthese darstellen. Da eine vollständige Nennung aller möglichen organischen Reste nicht möglich ist, sollen an dieser Stelle einige Beispiele vorgestellt werden.

### Alkyl:

Gesättigte disubstituierte Kohlenwasserstoffreste (Alkylreste): Methyl-, Ethyl-, n-Propyl-, iso-Propyl-, n-Butyl-, sek.-Butyl-, tert.-Butyl-, n-Pentyl- und isomere Pentyl-Reste (z. B. Neopentylreste), n-Hexylreste und isomere Strukturen, n-Heptyl- und isomere Strukturen, n-Octyl- und isomere Strukturen, n-Nonyl und isomere Strukturen, n-Decyl- und isomere Strukturen, C₁₁ bis C>₁₀₀-Ketten (linear und verzweigt)

### Alkenyl- und Alkinylreste und mehrfach ungesättigte Kohlenwasserstoffreste (Diene, Triene, Polyene, Düne, Triine und Polyine können kumuliert, konjugiert und isoliert sein):

Ethenyl- (Vinyl-), Propenyl- (Allyl-), 1-Butenyl-, 2-Butenyl-, Isobutenyl- (2-Methylpropenyl-), 1-Pentenyl-, 2-Pentenyl-, 3-Pentenyl- und isomere Strukturen, lineare und verzweigte Hexenylreste, lineare und verzweigte Heptenylreste, lineare und verzweigte Octenylreste, lineare und verzweigte Nonenylreste, lineare und verzweigte Decenylreste, C₁₁ bis C>₁₀₀-Ketten (linear und verzweigt), Ethinyl-, Propinyl- (Propargyl-), 1-Butinyl-, 2-Butinyl-, lineare und verzweigte Pentinylreste, lineare und verzweigte Hexinylreste, lineare und verzweigte Heptinylreste, lineare und verzweigte Octinylreste, lineare und verzweigte Noninylreste, lineare und verzweigte Dodecinylreste, C₁₁ bis C>₁₀₀-Ketten (linear und verzweigt),
Reste die sich vom Einsatz von C₁₂₋₂₂-Fettsäuren mit 1-5 Doppelbindungen ableiten, speziell von Stearin-, Öl-, Linol-, Linolen-, Arachidonsäure, z.B. Z-Heptadec-8-enyl von der Ölsäure als UGI-Carbonsäurekomponente,
Butadienyl-, Pentadienyl-, lineare und verzweigte Hexadienyl-, Hexatrienyl-, lineare und verzweigte Heptadienyl-, Heptatrienyl-, lineare und verzweigte Octadienyl- und Octatrienyl-, Octatetraenyl-

### Cyclische Kohlenwasserstoffe (gesättigt, ungesättigt, mehrfach ungesättigt und aromatisch):

Cyclopropyl-, Cyclobutyl-, Cyclopentyl-, Cyclohexyl-, Cycloheptyl-, Cyclooctyl-, Cyclononyl- , Cyclo-C₁₀-Reste bis macrocyclische Systeme (C>₁₀₀), Cyclopropenyl-, Cyclobutenyl-, Cyclopentenyl-, Cyclohexenyl-, Cycloheptenyl-, Cyclooctenyl-, Cyclononenyl-, ungesättigte Cyclo-C₁₀-Reste bis macrocyclische Systeme (C>₁₀₀), Cyclopentadienyl-, Cyclohexadienyl-, Cycloheptadienyl-, Cycloheptatrienyl-, Cyclooctadienyl-, Cyclooctatrienyl-, Cyclooctatetraenyl-, einfach und mehrfach ungesättigte Cyclo-C₉-Reste bis macrocyclische Systeme (C>₁₀₀), bicyclische, disubstituierte Kohlenwasserstoffe wie Campher, Camphen, Bornan, Norbornan, Norbornen und Spiroverbindungen, aromatische Systeme wie disubstiuierte Benzole, Toluole, Naphthaline, Anthracene, Phenanthrene, Pyrene, Chrysene, Fluorene, Indene, Acenaphthene, Azulene, Biphenyle, Estradiol, gallensäurenabgeleitetet Sterole, Phytosterole, Phytosterane, und viele andere polycyclische Verbindungen

### Heterocyclische Systeme (gesättigt, ungesättigt, mehrfach ungesättigt und aromatisch):

Furane, Pyrane, Dioxane, Benzofurane, Pyrone, Chromene, Dibenzofurane, Xanthene, Pyrrole, Pyrazole, Imidazole, Triazole, Pyridine, Pyridazine, Pyrimidine, Pyrazine, Triazine, Tetrazole, Indole, Chinoline, Isochinoline, Carbazole, Acridine, Phenanthridine, Indazole, Benzimidazole, Cinnoline, Chinazoline, Chinaxoline, Phenazine, Benzocinnoline, Phenanthroline, Purine, Thiophene, Thiopyrane, Benzothiophene, Thioxanthene, Isoxazole, Oxazole, Isothiazole, Thiazole, Benzoxazole und Phenoxazine und viele weitere Heterocyclen

### Alkohole und Ether:

PEG-OH, PEG-OMe, etc.

### Zucker, geschützte Zucker und über Linker verknüpfte Zucker:

z.B. Glucosyl, Mannosyl, Galaktosyl, etc.
Glukosyl-alkyl, Mannosyl-alkyl, etc.
Glucosyl-PEGyl, Mannosyl-PEGyl, etc.
N-Acetylglucosaminyl, N-Acetylglucosaminyl-alkyl, N-Acetylglucosaminyl-PEGyl Sialyl, Sialyl-alkyl, Sialyl-PEGyl

### Thiole, Sulfide, Disulfide, Selenide und deren Oxidationsprodukte (Sulfoxide, Sulfone, Sulfonsäuren, Sulfate), (PG = H, Schutzgruppe):

z.B. -S-PG, -S-Alkyl, -S-Aryl, -Se-PG, -Se-Alkyl, -Se-Aryl, -S(=O)-Alkyl, -SO₂-Alkyl,-S(=O)-Aryl, -SO₂-Aryl, SO₃-PG, -OSO₃-PG, -S-S-Alkyl, -S-S-Aryl.

### Farbstoffe (inkl. Fluoreszenzfarbstoffe):

z. B. Rhodaminyl, Fluoresceinyl, Cyanine dyes, Perylenyl, Coumarinyl (inkl. Derivate), Bodipy Dyes, etc.

### Chelatoren für die Komplexierung von Metallionen, speziell Lanthaniden zur MRT-Kontrastierung (Gd):

EDTA, DOTA, DTPA, etc.

Seitenketten und geschützte Seitenketten von Aminosäuren, deren homologe und nor-Verbindungen, N-geschützte oder carboxylatgeschützte Aminosäuren, die über die freie Carboxylat- bzw. Amino-Funktion in die UGI-Reaktion eingehen (PG = H oder eine oder mehrere Schutzgruppen - der Funktion angepaßt), z.B. umfassend:

**Cystein/Cysteinhomologe:**

| | |
|---|---|
| 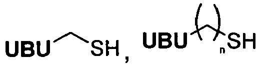 | 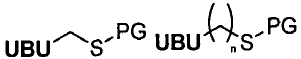 |
| 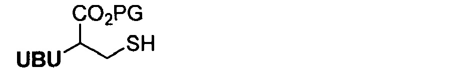 | 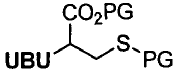 |
| 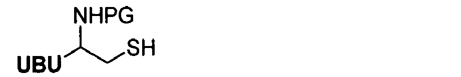 | 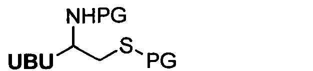 |

**Methionin:**

| |
|---|
| 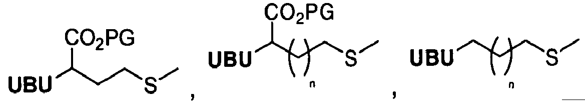 |
| 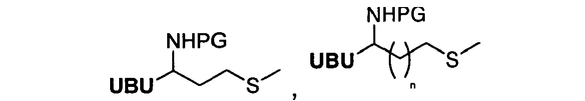 |

**Serin**

| |
|---|
| 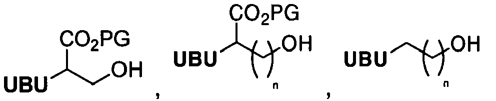 |
| 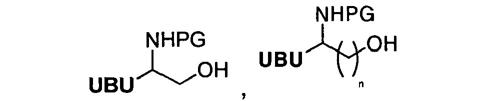 |

**Threonin**

| |
|---|
| 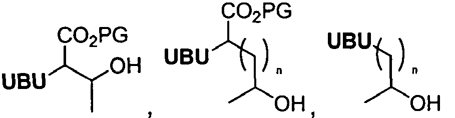 |
| 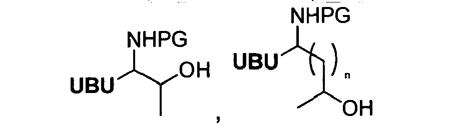 |

**Arginin**

| |
|---|
| 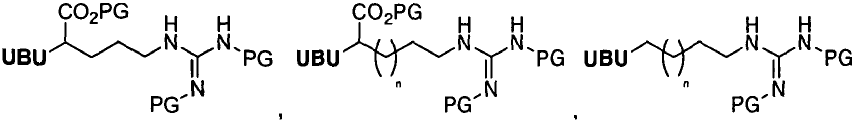 |
| 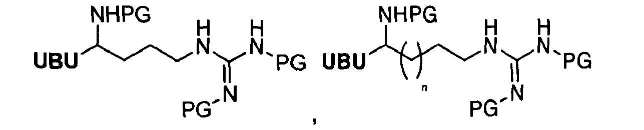 |

**Lysin**

| |
|---|
| 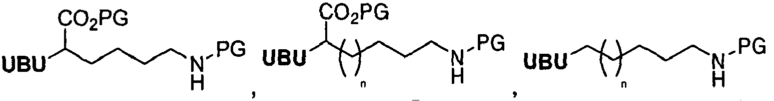 |
| 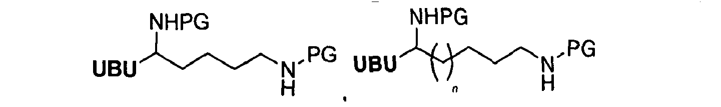 |
| 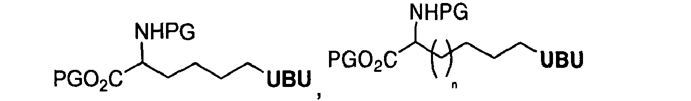 |

**Asparagin**

| |
|---|
| 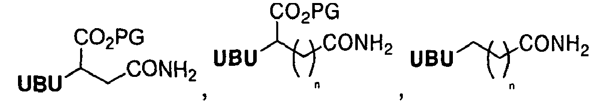 |
| 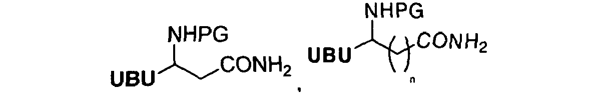 |

**Asparaginsäure**

| |
|---|
| 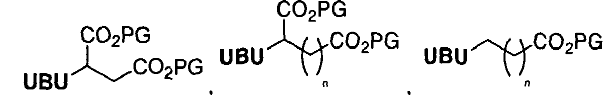 |
| 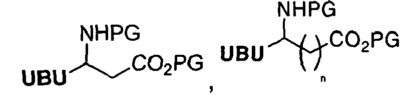 |
| 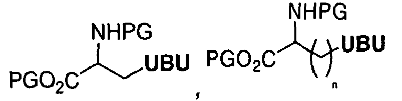 |

**Glutamin**

| |
|---|
| 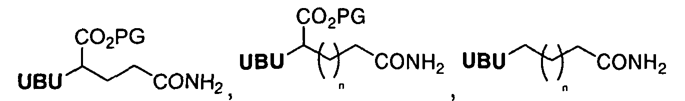 |
| 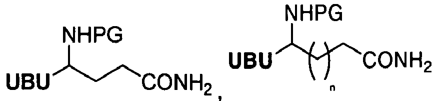 |

**Glutaminsäure**

| |
|---|
| 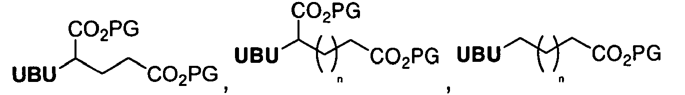 |
| 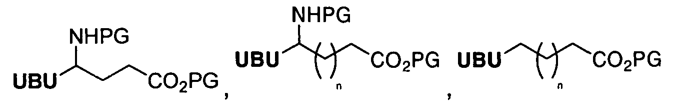 |
| 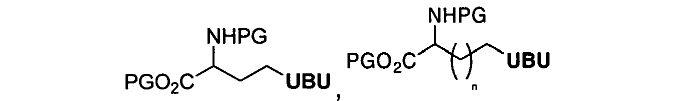 |

**Phenylalanin**

| |
|---|
| 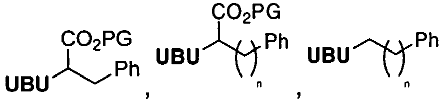 |
| 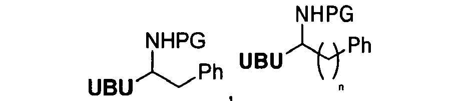 |

**Thyrosin**

| |
|---|
| 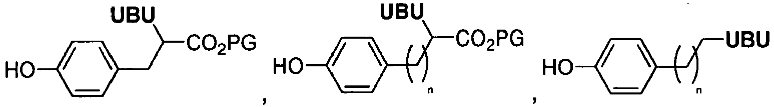 |
| 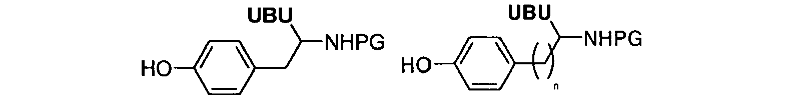 |

**Tryptophan**

| |
|---|
| 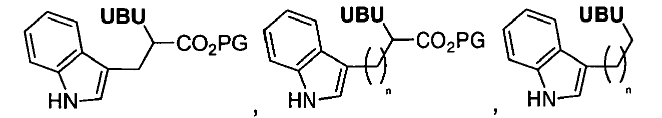 |
| 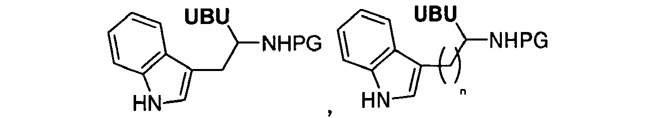 |

**Histidin**

| |
|---|
| 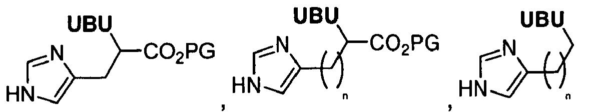 |
| 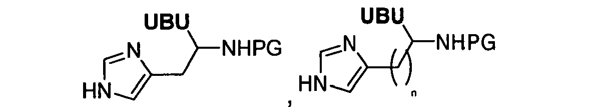 |

**Prolin / Hydroxyprolin**

| |
|---|
| 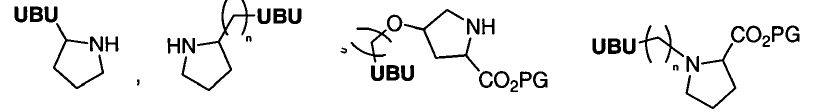 |

**Aminosäuren mit Alkylketten**

| |
|---|
| 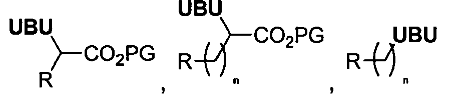 |
| 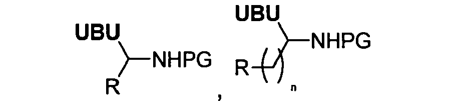 |

R = -H(Glycin), -CH₃(Alanin), -CH(CH₃)₂(Valin), -CH₂-CH(CH₃)₂(Leucin), -CH(CH₃)-CH₂CH₃(Isoleucin)

### Linker

Alkylidengruppen, Aryliden, -PEG-, etc., insbesondere auch sämtliche als FG aufgeführte Bausteine, als "di-yl"
PEG-Linker: n = 0, 1, 2, 3 .....

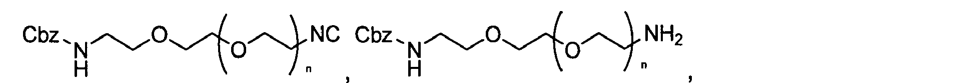

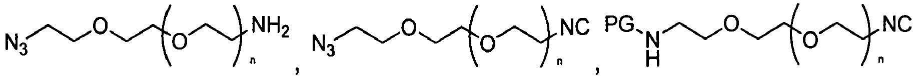

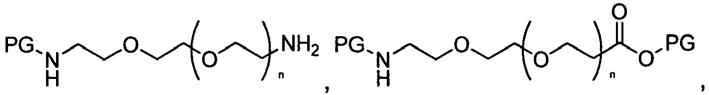

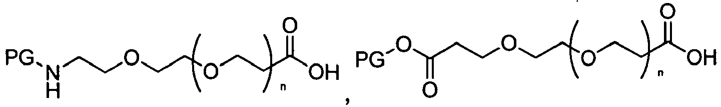

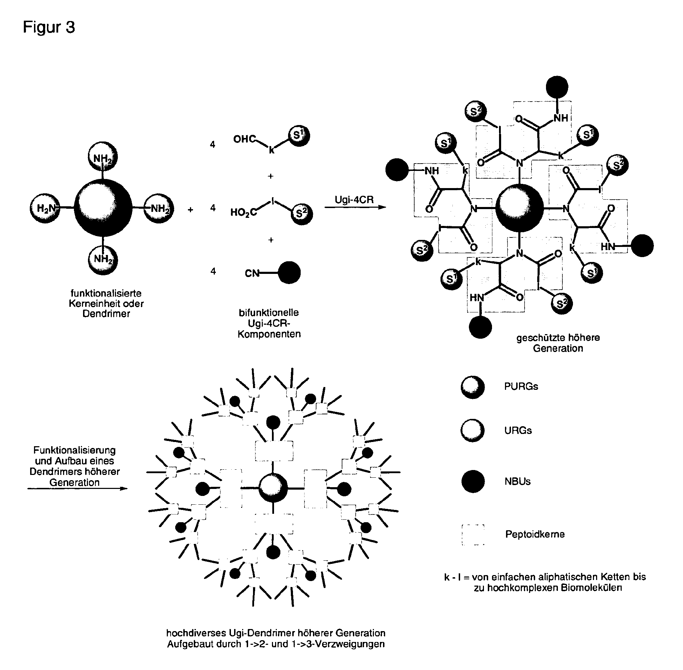

In einer bevorzugten Ausführungsform werden bifunktionelle Bausteine eingesetzt, die eine nicht verzweigende Einheit umfassen (NBU, engl. nonbranching unit), wodurch sich 1 →2-Verzweigungen oder lineare Verlängerungen in jeder Generation beliebig generieren lassen. Durch Verwendung unterschiedlichster NBUs als UGI-4CR-Komponenten im erfindungsgemäßen Verfahren kann die peptid-peptoidische Struktur des aufgebauten Dendrimers beeinflusst werden. Diese Erkenntnis erhöht die Diversität in der Synthese der UGI-Dendrimere weiterhin beträchtlich, wie in Figur 3 gezeigt.

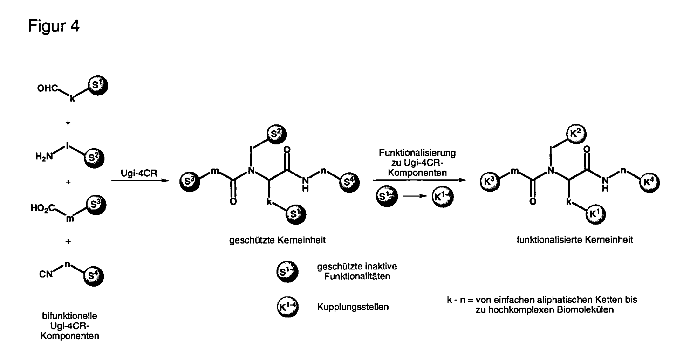

In einer weiteren bevorzugten Ausführungsform umfasst das erfindungsgemäße Verfahren die Verwendung von multifunktionellen Kerneinheiten, welche aus bekannten polyfunktionellen Molekülen mit URG an der Oberfläche bestehen können, oder welche ebenfalls über UGI-4CR's synthetisiert werden (was einer Union von Kerneinheit und 1. Generation entspricht). In letzterer Ausführungsform liefert die einfache Reaktion mit vier bifunktionellen Komponenten nach Aktivierung eine tetrafunktionelle Kerneinheit mit einer α-Aminoacylamid-Einheit. Da die Komponenten sich ebenfalls beliebig variieren lassen, kann eine hohe Diversität schon in der Kerneinheit generiert werden, wie es aus Figur 4 deutlich wird.

Natürlich gilt auch hier die Regel, dass nicht alle vier Komponenten bifunktionell aufgebaut sein müssen, sondern der Einsatz von NBU's di- oder trifunktionalisierte Kerneinheiten liefern kann. UGI-monofunktionalisierte Peptid-peptoide lassen sich als Ankereinheit von Dendrons in konvergenten Synthesen nutzen. Durch die vorgegebene Asymmetrie der α-Aminoacylamide lassen sich, je nach eingesetzter nichtverzweigender Komponente, grundlegend unterschiedliche peptid-peptoidische Strukturen im Kern erzeugen. Allein in der Kern- oder Ankereinheit lassen sich 15 strukturell verschiedene funtionalisierte α-Aminoacylamide formulieren. Tetra- und trifunktionelle Kerneinheiten eignen sich hervorragend für den divergenten Aufbau der Dendrimere. Bifunktionelle α-Aminoacylamide können ebenfalls in der erfindungsgemäßen divergenten Methode eingesetzt werden.

In einer bevorzugten Ausführungsform fungieren Ester zur Überführung der geschützten Funktionalitäten (S) in reaktive Kupplungsstellen (K), die nach erfolgter Darstellung der z.B. ersten Generation durch Hydrolyse in aktive Carboxylgruppen umgewandelt werden, die dann erneut in UGI-4CRs eingesetzt werden können.

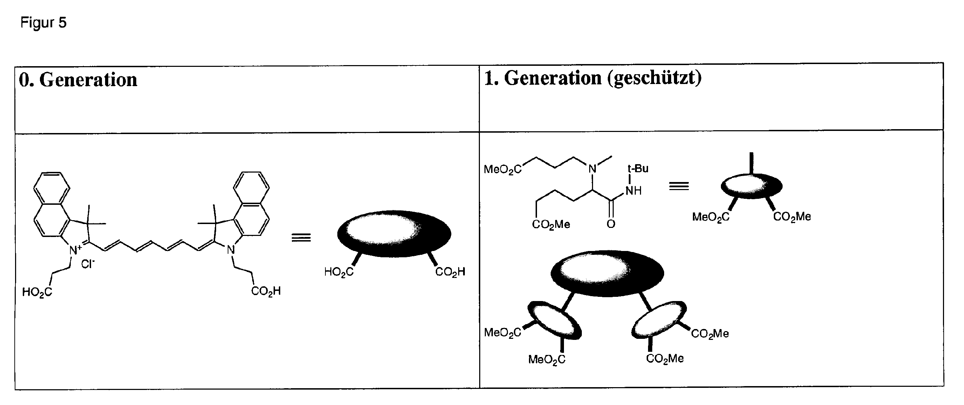

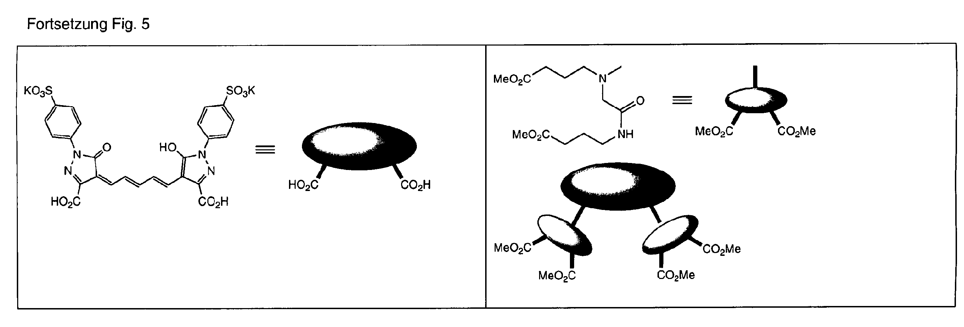

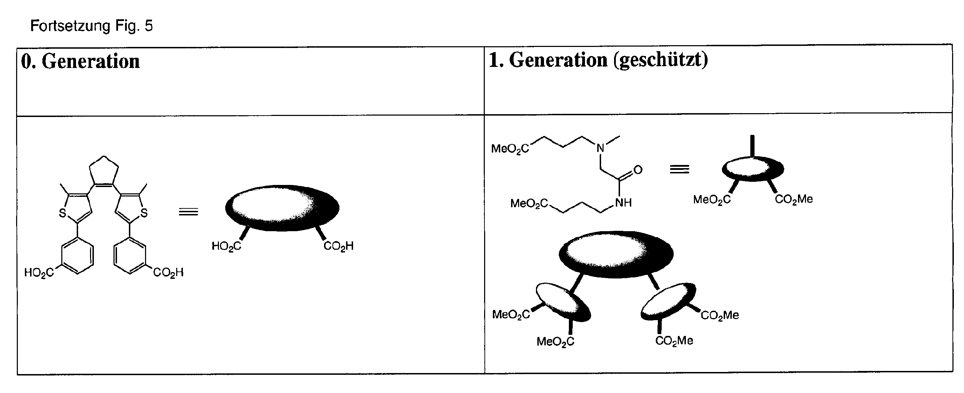

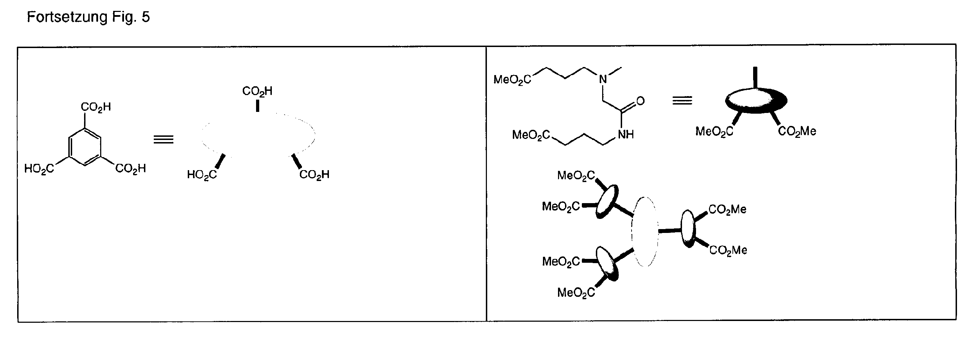

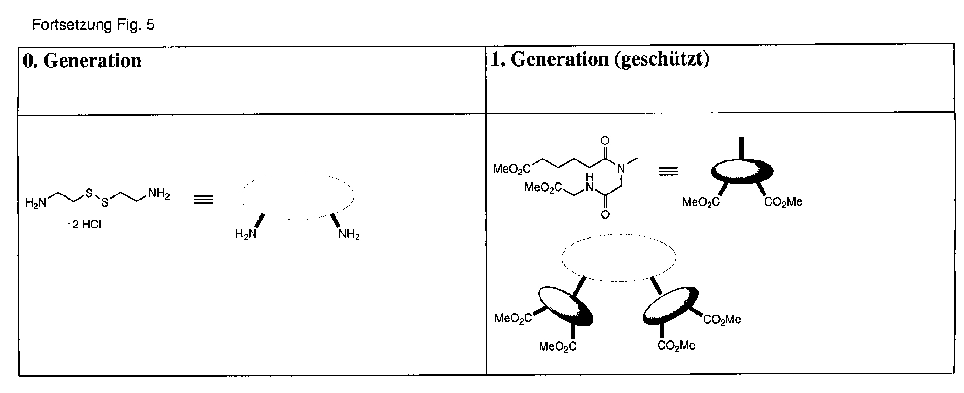

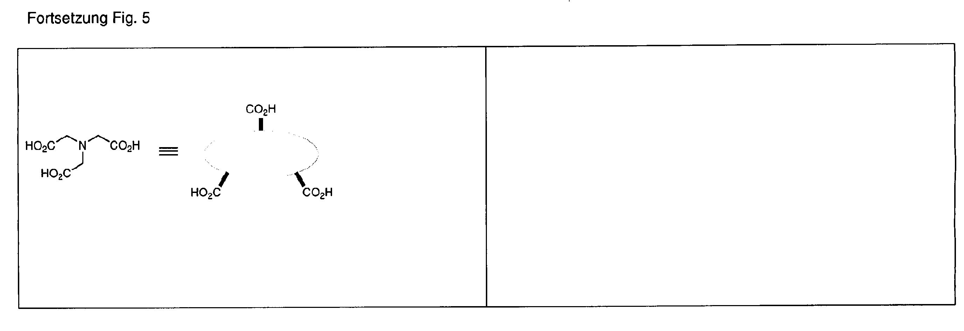

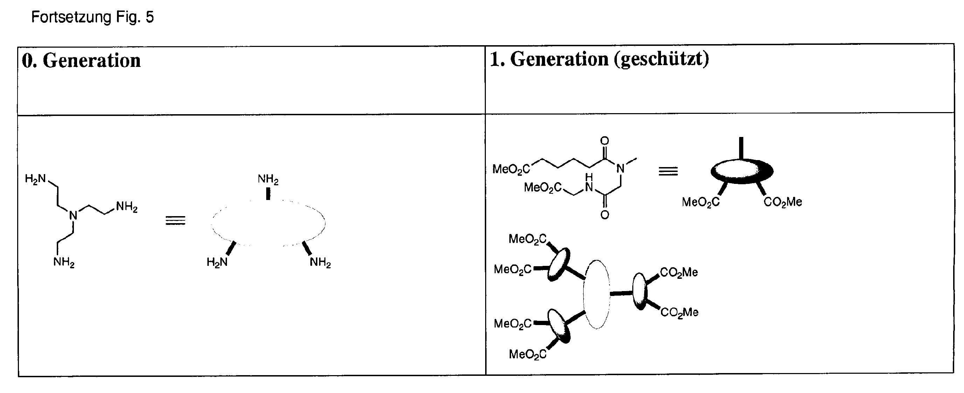

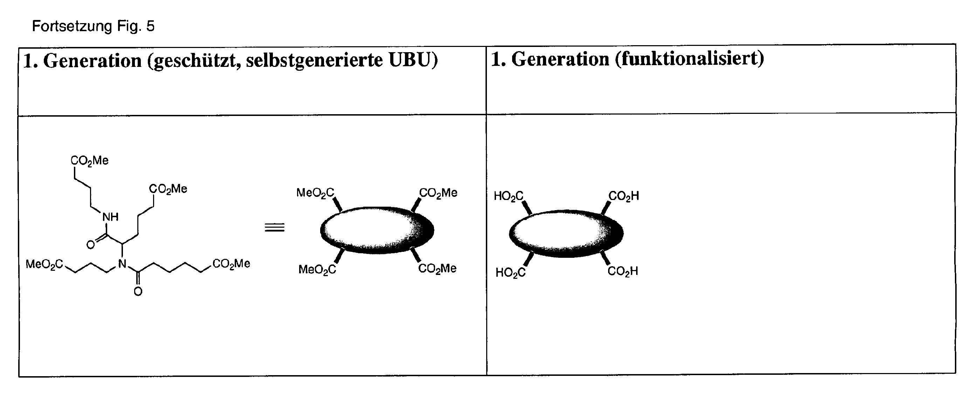

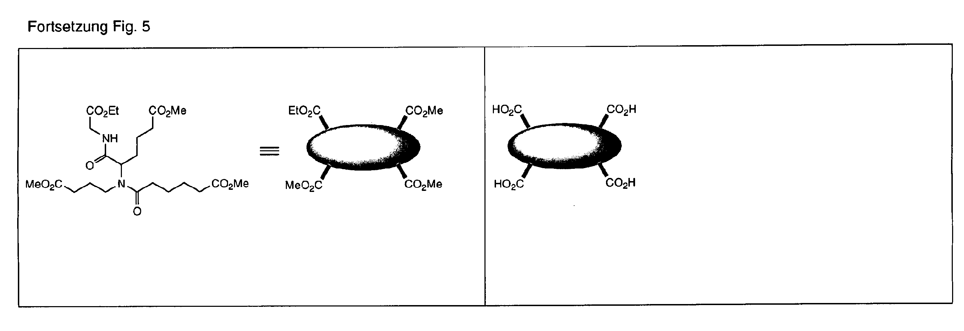

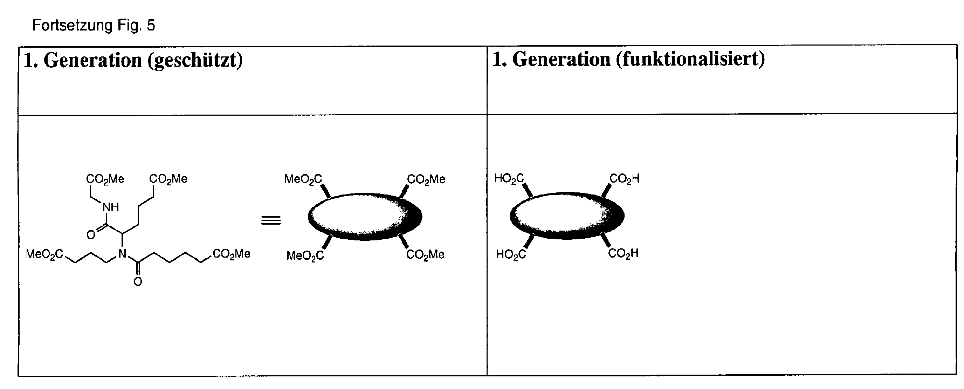

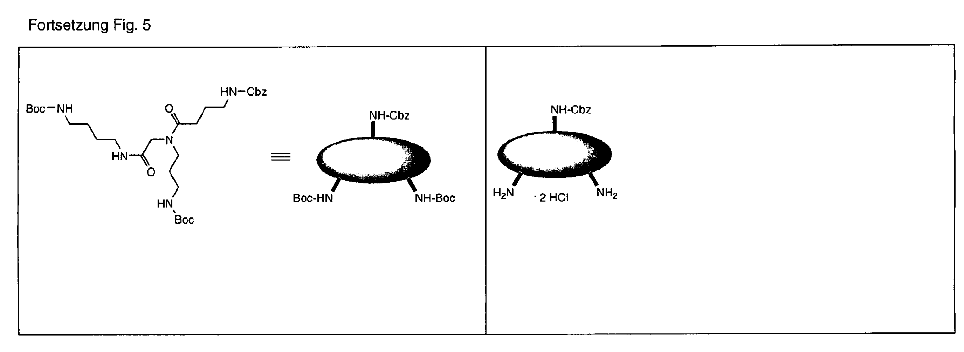

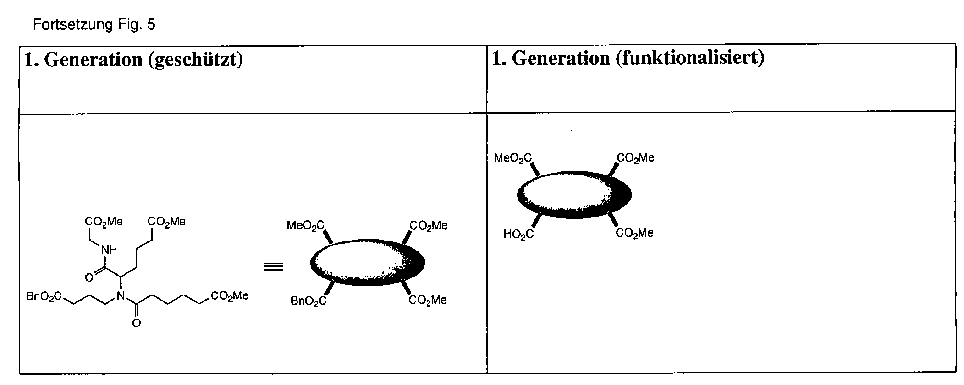

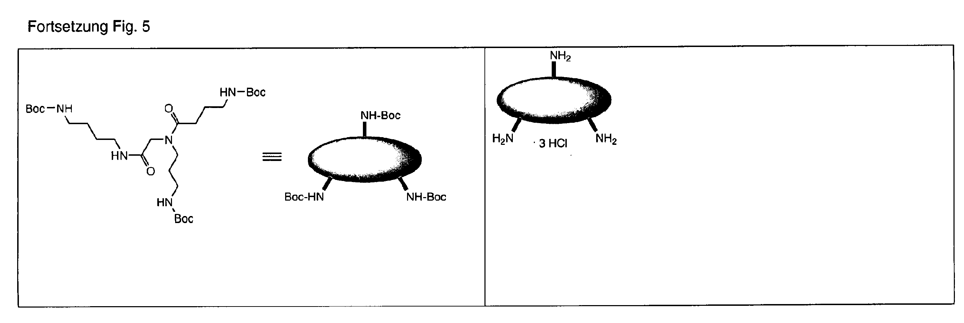

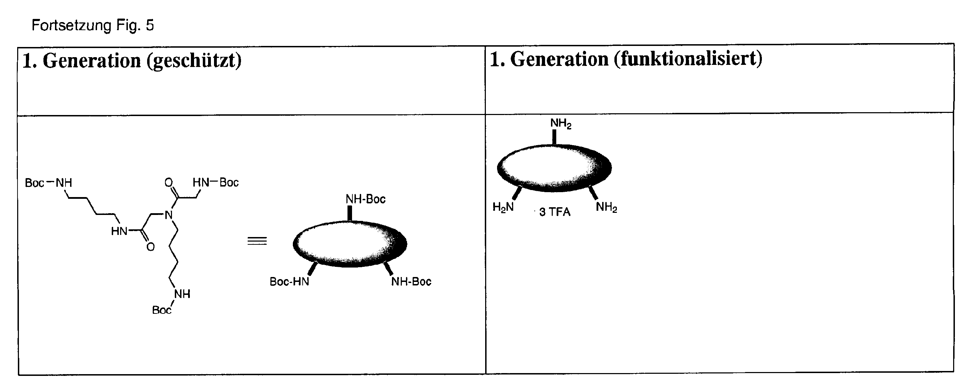

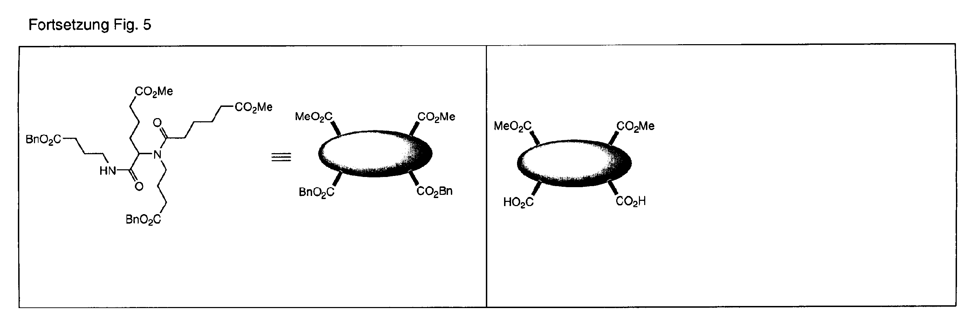

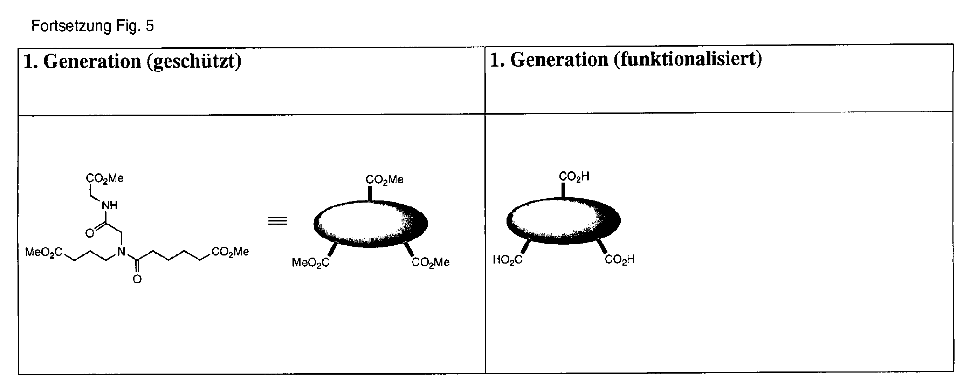

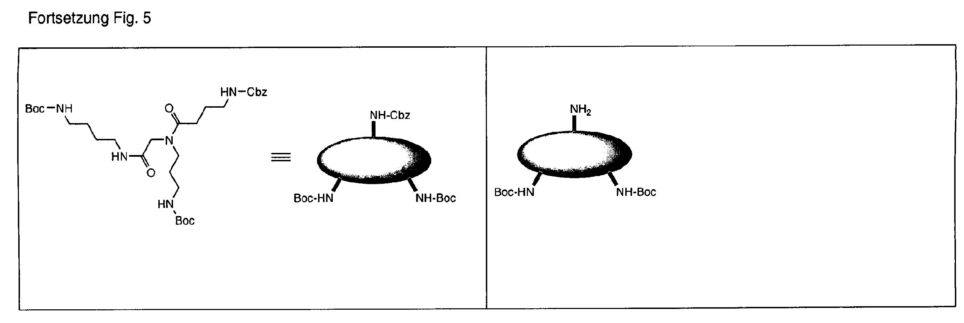

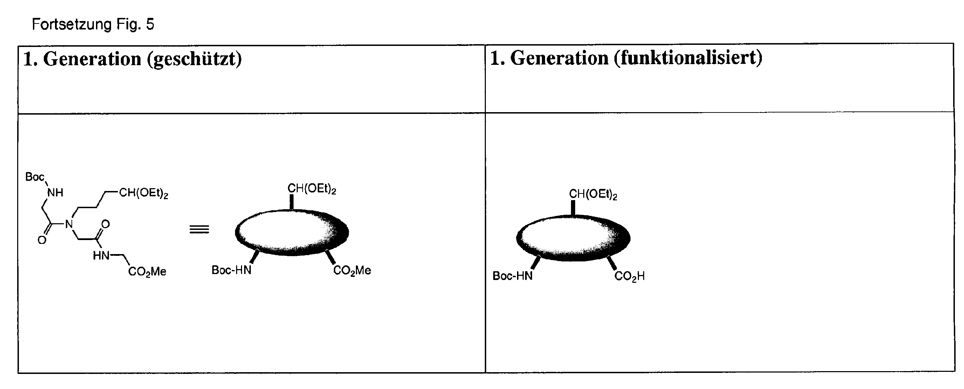

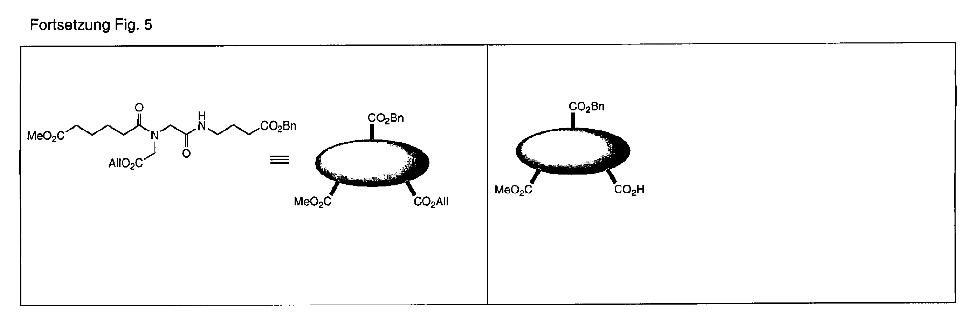

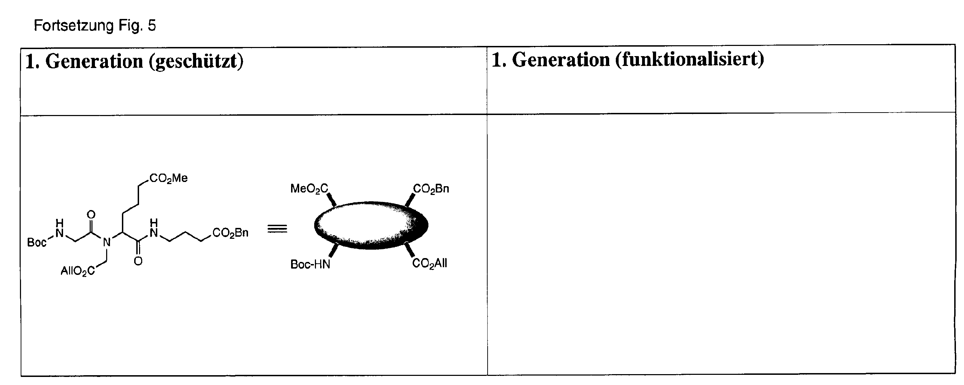

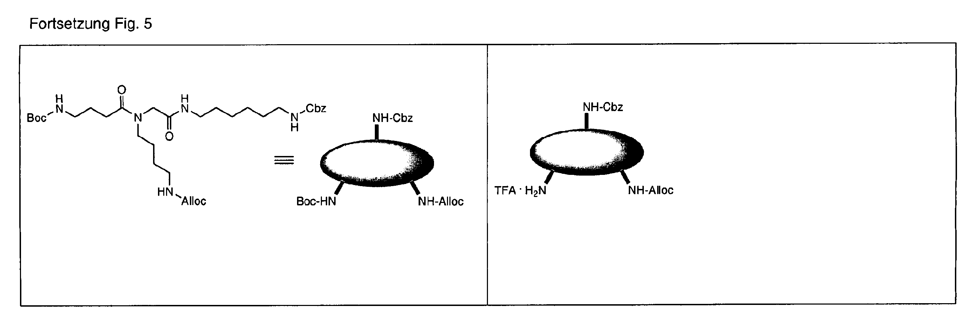

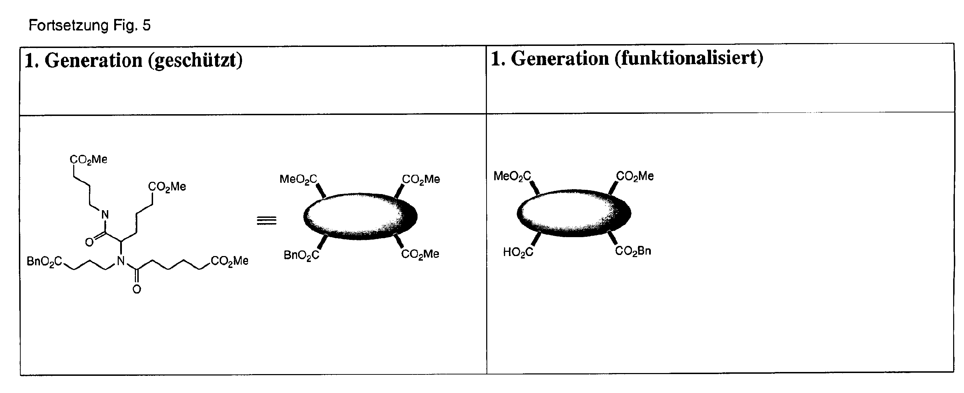

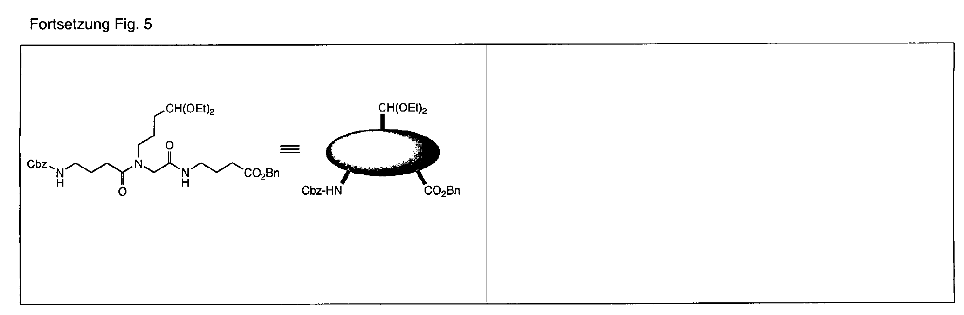

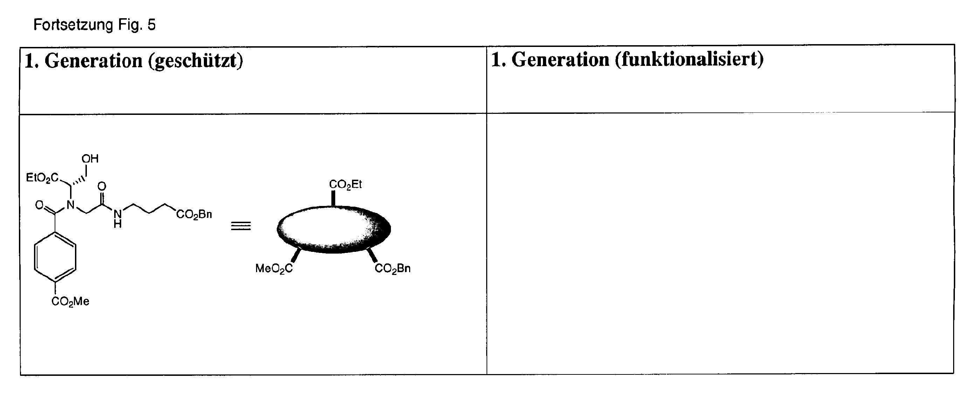

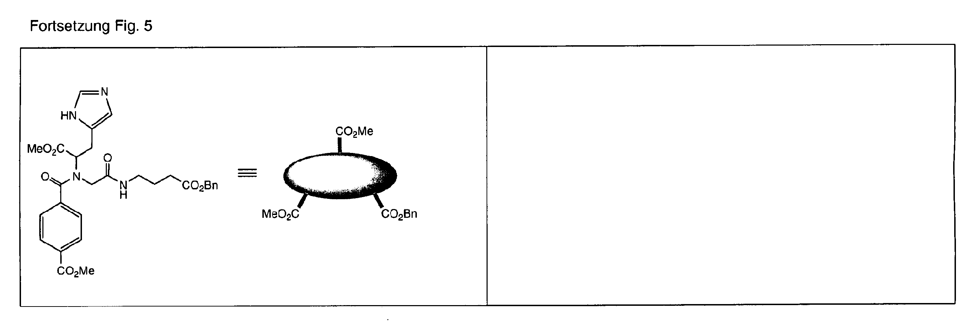

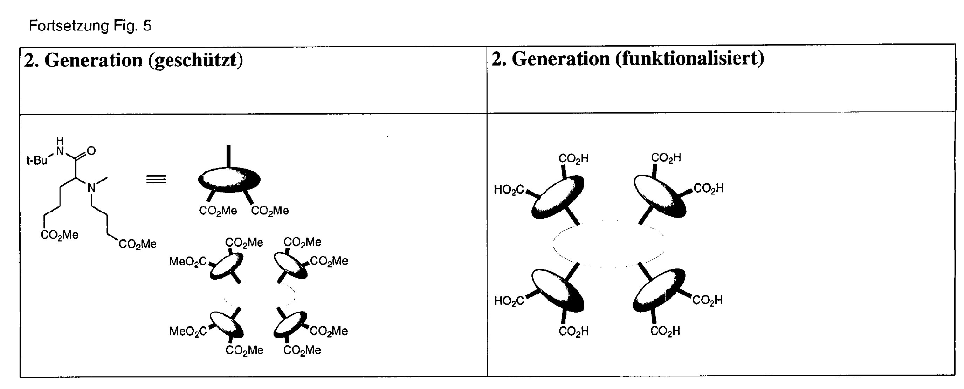

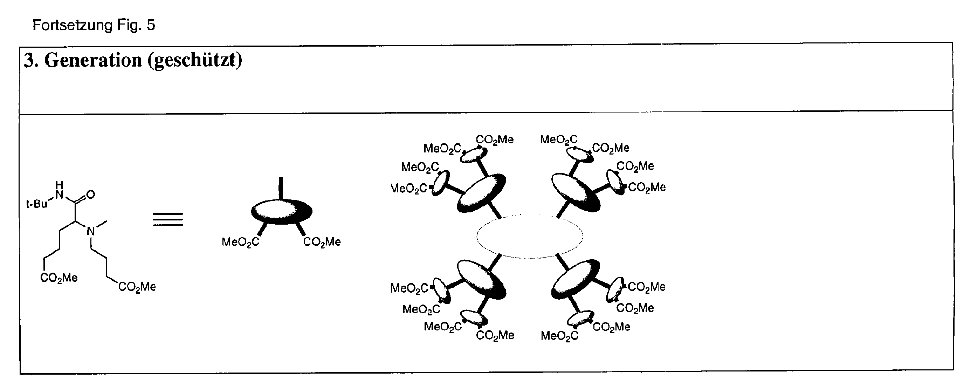

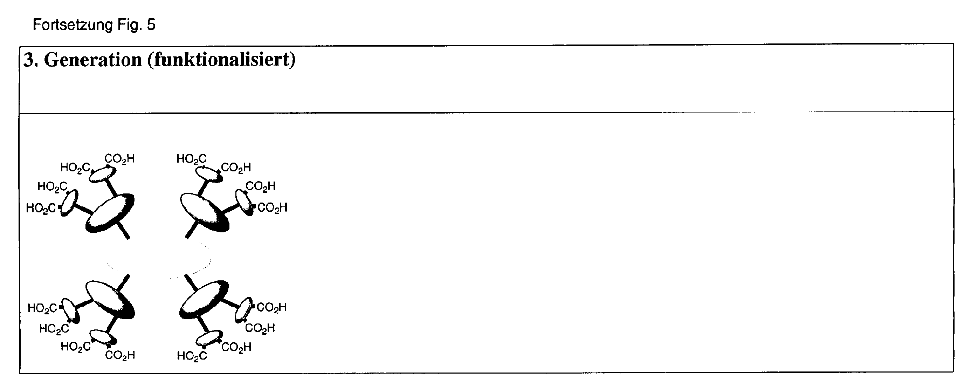

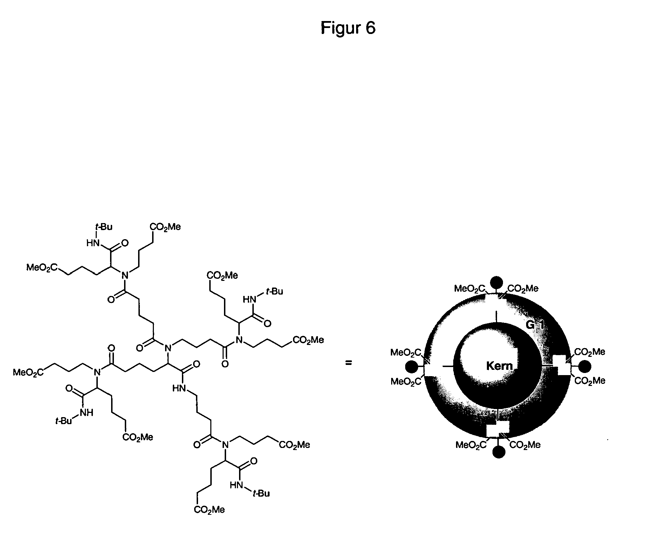

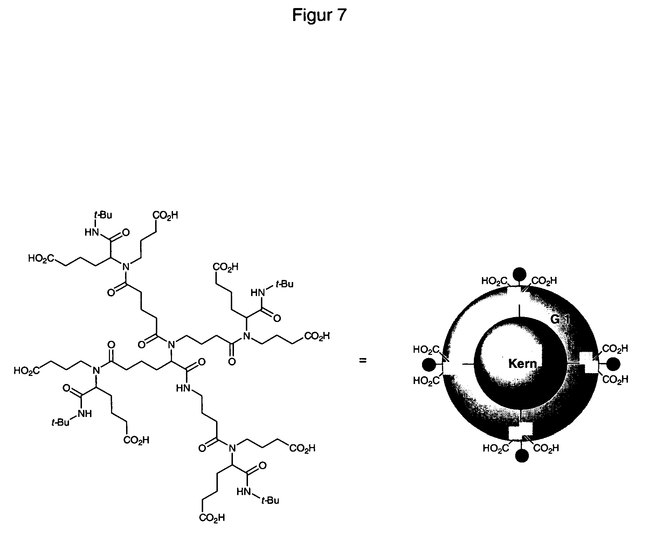

Figur 5 zeigt exemplarisch die "0. Generation", d.h. Kerneinheiten, die nicht durch verzweigte UGI-Reaktion hergestellt wurden, z.B. (funktionalisierte) Polysäuren und Polyamine, sowie daraus hervorgehende Dendrimere der 1. Generation gemäß der vorliegenden Erfindung.

In einer weiteren bevorzugten Ausführungsform werden durch geeignete Schutzgruppentaktiken gezielt bestimmte geschützte Funktionen selektiv in jeder Generation des divergent aufgebauten Dendrimers oder in der Kerneinheit aktiviert. Entscheidend ist, dass Schutzgruppen und Reaktionsbedingungen so gewählt werden müssen, dass andere geschützte Funktionen unverändert bleiben und keine Nebenreaktionen eingehen (orthogonaler Schutz). Dies erfordert je nach gewünschter Komplexität entsprechende, dem Fachmann bekannte Schutzgruppentaktiken, erlaubt dann aber die Synthese von hochkomplexen, "designten" Dendrimeren nach divergenter Methode, wie z.B. die Synthese von Janusdendrimeren.

In einer weiteren bevorzugten Ausführungsform umfasst das erfindungsgemäße Verfahren das Bilden von stereogenen Zentren in jeder α-Aminoacylamid-Einheit. Dadurch lassen sich bereits in der ersten Generation mit fünf stereogenen Zentren 32 verschiedene Stereoisomere formulieren. Das erfindungsgemäße Verfahren ermöglicht beispielsweise die Synthese von künstlichen Enzymen, um gezielt aktive Zentren mit nötiger Flexibilität und Zugänglichkeit für Substrate zu generieren.

In einer weiteren bevorzugten Ausführungsform umfasst das erfindungsgemäße Verfahren ferner das Aktivieren der Oberfläche der erhaltenen peptid-peptoidischen Dendrimeren in einer weiteren UGI-4CR, wobei peptid-peptoidische Dendrimere höherer Generation generiert werden, die unterschiedliche Funktionalitäten an ihrer Oberfläche aufweisen.

Das erfindungsgemäße Verfahren weist gegenüber den herkömmlichen Verfahren erhebliche Vorteile auf:
- Die Reste der eingesetzten bifunktionellen Bausteine können jede beliebige Struktur beinhalten (von einfachen aliphatischen Ketten bis zu hochkomplexen Biomolekülen). Die Bedingung ist allerdings, dass keine weiteren UGI-reaktiven Gruppen im Rest vorhanden sind.
- Der maximale Verzweigungsgrad beträgt drei (bei Verwendung von Ketonen: vier), ausgehend von jeder funktionellen Gruppe der Kerneinheit. Diese 1→3-Verzweigungen sind möglich, aber nicht notwendig. Durch Verwendung von NBUs lassen sich 1→2-Verzweigungen, lineare Verlängerungen oder nicht (P)URG funktionalisierte Segmente generieren.
- Die Asymmetrie der erhaltenen α-Aminoacylamid-Verzweigungspunkte erlaubt es, durch Verwendung unterschiedlicher nicht verzweigender Komponenten nahezu beliebig unterschiedliche Strukturen in der Verzweigungseinheit zu erzeugen (z.B. verschiedene Längen).
- Homogene Funktionalitäten in der Peripherie können unterschiedlich geschützt vorliegen (z.B. verschiedene Estergruppierungen), die selektiv aktiviert werden können und den sektionellen Aufbau des Dendrimers gestatten.
- Heterogene, geschützte Funktionalitäten in der Peripherie sind ebenfalls möglich (z.B. Esterfunktionen in Gegenwart geschützter primärer Amine), die selektiv aktiviert werden können und den sektionellen Aufbau des Dendrimers mit strukturell unterschiedlichen Verzweigungspunkten erlauben.

Durch das erfindungsgemäße Verfahren sind peptoidische, depsipeptidische und bevorzugt chimäre peptid-peptoidische Dendrimere sowie entsprechende Janus-Dendrimere, welche durch multiple iterative Mehrkomponentenreaktionen, insbesondere UGI- bzw. PASSERINI-Mehrkomponentenreaktionen, erhältlich.

Hierzu gehören peptid-peptoidische Dendrimere erster Generation, sofern sie auf einer Kerneinheit "nullter Generation" beruhen. Kerneinheiten sind "nullter Generation", wenn die Kerneinheit nicht aus einer Ugi- oder Passerini-Typ-Reaktion hervorgeht. Solche Kerneinheiten sind klassische polyfunktionelle Kerneinheiten von Dendrimeren oder klassiche kommerzielle Dendrimere selbst, die Ugi-reaktive Gruppen, bevorzugt Carbonsäure- oder Aminofunktionen, an der Oberfläche tragen.

Insbesondere gehören hierzu peptid-peptoidische Dendrimere zweiter, dritter, vierter und weiterer Generationen, wie sie nachfolgend in den Beispielen angegeben sind. Ebenso gehören dazu Janus-Dendrimere, wie sie nachfolgend in den Beispielen angegeben sind.

Im Rahmen der vorliegenden Erfindung wurde festgestellt, dass die durch das erfindungsgemäße Verfahren erhältlichen peptid-peptoidischen Dendrimere als pharmazeutisch verwendbare Produkte oder biologische Hilfsmittel eingesetzt werden können. Insbesondere können diese peptoidischen Dendrimere in folgenden Bereichen eingesetzt werden: *Drug-Delivery, Imaging,* Protein-Labeling und - Seperation, *DNA*/*RNA-Delivery,* Oberflächen-, Zell- und Gewebeerkennung und als Makroamphiphil.

Die folgenden Beispiele dienen dazu, die vorliegende Erfindung weiter zu veranschaulichen, ohne diese in irgendeiner Weise einzuschränken.

### Synthesesepuenz für Dendrimer der zweiten Generation 7 als Ausgangspunkt für weitere Synthesen

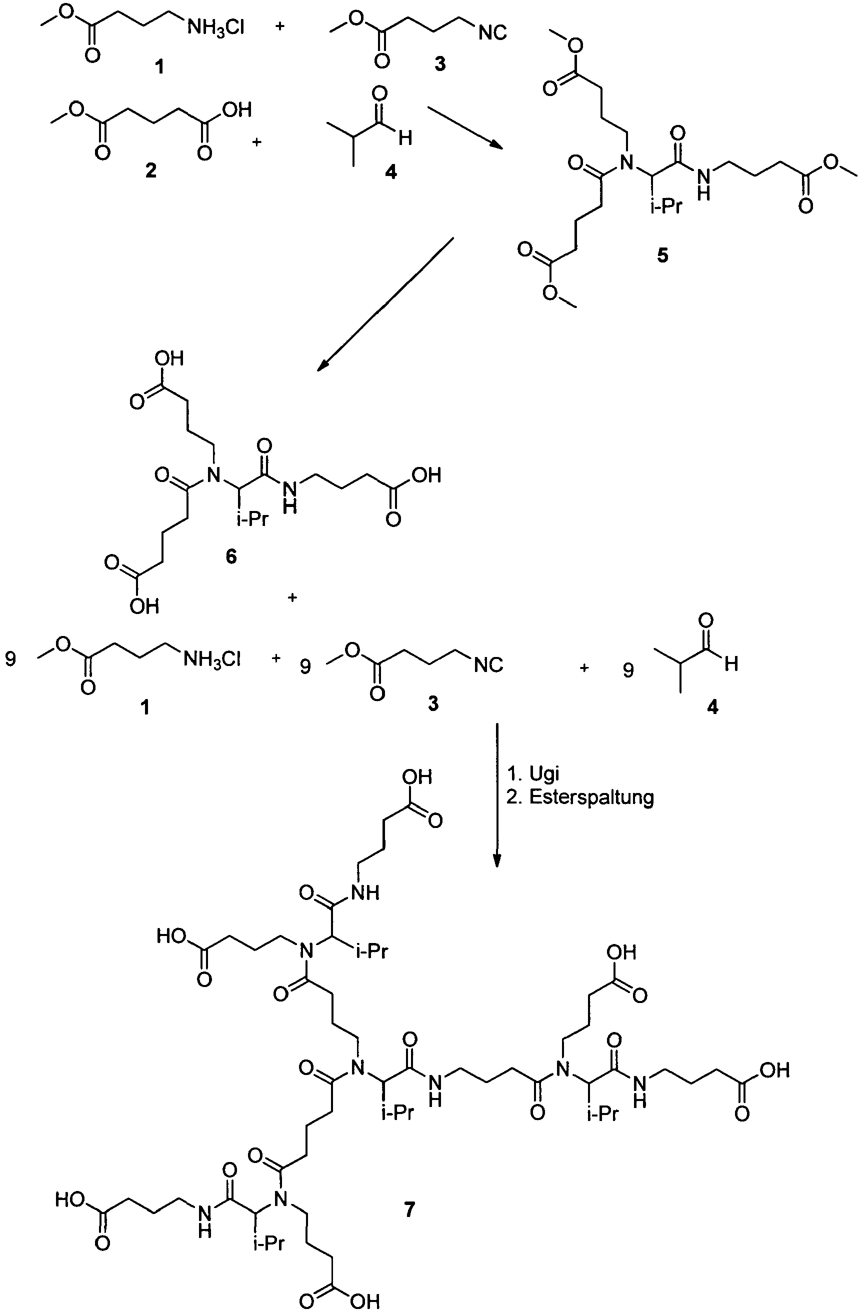

### Methyl 4-(chloramino)butanoat 1

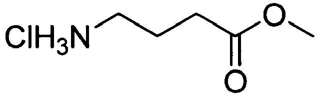

(20.0 g, 193 mmol) γ-Aminobuttersäure wurden in 320 mL Methanol gelöst und mittels Eisbad auf 0°C abgekühlt. Anschließend wurde mit einem Tropftrichter Thionylchlorid (43.5 mL, 600 mmol) zugetropft. Das Eisbad wurde entfernt und nach Erwärmung auf Raumtemperatur über Nacht gerührt.
Die flüchtigen Bestandteile wurden am Rotationsverdampfer entfernt, anschließend wurden 300 mL Et₂O zugegeben und für 30 min bei -30°C aufbewahrt. Der Feststoff wurde abfiltriert und gründlich mit Et₂O gewaschen. Nach Trocknen am Vakuum wurde ein weißer Feststoff (29.0 g, 97%) erhalten.
MS (ESI): [M+H]⁺ = 118.6

### Formamid von Methyl 4-(chloramino)butanoat

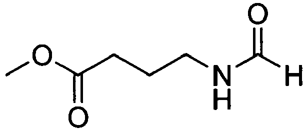

Methyl 4-(chloramino)butanoat **1** (15.0 g, 98.0 mol) wurde in 100 mL Trimethylorthoformiat gelöst und 4 Stunden unter Rückfluss erhitzt. Das Lösungsmittel wurde am Rotationsverdampfer entfernt. Das Produkt wurde als weißer Feststoff (14.2 g, quant.) erhalten.
¹H-NMR (CDCl₃): δ [ppm] = 8.16 (s, 1 H, C**H**O); 3.68 (s, 3H, O-C**H**₃); 3.33 (m, 2H, C**H**₂); 2.40 (t, 2H, ³*J*=7Hz, C**H**₂); 1.87 (q, 2H, ³*J*= 7Hz, C**H**₂).

### 4-Isocyanomethylbutyrat 3

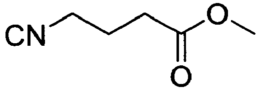

Das Formamid von Methyl 4-(chloramino)butanoat (12.9 g, 89.0 mol) wurde in 300 mL trockenem CH₂Cl₂ vorgelegt. Anschließend wurde Diisopropylamin (37.0 mL, 266 mmol) zugegeben. Die Lösung wurde auf 0°C abgekühlt (Eisbad). Anschließend wurde Phosphorylchlorid (9.76 mL, 107 mmol) langsam zugetropft. Nach beendeter Zugabe wurde die Lösung 2 h bei RT gerührt.
Die Reaktion wurde durch Zugabe von 20 g Na₂CO₃, gelöst in 100 mL H₂O, beendet. Nach 30 min Rühren bei RT wurde die Reaktionslösung mit 100 mL CH₂Cl₂, sowie 100 mL H₂O verdünnt und anschließend 3x mit je 100 mL CH₂Cl₂ extrahiert. Die vereinigten organischen Phasen wurden über Na₂SO₄ getrocknet und am Vakuum eingeengt. Säulenchromatographische Aufreinigung mit CH₂Cl₂/MeOH (9.75/.025, v/v) lieferte ein dunkelbraunes Öl (8.30 g, 73%).
¹H-NMR (CDCl₃): δ [ppm] = 3.70 (s, 3H, O-C**H**₃); 3.51 (m, 2H, CH₂); 2.52 (t, 2H, ³*J*=7Hz, C**H**₂); 1.87 (m, 2**H**, CH₂).

### Synthese erste Generation Methylester-funktionalisiert (5)

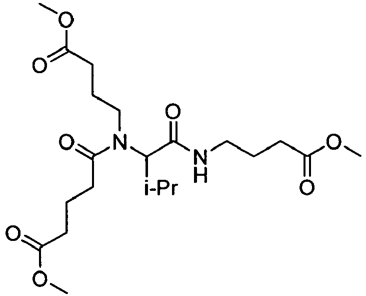

Methyl 4-(chloramino)butanoat **1** (2.00 mg, 13.0 mmol) und Isobutyraldehyd **4** (1.18 mL, 13.0 mmol) wurden zu einer Lösung von Triethylamin (1.8 mL, 13.0 mmol) in 50 mL Methanol gegeben. Die Lösung wurde 2 h bei RT gerührt. Anschließend wurde Methyl-4-isocyanobutanoat 3 (1.65 g, 13.0 mmol) und Monomethylglutarat 2 (1.63 mL, 13.0 mmol) zugegeben und es wurde über Nacht bei RT gerührt. Die Lösung wurde am Rotationsverdampfer eingeengt und der Rückstand säulenchromatographisch (MeOH/EE, 1/39) gereinigt. Das Produkt **5** wurde als farbloses Öl erhalten (5.07 g, 88%).
MS (ESI): [M+Na]⁺ = 467.4

### Synthese erste Generation Carboxy-funktionalisiert (6)

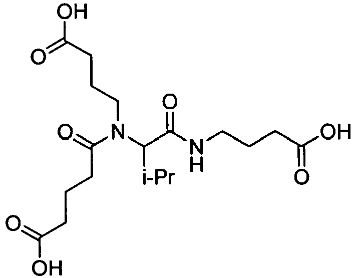

Erste Generation Methylester-funktionalisiert 5 (2.50 g, 5.80 mmol) wurde in 75 mL THF/H₂O (2/1) gelöst und mit einem Eisbad auf 0°C abgekühlt. Anschließend wurde Lithiumhydroxid Monohydrat (1.82 g, 43.4 mmol) zugegeben. Das Eisbad wurde entfernt und es wurde bei RT über Nacht gerührt. Die Lösung wurde mit ges. NaHSO₄ stark angesäuert und 3x mit je 150 mL Ethylacetat extrahiert. Die vereinigten organischen Phasen wurden über Na₂SO₄ getrocknet und am Rotationsverdampfer eingeengt. Das Produkt 6 wurde als farbloses Öl erhalten (2.30 mg, 98%).
MS (ESI): [M+H]⁺ = 401.3

### Synthese zweite Generation Methylester-funktionalisiert (7a)

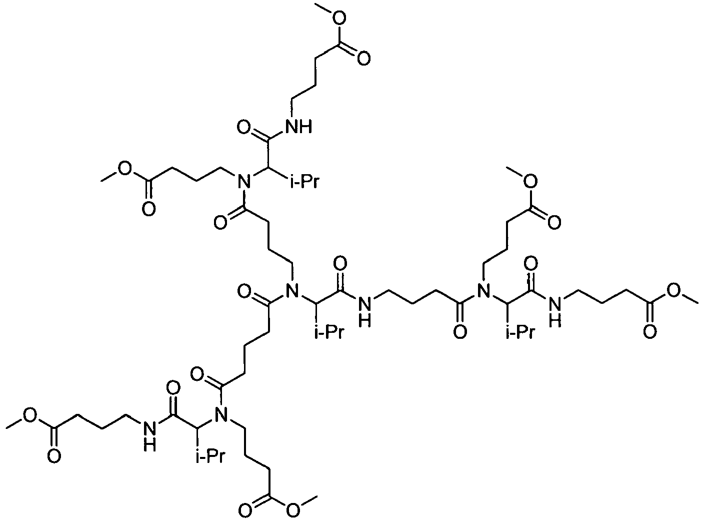

Methyl 4-(chloramino)butanoat **1** (1.00 g, 7.50 mmol) und Isobutyraldehyd **4** (0.69 mL, 7.50 mmol) wurden zu einer Lösung von Triethylamin (1.04 mL, 7.50 mmol) in 50 mL Methanol gegeben. Die Lösung wurde 2 h bei RT gerührt. Anschließend wurde Methyl-4-isocyanobutanoat **3** (1.15 mg, 7.50 mmol) und **6** (1.00 g, 2.50 mmol) zugegeben und es wurde über Nacht bei RT gerührt. Die Lösung wurde am Rotationsverdampfer eingeengt und der Rückstand säulenchromatographisch (MeOH/EE, 1/19) gereinigt. Das Produkt wurde als farbloses Öl erhalten (2.89 g, 89%).
MS (ESI): [M+Na]⁺ = 1320.1

### Synthese zweite Generation Carboxylat-funktionalisiert (7)

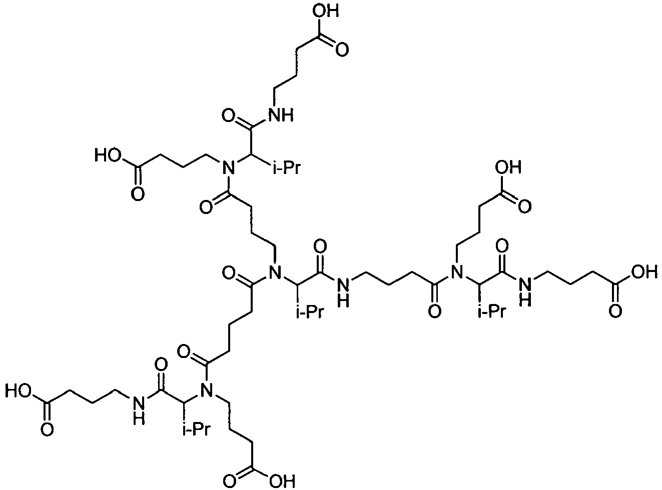

Zweite Generation Methylester-funktionalisiert (2.73 g, 2.11 mmol) wurde in 120 mL THF/H₂O (2/1) gelöst und mit einem Eisbad auf 0°C abgekühlt. Anschließend wurde Lithiumhydroxid Monohydrat (1.32 g, 31.6 mmol) zugegeben. Das Eisbad wurde entfernt und es wurde bei RT über Nacht gerührt. Die Lösung wurde mit ges. NaHSO₄ stark angesäuert und 3x mit je 150 mL Ethylacetat extrahiert. Die vereinigten organischen Phasen wurden über Na₂SO₄ getrocknet und am Rotationsverdampfer eingeengt. Das Produkt **7** wurde als farbloses Öl erhalten (2.40 mg, 94%).
MS (ESI): [M+Na]⁺ = 1235.9

### Synthese 3. Generation Methylester-funktionalisiert (8)

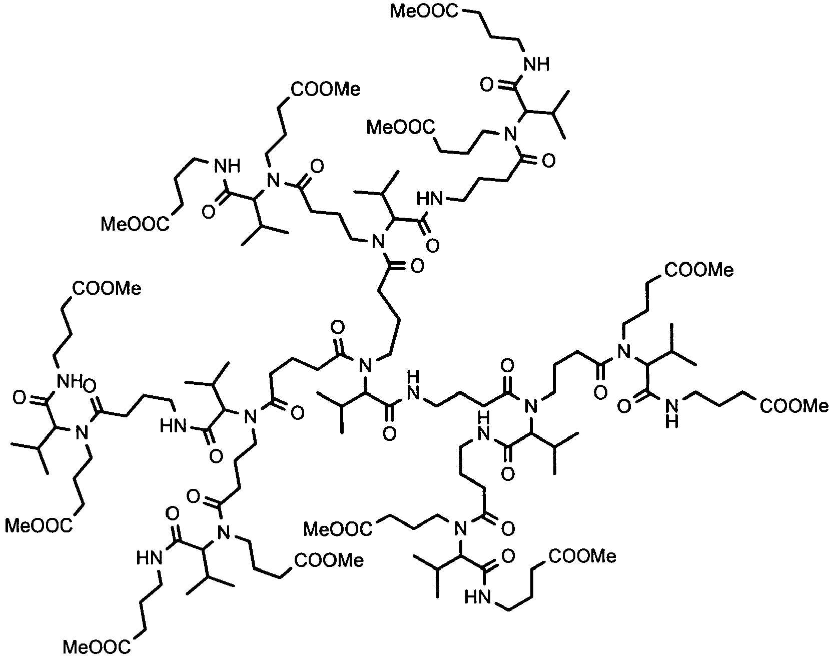

Methyl 4-(chloramino)butanoat 1 (553 g, 3.60 mmol) und Isobutyraldehyd 4 (0.33 mL, 3.60 mmol) wurden zu einer Lösung von Triethylamin (0.50 mL, 3.60 mmol) in 20 mL Methanol gegeben. Die Lösung wurde 2 h bei RT gerührt. Anschließend wurde Methyl-4-isocyanobutanoat 3 (458 mg, 3.60 mmol) und 7 (480 mg, 0.39 mmol) zugegeben und es wurde über Nacht bei RT gerührt. Die Lösung wurde am Rotationsverdampfer eingeengt und der Rückstand säulenchromatographisch (MeOH/EE, 1/9) gereinigt. Das Produkt 8 wurde als farbloses Öl erhalten (1.00 g, 89%).
MS (ESI): [M+2Na]²⁺ = 1524.9

### Allyl 4-(paratoluolsulfonylamino)butanoat 9

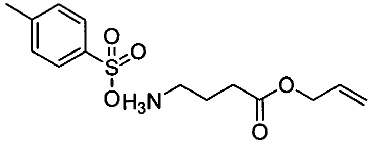

γ-Aminobuttersäure (25.8 g, 250 mmol), Allylalkohol (100 mL, 1.50 mol) und *p-*Toluolsulfonsäure (48.5 g, 255 mmol) wurden in 100 mL Toluol suspendiert und mit einem Wasserabscheider unter Rückfluss für 16 h erhitzt. Anschließend wurde die orangefarbene Lösung unter vermindertem Druck eingeengt. Nach vollständiger Kristallisation des Rückstandes wurde dieser gründlich mit Diethylether gewaschen (4 x 300 ml). Das Produkt wurde als farbloser Feststoff erhalten (77.8 g, 99%).
¹H-NMR (CD₃OD, 300 MHz): δ = 1.92 (quint., *J*=7.5 Hz, 2H, CH₂), 2.36 (s, 3H, CH₃), 2.46 (t, *J*=7.3 Hz, 2H, CH₂), 2.96 (t, *J*=7.7 Hz, 2H, CH₂), 4.58 (dt, *J*=5.8, 1.3 Hz, 2H, CH₂), 5.18-5.34 (m, 2H, CH₂), 5.86-6.00 (m, 1 H, CH), 7.23 (d, *J*=7.7 Hz, 2H, CH), 7.71 (d, *J*=8.1 Hz, 2H, CH) ppm.

### Synthese 3. Generation Allylester/Benzylester-funktionalisiert (orthogonal geschützte Oberfläche) (10)

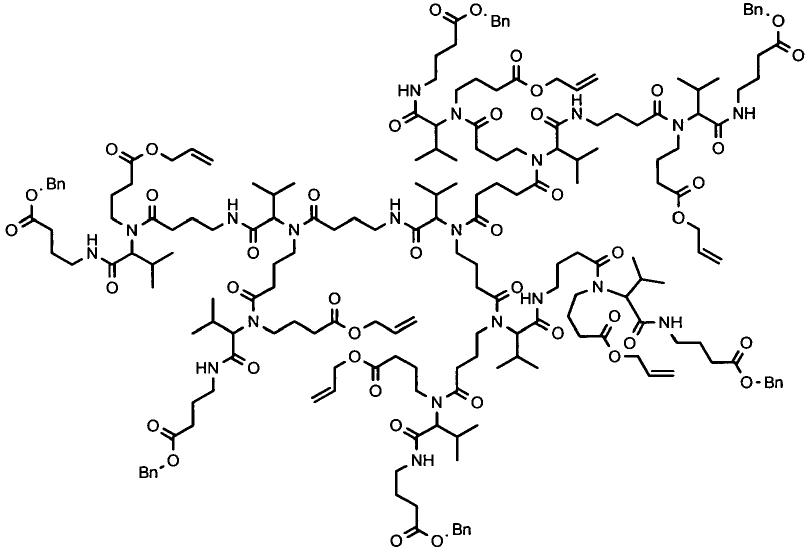

Allyl 4-(paratoluolsulfonylamino)butanoat 9 (233 mg, 0.74 mmol) und Isobutyraldehyd 4 (67.5 µL, 0.74 mmol) wurden zu einer Lösung von Triethylamin (103 µL, 0.74 mmol) in 15 mL Methanol gegeben. Die Lösung wurde 2 h bei RT gerührt. Anschließend wurde Benzyl-4-isocyanobutanoat (151 mg, 0.74 mmol) und 7 (100 mg, 0.08 mmol) zugegeben und es wurde über Nacht bei RT gerührt. Die Lösung wurde am Rotationsverdampfer eingeengt und der Rückstand säulenchromatographisch (MeOH/EE, 1/39) gereinigt. Das Produkt 10 wurde als farbloses Öl erhalten (215 mg, 73%).
MS (ESI): [M+2Na]²⁺ = 1830.9

### Synthese 3. Generation Allylester/Benzylester-funktionalisiert (11)

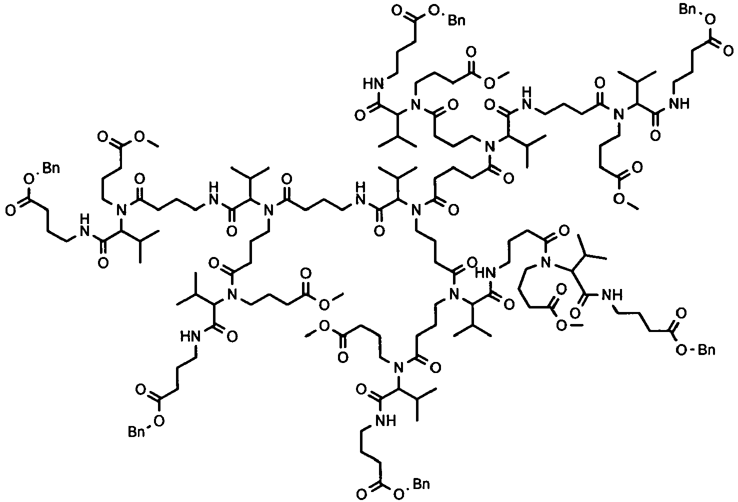

Methyl 4-(chloramino)butanoat 1 (114 mg, 0.74 mmol) und Isobutyraldehyd 4 (67.5 µL, 0.74 mmol) wurden zu einer Lösung von Triethylamin (103 µL, 0.74 mmol) in 15 mL Methanol gegeben. Die Lösung wurde 2 h bei RT gerührt. Anschließend wurde Benzyl-4-isocyanobutanoat (151 mg, 0.74 mmol) und 7 (100 mg, 0.08 mmol) zugegeben und es wurde über Nacht bei RT gerührt. Die Lösung wurde am Rotationsverdampfer eingeengt und der Rückstand säulenchromatographisch (MeOH/EE, 1/39) gereinigt. Das Produkt **11** wurde als farbloses Öl erhalten (214 mg, 75%).
MS (ESI): [M+Na]²⁺ = 1752.8

### Synthese von Dendrimeren mit Aminoberfläche

Synthese von Benzylphenylcarbonat (Synthesis, 2002, 15, 2195-2202)

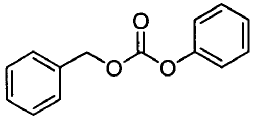

Benzylalkohol (10.8 g, 100.0 mol) wurde in 100 mL CH₂Cl₂ gelöst. Die Lösung wurde auf 0°C abgekühlt und Phenylchloroformiat (15.7 g, 100 mol) wurde langsam zugetropft. Die Lösung wurde über Nacht bei RT gerührt. Nach Zugabe von 100 mL H₂O wurde 2x mit je 100 mL 2 M H₂SO₄ gewaschen. Die organische Phase wurde abgetrennt und über Na₂SO₄ getrocknet. Das Lösungsmittel wurde am Vakuum entfernt. Es wurde einer farblose Flüssigkeit (22.5 g, 99%) erhalten.
¹H-NMR (CDCl₃, 300 MHz): δ = 7.45-7.31 (m, 6H, CH, aromat.), 7.25-7.14 (m, 4H, CH, aromat.), 5.25 (s, 2H, C**H**₂, benzyl.).

### Benzyl-3-aminopropylcarbamat (12) (Synthesis, 2002, 15, 2195-2202)

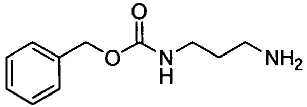

Zu einer Lösung von 1,3-Propandiamin (6.50 g, 87.0 mol) in 250 mL abs. EtOH wurde Benzylphenylcarbonat (20.0 g, 87.0 mmol) gegeben. Die Lösung wurde über Nacht gerührt. Es bildete sich ein weißer Niederschlag. Das Lösungsmittel wurde am Rotationsverdampfer entfernt, der Rückstand wurde mit 100 mL dest. H₂O aufgenommen. Anschließend wurde mit 2 M HCl auf ph∼1-2 angesäuert und 4 x mit je 250 mL CH₂Cl₂ extrahiert. Die wässrige Phase wurde mit 2 M NaOH stark alkalisch gemacht und 4 x mit je 250 mL CH₂Cl₂ extrahiert. Die vereinigten organischen Phasen wurden über Na₂SO₄ getrocknet, anschließend wurde das Lösungsmittel am Rotationsverdampfer entfernt. Das Produkt wurde als zähflüssige, weiße Masse (4.70 g, 26%) erhalten.
ESI-MS: [M+H]⁺ = 209.1

### Benzyl [3-(formylamino)propyl]carbamat

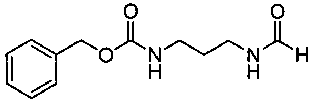

Benzyl-3-aminopropylcarbamat 12 (8.00 g, 36.0 mmol) wurde in 250 mL Ethylformiat gelöst. Die Lösung wurde 4 h unter Rückfluss erhitzt. Nach vollständiger Umsetzung des Amins wurde die Lösung am Rotationsverdampfer bis zur Trockne eingeengt. Das Formamid wurde als farbloses Öl (8.90 g, 99%) erhalten.
ESI-MS: [M+Na]⁺ = 259.2

### Benzyl-3-isocyanopropylcarbamat (13)

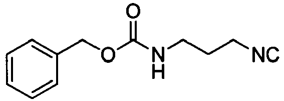

Zu einer Lösung von Benzyl [3-(formylamino)propyl]carbamat (15.8 g, 67.0 mmol) in 300 mL trockenem CH₂Cl₂ wurde Diisopropylamin (29.6 mL, 211 mmol) gegeben. Die Lösung wurde mittels Eisbad auf 0°C abgekühlt. Anschließend wurde POCl₃ (7.36 mL, 80.0 mmol) langsam zugetropft. Nach Erwärmen auf Raumtemperatur wurde für weitere 3 h gerührt. Nach Zugabe von 20 g Na₂CO₃, gelöst in 100 mL dest. H₂O wurde nochmals für 30 min bei RT gerührt. Anschließend wurde mit 100 mL CH₂Cl₂, sowie 100 ml dest. H₂O verdünnt und die wässrige Phase 3x mit je 150 mL CH₂Cl₂ extrahiert. Die vereinigten organischen Phasen wurden über Na₂SO₄ getrocknet und am Rotationsverdampfer eingeengt. Nach säulenchromatographischer Aufreinigung (MeOH/CH₂Cl₂, 1/9, v/v) wurde eine dunkelbraune Flüssigkeit (13.0 g, 89%) erhalten.
ESI-MS: [M+Na]⁺ = 241.1

### 4-(((Benzyloxy)carbonyl)amino)butansäure (14)

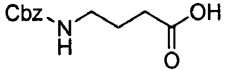

Zu einer Lösung aus γ-Aminobuttersäure (10.0 g, 103 mmol) in 150 mL CH₂Cl₂ und 400 mL 5% Na₂CO₃ wurde Benzyloxycarbonylchlorid (17,4 mL, 103 mmol) langsam zugetropft. Die Lösung wurde 6 h bei Raumtemperatur gerührt und anschließend mit 10% HCl angesäuert. Die Lösung wurde mit Ethylacetat extrahiert (4 x 200 mL). Die vereinigten organischen Phasen wurden über Na₂SO₄ getrocknet und am Rotationsverdampfer eingeengt. Das Produkt **14** wurde als farbloses Öl (19.0 g, 78%) erhalten.
MS (ESI): [M-H]⁻ = 236.0

### Benzyl-[4-(4-{[(benzyloxy)carbonyl]amino}butanoyl)-5-(1-methylethyl)-6,12-dioxo-14-phenyl-13-oxa-4,7,11-triazatetradec-1-yl]carbamat (15a)

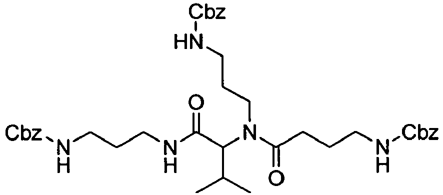

Zu einer Lösung von Benzyl-3-aminopropylcarbamat 12 (208 mg, 1.00 mmol) in MeOH wurde Isobutyraldehyd **4** (72 mg, 1.00 mmol) hinzugegeben. Diese Lösung wurde für 2 Stunden bei Raumtemperatur gerührt. Anschließend wurden Benzyl-3-isocyanopropylcarbamat **13** (218 mg, 1.00 mmol) und 4-[(Benzyloxy)carbonyl]-aminobutansäure **14** (237 mg, 1.00 mmol) hinzugefügt. Die Lösung wurde über Nacht bei Raumtemperatur gerührt. Das Lösungsmittel wurde im Rotationsverdampfer entfernt und der Rückstand wurde säulenchromatographisch (MeOH/CH₂Cl₂, 1/19, v/v) aufgereinigt. Das Produkt **15a** wurde als farbloses Öl (623 mg, 87%) erhalten.
MS (ESI): [M+H]⁺ = 718.7, [M+Na]⁺ = 740.7

### N-(4-aminobutanoyl)-N,N²-bis(3-aminopropyl)valinamid (15)

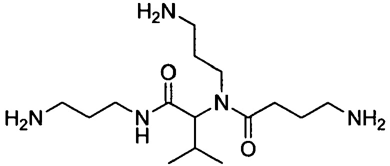

Zu einer Lösung von Verbindung **15** (546 mg, 0.76 mmol) in MeOH wurde Pd(OH)₂/C (55 mg,) zugegeben. Die Lösung wurde 3 x entgast und mit Wasserstoff aus einem Ballon belüftet. Unter Wasserstoffatmosphäre wurde die Lösung über Nacht stark gerührt. Die Lösung wurde über ein Celitepad filtriert und das Lösungsmittel am Rotationsverdampfer entfernt. Das Amin 15 wurde als farbloses Öl (231 mg, 97%) erhalten. MS (ESI): [M+H]⁺ = 316.1, [M+Na]⁺ = 338.4

### Dendrimer zweite Generation (16a)

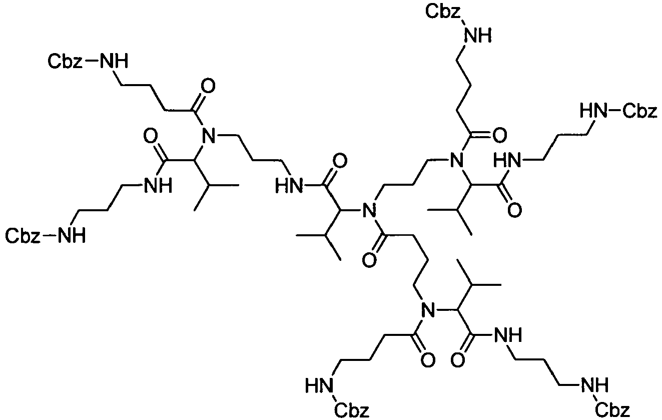

Zu einer Lösung von Triamin **15** (190 mg, 0.60 mmol) in 5 mL MeOH wurde Isobutyraldehyd **4** (129.8 mg, 1.80 mmol) hinzugegeben. Die Lösung wurde für 2 Stunden bei Raumtemperatur gerührt. Anschließend wurden Benzyl-3-isocyanopropylcarbamat 13 (393 mg, 1.80 mmol) und 4-[(Benzyloxy)carbonyl]-aminobutansäure **14** (428 mg, 1.80 mmol) hinzugefügt. Die Lösung wurde über Nacht bei Raumtemperatur gerührt. Das Lösungsmittel wurde im Rotationsverdampfer eingeengt und der Rückstand wurde säulenchromatographisch (MeOH/CH₂Cl₂, 1/19, v/v) gereinigt. Das Produkt **16a** wurde als farbloses Öl (790 mg, 71%) erhalten.
MS (ESI): [M+2Na]²⁺ = 945.2, [M+Na]⁺ = 1867.9

### Funktionalisiertes Dendrimer zweite Generation (16)

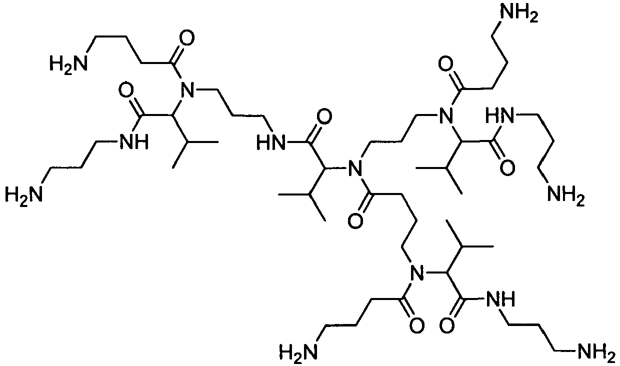

Zu einer Lösung von Verbindung **16a** (710 mg, 0.39 mmol) in 5 mL MeOH wurde Pd(OH)₂/C (70 mg) zugegeben. Die Lösung wurde 3 x entgast und mit Wasserstoff aus einem Ballon belüftet. Unter Wasserstoffatmosphäre wurde die Lösung über Nacht stark gerührt. Die Lösung wurde über ein Celitepad filtriert und das Lösungsmittel im Rotationsverdampfer entfernt. Das Amin **16** wurde als farbloses Öl (400 mg, 97%) erhalten.
MS (ESI): [M+2H]²⁺ = 521.0, [M+H]⁺ = 1039.9

### Wechsel der funktionellen Oberflächengruppen

Ein Dendrimer mit Aminooberfläche kann auch mit einer carboxyfunktionalisierten 2. Dendrimergeneration aufgebaut werden. Dazu wird eine UGI-Reaktion mit den Verbindungen **13, 12, 4** und **7** durchgeführt. Als Produkt wird das Dendrimer **17** erhalten. Hier kann gezeigt werden, dass ein Wechsel zwischen verschiedenen reaktiven Gruppen in den einzelnen Generationen möglich ist.

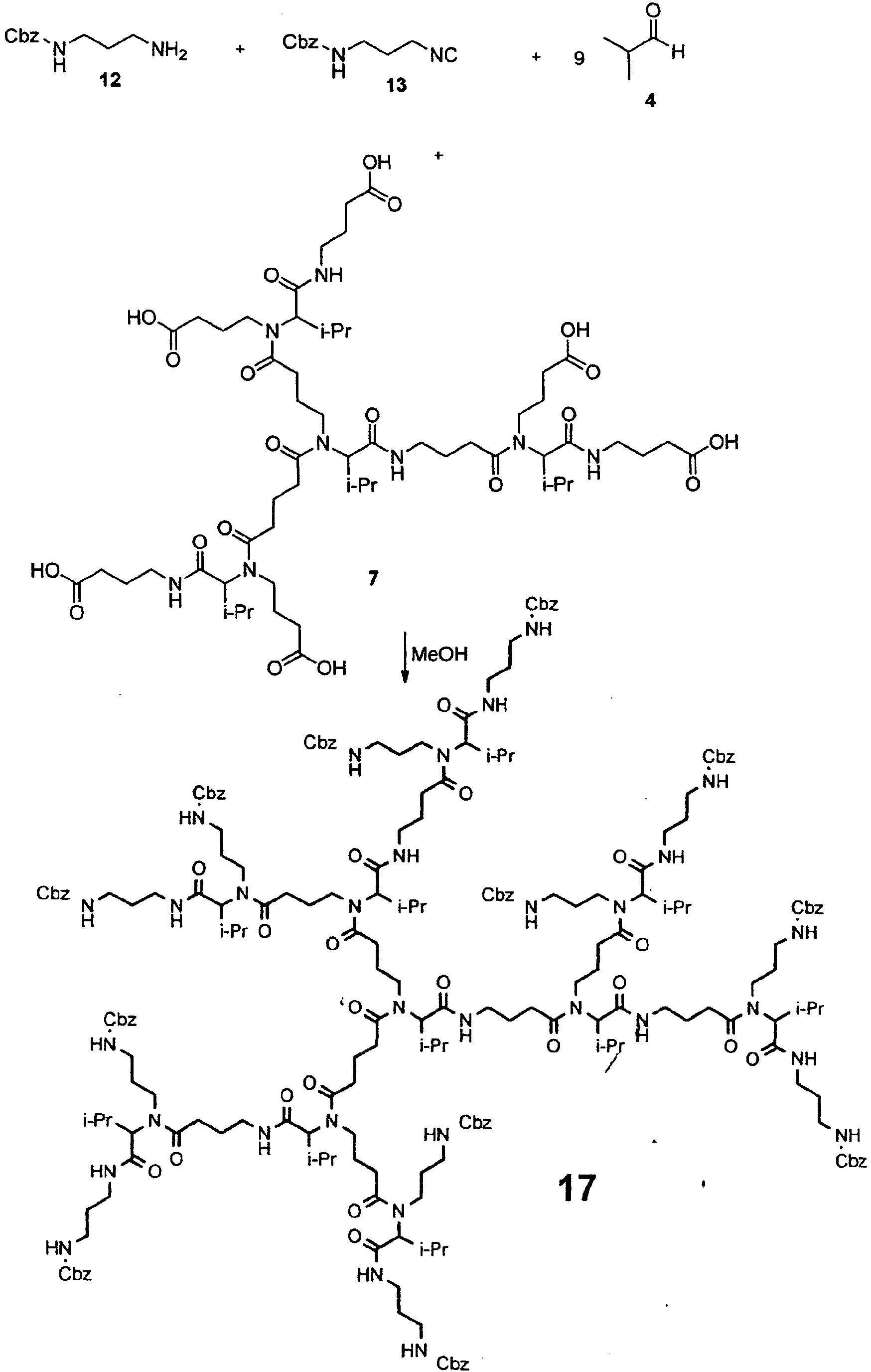

### Synthese von Dendrimer der 3. Generation (17)

Zu einer Lösung von Benzyl-3-aminopropylcarbamat **12** (625 mg, 3.00 mmol) in 5 mL MeOH wurde Isobutyraldehyd **4** (274 µL, 3.00 mmol) hinzugegeben. Die Lösung wurde für 2 Stunden bei Raumtemperatur gerührt. Anschließend wurden Benzyl-3-isocyanopropylcarbamat **13** (655 mg, 3.00 mmol), sowie Dendrimer **7** (400 mg, 0.33 mmol) hinzugefügt. Die Lösung wurde 20 h bei RT gerührt. Das Lösungsmittel wurde im Rotationsverdampfer entfernt und der Rückstand säulenchromatographisch (MeOH/EE, 1/20) aufgereinigt. Das Produkt **17** wurde als farbloses Öl (1.13 g, 84%) erhalten.
MS (ESI): [M+3Na]³⁺ = 1388.8

### Synthese von S-t-Bu geschützten Dendrimeren mit Schwefeloberfläche

Ausgehend von der literaturbekannten Verbindung **18** kann ein schwefelfunktonalisiertes Dendrimer der zweiten Generation synthetisiert werden. Als Basis dient die carboxyfunktonalisierte zweite Generation **7.** Die UGI-Reaktion mit jeweils 9 Äquivalenten **18,** Isobutyraldehyd **4,** sowie 9 Äquivalenten *t*-Butylisocyanid **19** und 1 Äquivalent **7** liefert das S-*t*-Bu geschützte schwefelfunktionalisierte Dendrimer **20** der 3. Generation in 89% Ausbeute.

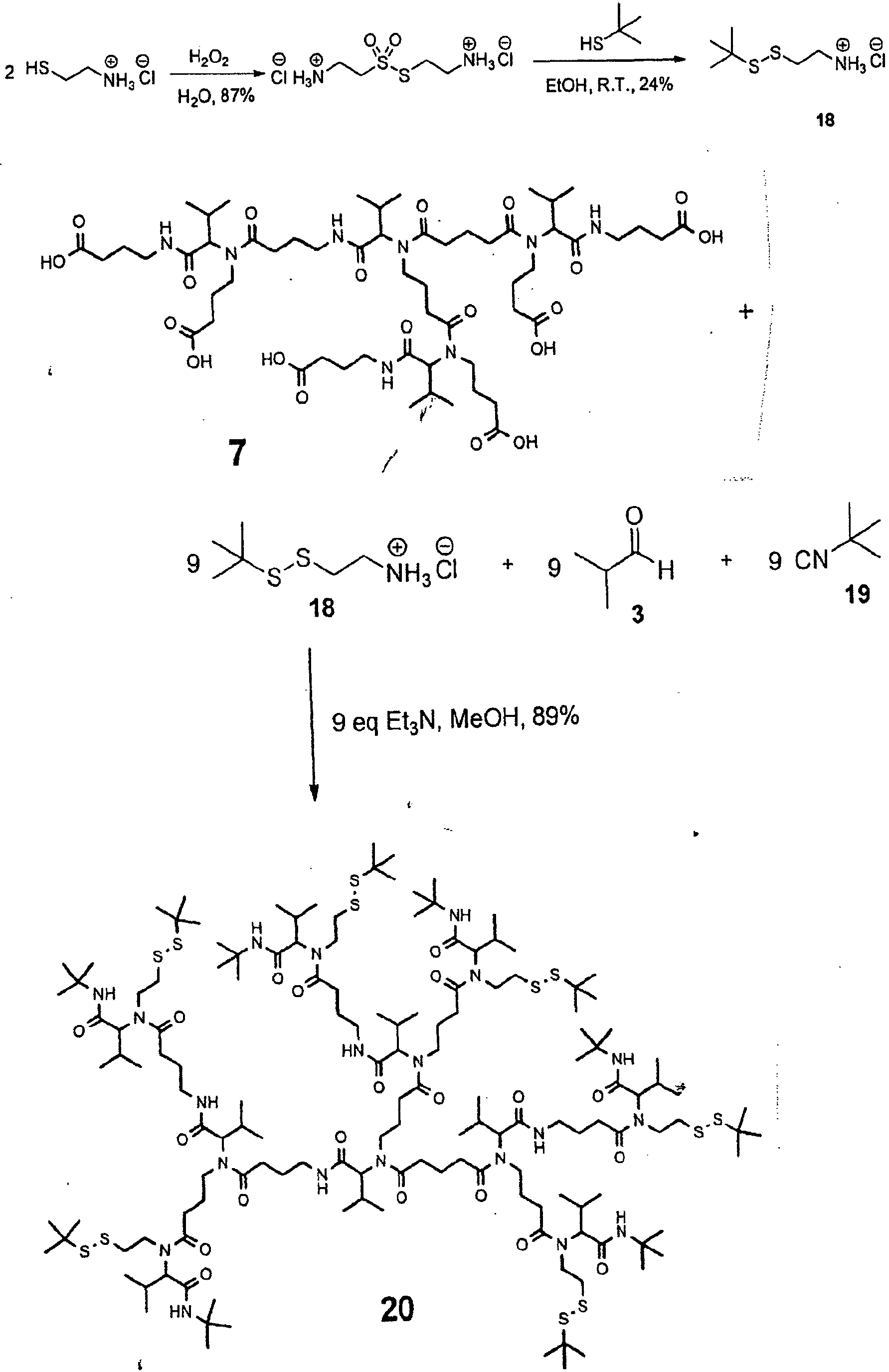

### Synthese von 18 (siehe JACS, 83, 1961, 4416-4417).

Cysteaminhydrochlorid (5.00 g, 44.0 mmol) wurde in 50 mL dest. H₂O gelöst und mit einem Eisbad auf 0°C abgekühlt. Zu dieser Lösung wurde eine 30% H₂O₂-Lösung (7.30 mL, 72.6 mmol) langsam zugetropft. Anschließend wurden einige Kristalle Kl als Katalysator zugesetzt. Die Lösung wurde 20 h bei RT gerührt. Das Lösungsmittel wurde im Rotationsverdampfer entfernt und der Rückstand in 30 ml Eisessig aufgenommen. Nach Lagerung im Kühlschrank für 2 Stunden bildete sich ein farbloser Niederschlag, der abfiltriert und gründlich mit Eisessig gewaschen wurde. Man erhielt das Dihydrochlorid als farblosen Feststoff (5.30 g, 94%), welcher ohne weitere Aufreinigung weiter umgesetzt wurde. Dihydrochlorid (5.30g, 20.6 mmol) wurde in 10 mL dest. H₂O gelöst. Anschließend wurde *t-*BuSH (2.06 mL, 20.6 mmol) in 10 mL EtOH zugegeben und die Lösung wurde 20 h bei RT gerührt. Die Lösung wurde im Rotationsverdampfer eingeengt und der Rückstand mit einer Mischung aus 20 mL dest. H₂O und 20 mL Et₂O aufgenommen. Die Lösung wurde mit NaHCO₃ neutralisiert. Anschließend wurde die Etherphase abgetrennt und mit dest. H₂O gewaschen. Die organische Phase wurde 2 x mit je 6 mL conc. HCl extrahiert. Die saure wässrige Phase wurde im Rotationsverdampfer eingeengt. Das Hydrochlorid **18** wurde als farbloser Feststoff (700 mg, 17%) erhalten.
¹H-NMR (300 MHz, CD₃OD): δ = 1.33 (s, 9H, CH₃), 2.94 (t, 2H, CH₂), 3.22 (t, 2H, CH₂) ppm.

### Synthese von (20)

Hydrochlorid **18** (150 mg, 0.74 mmol), Isobutyraldehyd **4** (68.0 µL, 0.74 mmol) und Et₃N (103 µl, 0.74 mmol) wurden in 5 mL MeOH gelöst und 2 h bei RT gerührt. Anschließend wurde Dendrimer **7** (100 mg, 0.08 mmol) und *t*-Butylisocyanid **19** (84.0 µl, 0.74 mmol) zugegeben. Die Lösung wurde 20 h bei RT gerührt. Das Lösungsmittel wurde im Rotationsverdampfer entfernt und der Rückstand säulenchromatographisch (EE/MeOH, 20/1) aufgereinigt. Das Produkt **20** wurde als farbloser Feststoff (200 mg, 89%) erhalten. MS (ESI): [M+2Na]⁺ = 1537.6

### Synthese eines N-Boc-Serin funktionalisierten Dendrimers

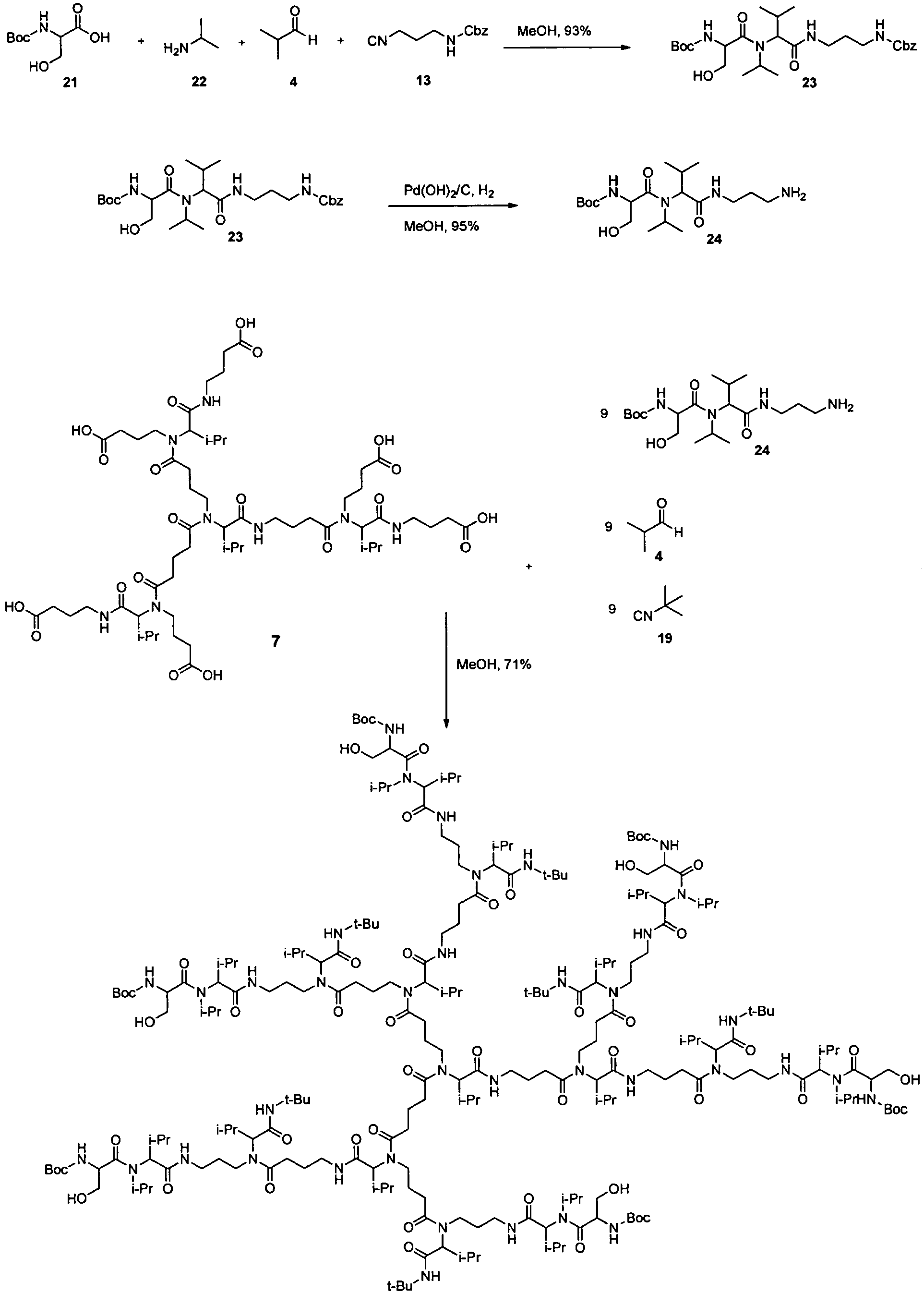

Ausgehend von der carboxyfunktionalisierten 2. Dendrimergeneration **7** kann ein *N-*Boc-Serin funktionalisertes Dendrimer der 3. Generation aufgebaut werden. Zu diesem Zweck wurde *N*-Boc-Serin **21** per UGI-Reaktion mit Isopropylamin **22,** Isobutyraldehyd **4** und dem Isonitril **19** zur Verbindung **23** umgesetzt. Nachfolgende Hydrogenolyse liefert das Amin **24,** welches anschließend mittels der UGI-Reaktion mit **7** zum Dendrimer **25** umgesetzt wird.

### Synthese von UGI-Produkt (23)

Isopropylamin **22** (827 µL, 9.70 mmol) und Isobutyraldehyd **4** (885 µL, 9.70 mmol) wurden in 10 mL MeOH gelöst und 2h bei RT gerührt. *N*-Boc-Ser-OH **21** (2.00 g, 9.70 mmol) und Benzyl-3-isocyanopropylcarbamat **13** (2.10 g, 9.70 mmol) wurden zugegeben und die Lösung wurde für 20 h bei RT gerührt. Anschließend wurde das Lösungsmittel im Rotationsverdampfer entfernt und der Rückstand wurde säulenchromatographisch (EE/Hexan, 4/1) aufgereinigt. Das Produkt **23** wurde als farbloses Öl (4.85 g, 93%) erhalten.
MS (ESI): [M+H]⁺ = 537.6, [M+Na]⁺ = 559.1

### Synthese von Amin (24)

UGI-Produkt **23** (4.35 g, 8.10 mmol) wurde in 50 mL MeOH gelöst. Anschließend wurden 300 mg Pd(OH)₂/C zugegeben. Unter kräftigem Rühren wurde die Lösung 3 x entgast und mit Wasserstoff aus einem Ballon belüftet. Die Lösung wurde 4 h kräftig bei RT gerührt. Anschließend wurde der Katalysator über ein Celite-Pad abfiltriert. Das Lösungsmittel wurde im Rotationsverdampfer eingeengt und das Produkt **24** wurde als farbloses Öl (3.10 g, 95%) erhalten.
ESI-MS: ber. 403.5, gef. 403.7
MS (ESI): [M+H]⁺ = 403.6

### Synthese von Dendrimer (25)

Amin **24** (1.50 g, 3.69 mmol) und Isobutyraldehyd **4** (340 µL, 3.69 mmol) wurden in 20 mL MeOH gelöst und für 2h bei RT gerührt. Anschließend wurden Dendrimer **7** (500 mg, 0.41 mmol) und *t*-Butylisocyanid **19** (422 µL, 3.69 mmol) zugegeben und die Lösung für 20h bei RT gerührt. Das Lösungsmittel wurde im Rotationsverdampfer entfernt und der Rückstand säulenchromatographisch (MeOH/EE, 1/15) aufgereinigt. Das Produkt 25 wurde als farbloses Öl (1.30 g, 71%) erhalten. MS (ESI): [M+3Na]³⁺ = 1505.6, [M+2Na]²⁺ = 2248.7

### Synthese von Aminodendrimeren mit anderen Ugi-Komponenten

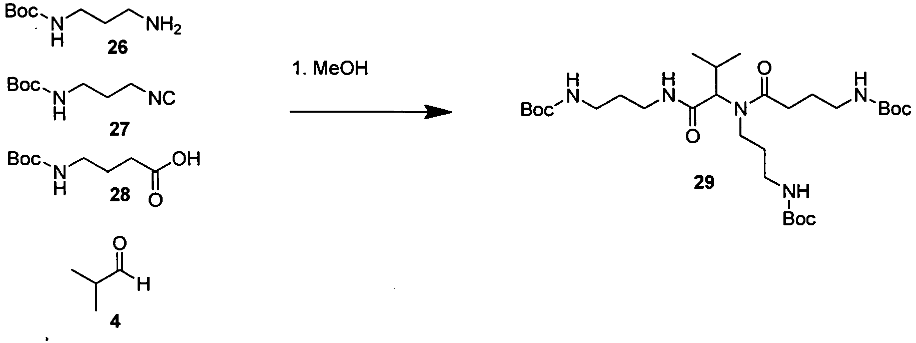

Synthese von *t*-Butyl-3-aminopropylcarbamat **26*** (siehe Synthesis, 2002, 15, 2195-2202).

### Synthese des Formamids von t-Butyl-3-aminopropylcarbamat (26)

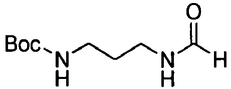

Amin **26*** (8.30 g, 47.0 mmol) wurde in Ethylformiat (150 mL) gelöst und für 3h unter Rückfluss erhitzt. Das Lösungsmittel wurde am Rotationsverdampfer entfernt und das Produkt (9.1 g, 95%) als gelbes Öl erhalten. Das Formamid wurde ohne weitere Aufreinigung weiter umgesetzt. MS (ESI): [M+Na]⁺ = 225.3

### t-Butyl-3-isocyanopropylcarbamat (27)

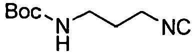

Das Formamid (9.1 g, 45 mmol) wurde in 250 mL trockenem CH₂Cl₂ gelöst und mittels Eisbad auf 0°C abgekühlt. Anschließend wurde Diisopropylamin (18.8 mL, 134 mmol) zugegeben und Phosphorylchlorid (4.89 mL, 53.8 mmol) langsam zugetropft. Die Lösung wurde 2h bei Raumtemperatur gerührt. Anschließend wurde Na₂CO₃ (10.0 g in 100 mL H₂O) zugegeben und für 30 min bei R.T. gerührt. Die Phasen wurden getrennt und die wässrige Phase mit CH₂Cl₂ (3 x 100 mL) extrahiert. Die organischen Phasen wurden über Na₂SO₄ getrocknet und am Rotationsverdampfer bis zur Trockne eingeengt. Der Rückstand wurde säulenchromatographisch (CH₂Cl₂/MeOH, 40/1) gereinigt. Das Produkt **27** wurde als braunes Öl (6.56 g, 79%) erhalten. MS (ESI): [M+Na]⁺ = 207.1

### Synthese von 4-(((t-Butyloxy)carbonyl)amino)butansäure (28)

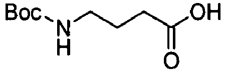

γ-Aminobuttersäure (10.0 g, 97.0 mmol) wurde in 100 mL MeOH gelöst und Triethylamin (84.0 mL, 600 mmol) wurde zugegeben. Boc₂O (43.7 g, 200 mmol) wurde zugegeben und die Lösung wurde über Nacht unter Rückfluss erhitzt. Die Reaktionslösung wurde bis zur Trockne eingeengt und der Rückstand in ges. NaHCO₃ (200mL) gelöst. Die Lösung wurde mit Petrolether (3 x 150 mL) extrahiert. Die wässrige Phase wurde mit 2 M HCl angesäuert und mit Ethylacetat (3 x 150 mL) extrahiert. Die organischen Phasen wurden vereinigt und über Na₂SO₄ getrocknet. Die Lösung wurde am Rotationsverdampfer eingeengt und das Produkt wurde als farbloser Feststoff (16.0 g, 78.8 mmol) erhalten. Schmelzpunkt: 58°C

### Synthese von Boc-geschützter erster Generation (29)

Amin **26** (87.1 mg, 0.50 mmol) und Isobutyraldehyd **4** (46 µL, 0.50 mmol) wurden in 10 mL MeOH gelöst und 2h bei RT gerührt. Carbonsäure **28** (109 mg, 0.50 mmol) und *t-*Butyl-3-isocyanopropylcarbamat **27** (292.1 mg, 0.50 mmol) wurden zugegeben und die Lösung wurde für 20 h bei RT gerührt. Anschließend wurde das Lösungsmittel im Rotationsverdampfer entfernt und der Rückstand wurde säulenchromatographisch (CH₂Cl₂/MeOH, 20/1) aufgereinigt. Das Produkt **29** wurde als farbloses Öl (194 mg, 63%) erhalten. MS (ESI): [M+H]⁺ = 616.4

### Synthese von erster Generation (30)

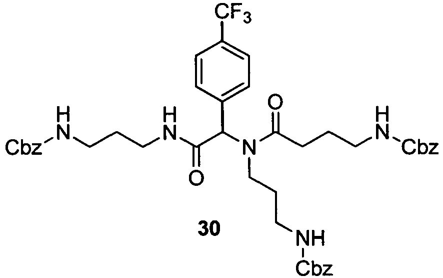

Amin **12** (2.70 g, 13.0 mmol) und *p*-Trifluormethylbenzaldehyd (1.78 mL, 13.0 mmol) wurden in 50 mL MeOH gelöst und 2h bei RT gerührt. Carbonsäure **14** (3.08 g, 13.0 mmol) und Isocyanid **13** (2.80 g, 13.0 mmol) wurden zugegeben und die Lösung wurde für 20 h bei RT gerührt. Anschließend wurde das Lösungsmittel im Rotationsverdampfer entfernt und der Rückstand wurde säulenchromatographisch (EE/PE, 1/4) aufgereinigt. Das Produkt 30 wurde als farbloses Öl (7.32 g, 69%) erhalten.

### Synthese von erster Generation (31)

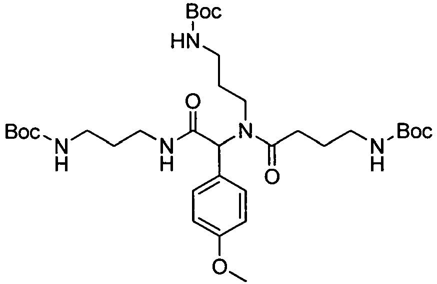

Amin **26** (87.1 mg, 0.50 mmol) und *p*-Methoxybenzaldehyd (60.3 µL, 0.50 mmol) wurden in 10 mL MeOH gelöst und 2h bei RT gerührt. Carbonsäure **28** (109 mg, 0.50 mmol) und *t-*Butyl-3-isocyanopropylcarbamat **27** (292.1 mg, 0.50 mmol) wurden zugegeben und die Lösung wurde für 20 h bei RT gerührt. Anschließend wurde das Lösungsmittel im Rotationsverdampfer entfernt und der Rückstand wurde säulenchromatographisch (CH₂Cl₂/MeOH, 20/1) aufgereinigt. Das Produkt **31** wurde als farbloses Öl (194 mg, 63%) erhalten. MS (ESI): [M+H]⁺ = 680.8

### Synthese von Dendrimeren mit Zuckerfunktionen über Klick-Reaktionen

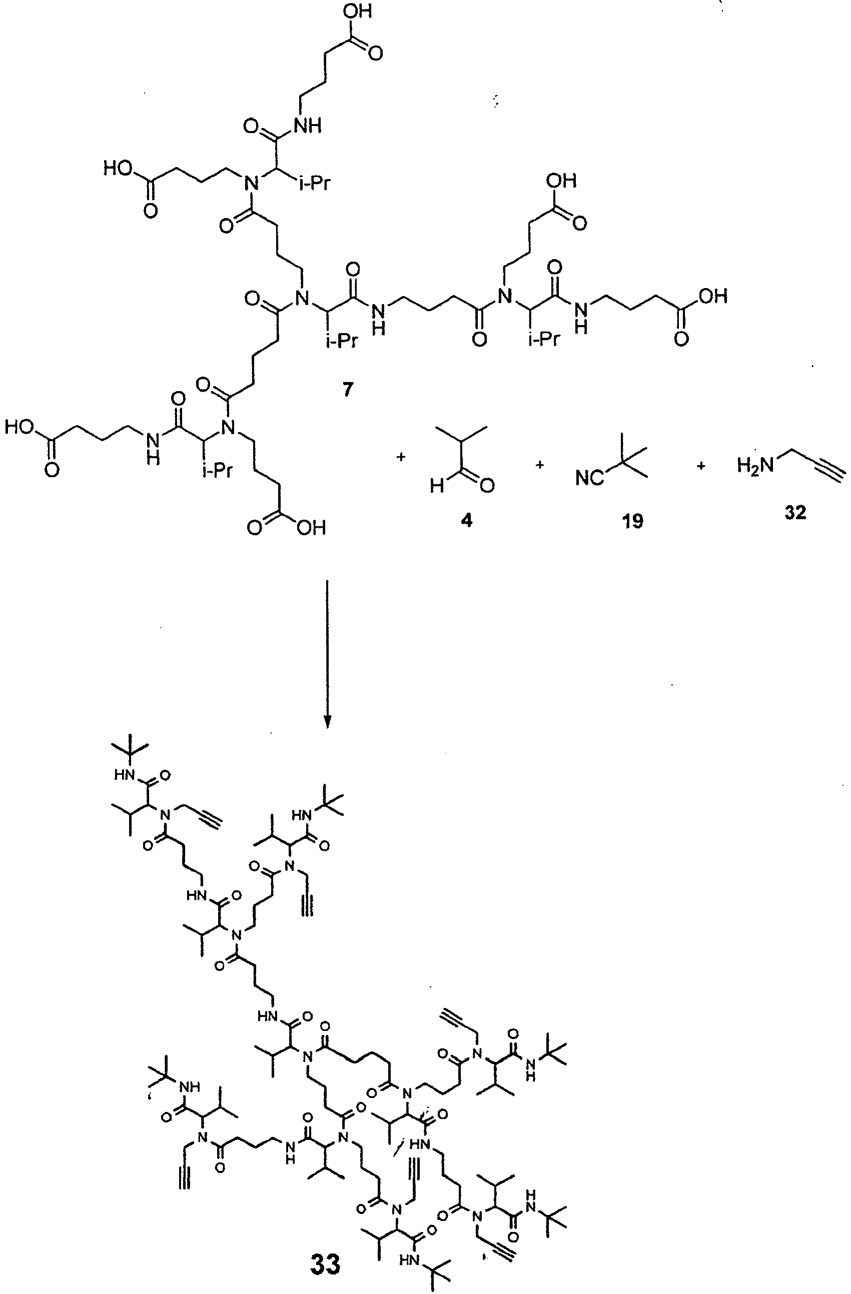

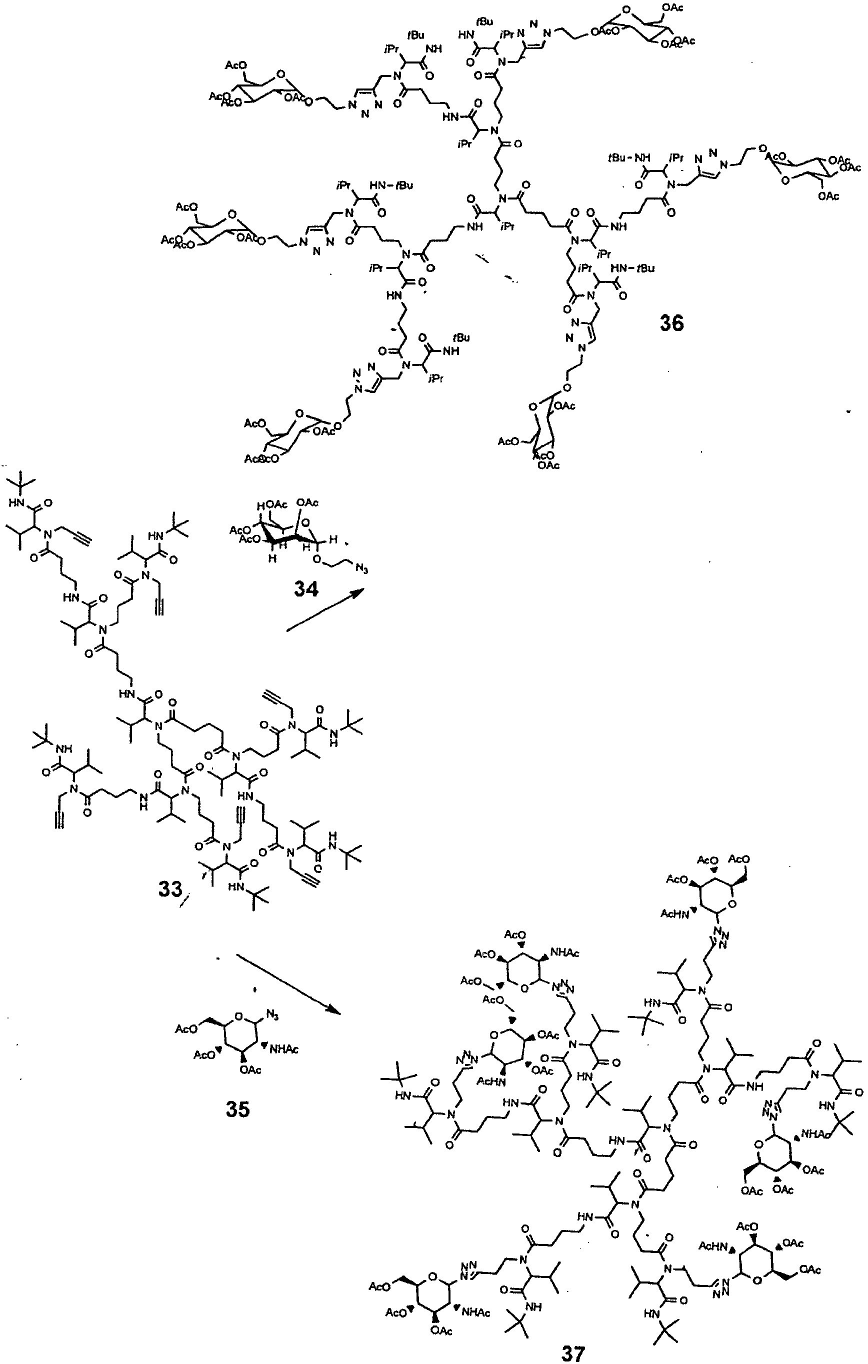

### Synthese von 3. Generation-Alkin-funktionalisiert (33)

Propargylamin **32** (97.0 µl, 1.50 mmol) und Isobutyraldehyd **4** (137 µl, 1.50 mmol) wurden in 5 mL MeOH gelöst und 2 bei R.T. gerührt. Anschließend wurde Dendrimer **7** (200 mg, 165 µmol) und *t*-Butylisocyanid **19** (170 µl, 1.50 mmol) zugegeben. Die Lösung wurde über Nacht bei R.T. gerührt. Das Lösungsmittel wurde am Rotationsverdampfer bis zur Trockne eingeengt und der Rückstand säulenchromatographisch (EE/MeOH, 20/1) aufgereinigt. Das Produkt **33** wurde als gelbes Öl (230 mg, 59%) erhalten. MS (ESI): [M+Na]⁺ = 2390.2

Synthese von Verbindung **34** ist aus der Literatur bekannt: J. Chem. Soc.: Perkin Trans. I, 2001, 823. Verbindung **35** ist kommerziell erhältlich.

### Synthese des Mannose-modifizierten Dendrimers (36)

Dendrimer **33** (190 mg, 0.08 mmol) und Mannosederivat **34** (201 mg, 0.48 mmol) wurden in *t*-BuOH gelöst. Anschließend wurden Cu(II)SO₄ (18.0 mg, 0.10 mmol), gelöst in 2.5 mL H₂O, sowie Natriumascorbat (38.0 mg, 0.20 mmol), gelöst in 5 mL H₂O zugegeben. Die Lösung wurde über Nacht bei R.T. gerührt. Die Reaktionslösung wurde mit H₂O und CH₂Cl₂ (je 10 mL) verdünnt. Die organische Phase wurde abgetrennt und die wässrige Phase mit CH₂Cl₂ (3 x 20 mL) extrahiert. Die vereinigten organischen Phasen wurden über Na₂SO₄ getrocknet und das Lösungsmittel im Rotationsverdampfer eingeengt. Anschließende säulenchromatographische Aufreinigung lieferte das Produkt **36** als farbloses Öl (200 mg, 51%). MS (ESI): [M+3Na]³⁺ = 1647.2

### Synthese des GlucNAc-modifizierten Dendrimers (37)

Dendrimer **33** (190 mg, 0.08 mmol) und *N*-Acetylglucosederivat **35** (180 mg, 0.48 mmol) wurden in *t*-BuOH gelöst. Anschließend wurden Cu(II)SO₄ (18.0 mg, 0.10 mmol), gelöst in 2.5 mL H₂O, sowie Natriumascorbat (38.0 mg, 0.20 mmol), gelöst in 5 mL H₂O zugegeben. Die Lösung wurde über Nacht bei R.T. gerührt. Die Reaktionslösung wurde mit H₂O und CH₂CL₂ (je 10 mL) verdünnt. Die organische Phase wurde abgetrennt und die wässrige Phase mit CH₂CL₂ (3 x 20 mL) extrahiert.

Die vereinigten organischen Phasen wurden über Na₂SO₄ getrocknet und das Lösungsmittel im Rotationsverdampfer eingeengt. Anschließende säulenchromatographische Aufreinigung lieferte das Produkt **37** als farbloses Öl (190 mg, 52%). MS (ESI): [M+3Na]³⁺ = 1556.5

### Synthese von Dendrimeren mit terminalen Hydroxygruppen

Ausgehend von der carboxyfunktionalisierten zweiten Generation **7** kann mit Isobutyraldehyd **4,** *t*-Butylisocyanid **19** und 6-Aminohexanol **38** das Dendrimer der 3. Generation **39** synthetisiert werden.

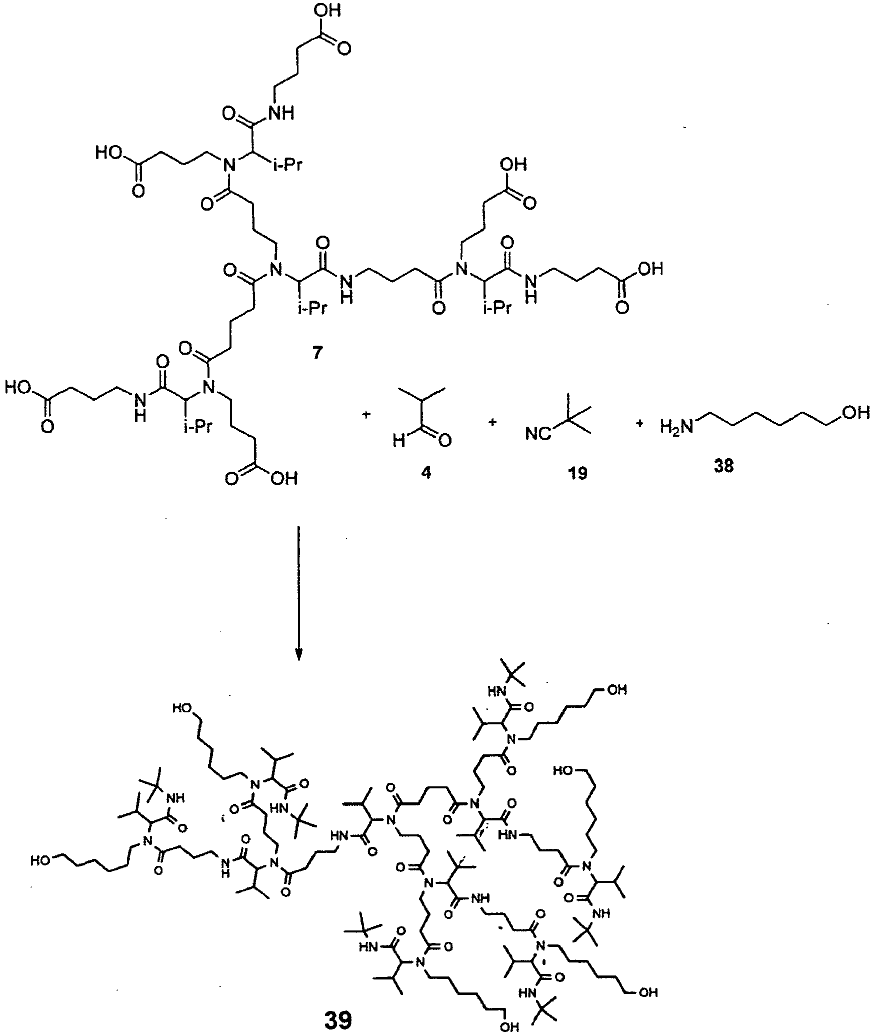

### Synthese von Dendrimer (39)

6-Aminohexanol **38** (522 mg, 4.45 mmol) und Isobutyraldehyd **4** (406 µl, 4.45 mmol) wurden in 10 mL MeOH gelöst und für 2h bei R.T. gerührt. Anschließend wurden Dendrimer **7** (300 mg, 0.25 mmol) und *t*-Butylisocyanid **19** (503 µl, 4.45 mmol) zugegeben. Die Lösung wurde über Nacht bei R.T. gerührt. Die Lösung wurde im Rotationsverdampfer bis zur Trockne eingeengt und der Rückstand säulenchromatograpisch aufgereinigt. Das Produkt wurde als farbloses Öl (588 mg, 86%) erhalten.
MS (ESI): [M+Na]⁺ = 2763.6

### Synthese von Dendrimeren mit Fluoreszenzfarbstoffen

Als Fluoreszenzfarbstoffe wurden 5(6)-Carboxyfluorescein, ein Pyrenderivat und Rhodamin B eingesetzt.

### Synthese von Fluorescein-markierten Dendrimeren:

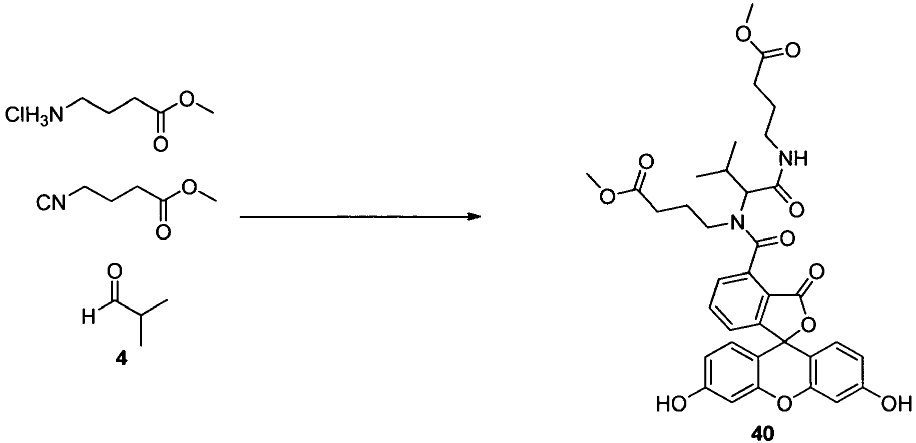

### Synthese von (40)

Methyl 4-(chloramino)butanoat **1** (41.5 mg, 0.27 mmol), Triethylamin (37.4 µL, 0.27 mmol) und Isobutyraldehyd **4** (24.6 µl, 0.27 mmol) wurde in 5 mL MeOH gelöst und für 2h bei R.T. gerührt. Anschließend wurden 5(6)-Carboxyfluorescein (100 mg, 0.27 mmol) und Methyl 4-(isocyano)butanoat **3** (34.0 µl, 0.27 mmol) zugegeben. Die Lösung wurde über Nacht bei R.T. gerührt. Die Lösung wurde im Rotationsverdampfer bis zur Trockne eingeengt und der Rückstand säulenchromatographisch aufgereinigt. Das Produkt wurde als oranges Öl (80 mg, 44%) erhalten. MS (ESI): [M+H]⁺ = 675.5

### Synthese von Dendrimeren mit Pyrenfarbstoffen:

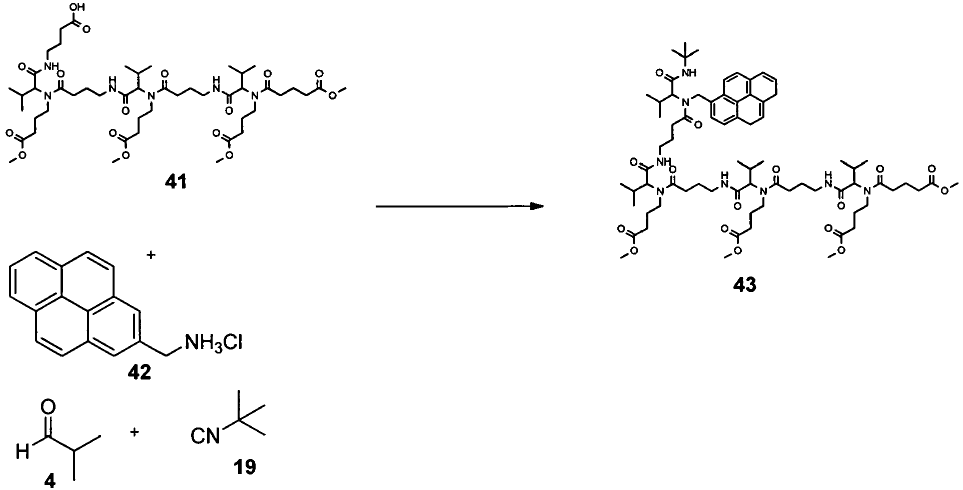

### Synthese von (43)

1-Pyrenmethylaminhydrochlorid **42** (26.8 mg, 0.10 mmol), Triethylamin (14.0 µl, 0.10 mmol) und Isobutyraldehyd **4** (10.0 µl, 0.10 mmol) wurden in 5 mL MeOH gelöst und für 2h bei R.T. gerührt. Anschließend wurden Dendrimer **41** (100 mg, 0.10 mmol) und *t*-Butylisocyanid **19** (11 µl, 0.10 mmol) zugegeben. Die Lösung wurde über Nacht bei R.T. gerührt. Die Lösung wurde im Rotationsverdampfer bis zur Trockne eingeengt und der Rückstand säulenchromatograpisch (EE/Hex., 10/1) aufgereinigt. Das Produkt wurde als farbloses Öl (70.0 mg, 57%) erhalten.
MS (ESI): [M+Na]⁺ = 1391.0

### Synthese von Rhodamin B - markierten Dendrimeren:

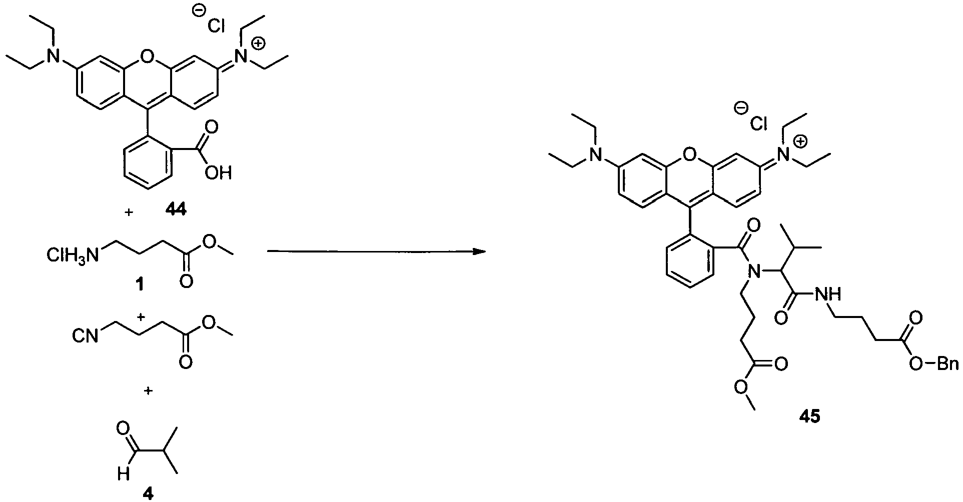

### Synthese von (45)

Methyl 4-(chloramino)butanoat **1** (644 mg, 4.20 mmol), Triethylamin (583 µL, 4.20 mmol) und Isobutyraldehyd **4** (382 µl, 4.20 mmol) wurden in 20 mL MeOH gelöst und für 2h bei R.T. gerührt. Anschließend wurden Rhodamin B (2.00 g, 4.20 mmol) und Benzyl 4-(isocyano)butanoat (854 mg, 4.20 mmol) zugegeben. Die Lösung wurde über Nacht bei R.T. gerührt. Die Lösung wurde im Rotationsverdampfer bis zur Trockne eingeengt und der Rückstand säulenchromatographisch (CH₂Cl₂/MeOH, 20/1) aufgereinigt. Das Produkt wurde als violettes Öl (2.69 g, 55%) erhalten.
MS (ESI): [M]⁺ = 817.5

### Synthese von Kerneinheiten über UGl-4CRs.

### Allgemeine Arbeitsvorschrift zu UGl-4CRs mit primären Alkylammoniumchloriden:

Die Aldehyd- oder Ketokomponente (6.50 mmol), das primäre Ammoniumchlorid (6.50 mmol) und Triethylamin (6.50 mmol) in MeOH (10 mL) werden für zwei Stunden bei Raumtemperatur gerührt, um das Imin-Intermediat zu bilden. Anschließend gibt man nacheinander die Carbonsäurekomponente (6.50 mmol) und die Isonitrilkomponente (6.50 mmol) hinzu. Die Reaktion verläuft bei Raumtemperatur durch eintägiges Rühren und der Verlauf wird per DC kontrolliert. Nach beendeter Reaktion wird die methanolische Lösung eingedampft und das erhaltene Rohprodukt säulenchromatographisch aufgereinigt.

### Methyl 5-[(5-Methoxy-1-{[(4-methoxy-4-oxobutyl)amino]carbonyl}-5-oxopentyl)-(4-methoxy-4-oxobutyl-)amino]-5-oxopentanoat (50)

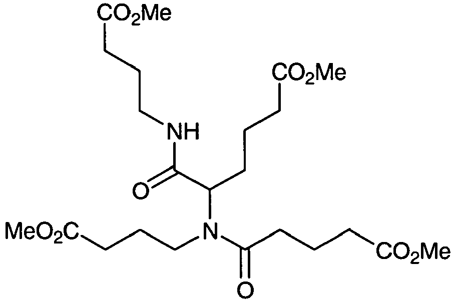

Die Ugi-4CR von Monomethyl Glutarat (0.95 g, 6.51 mmol) mit Methyl 5-Oxopentanoat (0.85 g, 6.51 mmol), Methyl 4-Aminobutyrat-Hydrochlorid (1.00 g, 6.51 mmol) und Methyl 4-lsocyanobutyrat (0.83 g, 6.51 mmol) liefert die methylestergeschützte Kerneinheit **50** nach säulenchromatographischer Aufreinigung (Ethylacetat/MeOH 4:1) als leicht gelbliches Öl (0.82 g, 25%). DC (Ethylacetat) *R*_{f} = 0.40; ¹H NMR (CDCl₃, 300 MHz) δ = 1.54 - 1.61 (m, 2 H, CH₂), 1.71 - 2.04 (m, 8 H, 4 CH₂), 2.24 - 2.44 (m, 8 H, 4 CH₂), 2.51 (t, *J =* 7.2 Hz, 2 H, CH₂), 3.20 - 3.35 (m, 4 H, 2 CH₂), 3.66, 3.67, 3.68, 3.69 (4s, 12 H, 4 CH₃), 4.80 (t, *J =* 7.6 Hz, 1 H, CH), 6.83 (t, *J* = 5.8 Hz, 1 H, NH) ppm; ¹³C NMR (CDCl₃, 75 MHz) δ = 19.97, 20.61, 20.89, 21.55, 24.61, 24.78, 27.49, 30.81, 31.32, 31.46, 31.54, 32.25, 32.81, 32.89, 33.01, 33.05, 33.15, 33.51, 35.42, 38.68, 38.93, 44.46, 51.55, 51.59, 51.62, 51.71, 57.19, 170.93, 172.71, 173.14, 173.21, 173.31, 173.46, 173.88, 175.67 ppm; ESI-MS von C₂₃H₃₈N₂O₁₀ (M+H⁺ = 502.8; M+Na⁺ = 525.6; M-H⁻ = 501.5); IR (ATR) ν = 2953.1, 2917.1, 2851.3, 1728.5 (CO₂Me), 1672.6, 1633.2 (Amid), 1531.1, 1435.4, 1365.9, 1253.1, 1195.1, 1167.6, 1091.9, 1059.4, 992.2, 866.8, 731.7 cm⁻¹; HRMS von C₂₃H₃₈N₂O₁₀ [M+Na]⁺ ber. 525.24242 gef. 525.24111.

### Allgemeine Arbeitsvorschrift für Verseifungen von Methylestern:

Das Methylesterderivat (1.50 mmol) in einem Gemisch aus THF (20 mL) und Wasser (10 mL) wird mit Hilfe eines Eisbades auf 0°C gekühlt. Anschließend versetzt man mit LiOH Monohydrat (2.5 Äquivalente je Methylestergruppe) und lässt das Reaktionsgemisch auf Raumtemperatur erwärmen. Man lässt für ca. einen Tag reagieren und kontrolliert den Reaktionsverlauf per DC. Nach beendeter Reaktion säuert man mit 2 M NaHSO₄ an (pH 2) und extrahiert mit Ethylacetat (5 x 30 mL). Die vereinten organischen Lösungen werden über Na₂SO₄ getrocknet, filtriert und im Vakuum zur Trockene eingeengt, um das im Allgemeinen reine Carbonsäurederivat zu erhalten.

### 5-[(4-Carboxy-1-{[(3-carboxypropyl)amino]carbonyl}butyl)(3-carboxypropyl)-amino]-5-oxopentansäure (51) (Erste Generation - selbstgenerierte Kerneinheit)

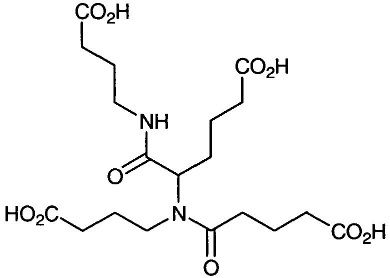

Die Verseifung der Methylestergruppen von der Kerneinheit **50** (0.76 g, 1.51 mmol) liefert das Tetracarbonsäurederivat **51** als leicht gelbliches Öl (0.61 g, 90%). DC (Ethylacetat/MeOH/H₂O 5:2:1) *R*f = 0.36; ¹H NMR (CD₃OD, 300 MHz) δ = 1.54 - 1.62 (m, 2 H, CH₂), 1.73-1.97 (m, 8 H, 4 CH₂), 2.29 - 2.41 (m, 8 H, 4 CH₂), 2.55 (t, *J* = 7.4 Hz, 2 H, CH₂), 3.19 - 3.42 (m, 4 H, 2 CH₂), 4.75 (t, *J* = 6.6 Hz, 1 H, CH) ppm; ¹³C NMR (CD₃OD, 75 MHz) δ = 21.38, 21.73, 21.97, 22.95, 25.59, 26.24, 29.44, 30.57, 31.77, 32.24, 32.62, 33.39, 33.66, 33.91, 34.04, 34.40, 39.92, 40.10, 46.48, 59.19, 61.86, 171.90, 172.82, 175,20, 175.74, 176.13, 176.59, 176.66, 176.70, 176.77, 176.81, 176.94 ppm; ESI-MS C₁₉H₃₀N₂O₁₀ (M+H⁺ = 447.4; M+Na⁺ = 469.6; M-H⁻ = 445.7); IR (ATR) ν = 3324.8, 2944.2, 2833.3, 1708.5 (CO₂H), 1626.2 (Amid), 1543.2, 1412.7, 1201.8, 1018.9 cm⁻¹; HRMS C₁₉H₃₀N₂O₁₀ [M+Na]⁺ ber. 469.17981 gef. 469.18014.

### Methyl N-(6-Methoxy-6-oxohexyl)-N-(5-methoxy-5-oxopentanoyl)-phenylalanyl-β-alaninat (52)

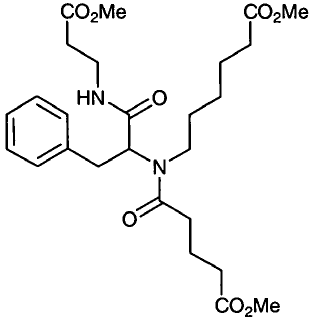

Die Ugi-4CR von Monomethyl Glutarat (2.00 g, 13.7 mmol) mit Phenylacetaldehyd (1.64 g, 13.7 mmol), Methyl 6-Aminohexanoat-Hydrochlorid (2.49 g, 13.7 mmol) und Methyl 3-lsocyanopropionat (1.55 g, 13.7 mmol) liefert die methylestergeschützte Kerneinheit **52** nach säulenchromatographischer Aufreinigung (Ethylacetat/MeOH 19:1) als gelbliches Öl (1.87 g, 27%). DC (Ethylacetat) *R*_{f} = 0.49; ¹H NMR (CDCl₃, 300 MHz) δ = 1.20 - 1.63 (m, 4 H, 2 CH₂), 1.86 - 2.00 (m, 4 H, 2 CH₂), 2.26 - 2.57 (m, 8 H, 4 CH₂), 3.02 - 3.30 (m, 4 H, 2 CH₂), 3.45 (q, *J =* 6.2 Hz, 2 H, CH₂), 3.66, 3.66, 3.67 (3s, 9 H, 3 CH₃), 4.79 (br, t, *J =* 7.4 Hz, 1 H, CH), 7.10 - 7.29 (m, 5 H, 5 CH) ppm; ¹³C NMR (CDCl₃, 75 MHz) δ = 19.86, 20.33, 24.35, 26.30, 26.92, 29.09, 32.38, 32.87, 32.91, 33.55, 33.67, 33.87, 34.20, 34.98, 46.86, 51.47, 51.50, 51.53, 51.65, 60.57, 126.37, 128.23, 128.79, 137.17, 170.67, 172.11, 173.13, 173.37, 173.47, 173.58, 176.85 ppm; ESI-MS von C₂₆H₃₈N₂O₈ (M+H⁺ = 507.2; M+Na⁺ = 529.3; 2M+Na⁺ = 1036.1; M-H- = 505.4); IR (ATR) ν = 3352.7, 2951.7, 1730.9 (CO₂Me), 1644.3 (Amid), 1530.9, 1436.8, 1367.3, 1196.7, 1172.0, 1062.1, 1024.7, 842.8, 752.0, 701.5 cm⁻¹; HRMS von C₂₆H₃₈N₂O₈ [M+Na]⁺ ber. 529.25259 gef. 529.25267.

### N-(4-Carboxybutanoyl)-N-(5-carboxypentyl)phenylalanyl-β-alanin (53)

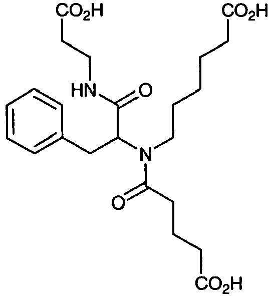

Die Verseifung der Methylestergruppen von der Kerneinheit **52** (1.10 g, 2.17 mmol) liefert das Tricarbonsäurederivat **53** als gelbliches Öl (0.97 g, 96%). DC (Ethylacetat/MeOH/H₂O 5:2:1) *R*_{f} = 0.59; ¹H NMR (CD₃OD, 300 MHz) δ = 1.20 - 1.64 (m, 4 H, 2 CH₂), 1.80 - 1.92 (m, 4 H, 2 CH₂), 2.22 - 2.50 (m, 8 H, 4 CH₂), 3.00 - 3.32 (m, 4 H, 2 CH₂), 3.35 - 3.43 (m, 2 H, CH₂), 4.63 - 4.69 (m, 1 H, CH), 7.16 - 7.29 (m, 5 H, 5 CH) ppm; ¹³C NMR (CD₃OD, 75 MHz) δ = 21.37, 21.69, 25.60, 27.32, 30.05, 33.51, 33.91, 34.30, 34.65, 35.48, 36.41, 62.68, 127.53, 129.37, 130.19, 139.05, 172.38, 174.93, 175.17, 175.26, 176.60, 176.73, 177.21 ppm; ESI-MS von C₂₃H₃₂N₂O₈ (M+H⁺ = 465.2; M+Na⁺ = 487.3; 2M+Na⁺ = 951.8; M-H- = 463.6); IR (ATR) ν = 2940.7, 1703.4 (CO₂H) 1538.2, 1496.1, 1409.5, 1190.8, 1056.8, 864.9, 752.4, 701.7 cm⁻¹; HRMS C₂₃H₃₂N₂O₈ [M+Na]⁺ ber. 487.20563 gef. 487.20562.

### Methyl 6-Methoxy-N-(6-methoxy-6-oxohexyl)-N-(5-methoxy-5-oxopentanoyl)-6-oxonorleucyl-β-alaninat (54)

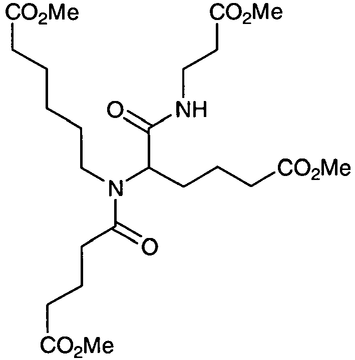

Die Ugi-4CR von Monomethyl Glutarat (2.00 g, 13.7 mmol) mit Methyl 5-Oxopentanoat (1.78 g, 13.7 mmol), Methyl 6-Aminohexanoat-Hydrochlorid (2.49 g, 13.7 mmol) und Methyl 3-Isocyanopropionat (1.55 g, 13.7 mmol) liefert die methylestergeschützte Kerneinheit **54** nach säulenchromatographischer Aufreinigung (Ethylacetat/MeOH 9:1) als braunes Öl (1.95 g, 28%). DC (Ethylacetat/MeOH 19:1) *R*_{f} = 0.71; ¹H NMR (CDCl₃, 300 MHz) δ = 1.23 - 1.34 (m, 2 H, CH₂), 1.48 - 1.77 (m, 8 H, 4 CH₂), 1.93 - 2.03 (m, 2 H, CH₂), 2.28 - 2.45 (m, 8 H, 4 CH₂), 2.51 (t, *J =* 6.3 Hz, 2 H, CH₂), 3.22 (t, *J* = 8.2 Hz, 2 H, CH₂), 3.42 - 3.50 (m, 2 H, CH₂), 3.66, 3.67, 3.68, 3.68 (4s, 12 H, 4 CH₃), 4.76 (t, *J =* 7.6 Hz, 1 H, CH), 6.99 (t, *J =* 5.8 Hz, 1 H, NH) ppm; ¹³C NMR (CDCl₃, 75 MHz) δ = 20.27, 20.40, 21.47, 24.30, 26.43, 27.33, 29.44, 32.21, 32.94, 33.09, 33.43, 33.65, 33.68, 34.90, 36.96, 45.20, 51.42, 51.46, 51.49, 51.63, 57.13, 170.86, 171.96, 173.23, 173.37, 173.41, 173.47, 176.37 ppm; ESI-MS von C₂₄H₄₀N₂O₁₀ (M+H⁺ = 517.2; M+Na⁺ = 539.4; 2M+Na⁺ = 1055.6; M-H- = 515.6); IR (ATR) ν = 3372.9, 2951.5, 2835.4, 1731.4 (CO₂Me), 1625.5 (Amid), 1533.0, 1437.4, 1367.8, 1198.1, 1173.5, 1024.4, 842.0 cm⁻¹; HRMS von C₂₄H₄₀N₂O₁₀ [M+Na]⁺ ber. 539.25752 gef. 539.25756.

### N-(4-Carboxybutanoyl)-N-(5-carboxypentyl)-6-oxidanyl-6-oxidanylidenenorleucyl-β-alanin (55)

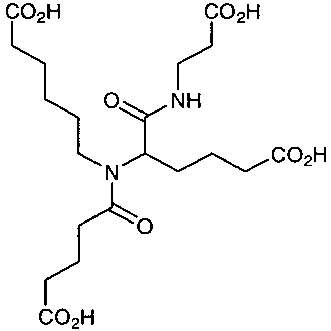

Die Verseifung der Methylestergruppen von der Kerneinheit **54** (1.87 g, 3.62 mmol) liefert das Tetracarbonsäurederivat **55** als rotbraunes Öl (1.55 g, 93%). DC (Ethylacetat/MeOH/H₂O 5:2:1) *R*_{f} = 0.40; ¹H NMR (CDCl₃, 300 MHz) δ = 1.21 - 1.41 (m, 2 H, CH₂), 1.53 - 1.75 (m, 8 H, 4 CH₂), 1.84 - 1.99 (m, 2 H, CH₂), 2.26 - 2.40 (m, 8 H, 4 CH₂), 2.43 - 2.55 (m, 2 H, CH₂), 3.10 - 3.38 (m, 2 H, CH₂), 3.40 - 3.47 (m, 2 H, CH₂), 4.75 (t, *J =* 6.7 Hz, 1 H, CH) ppm; ¹³C NMR (CD₃OD, 75 MHz) δ = 21.39, 21.78, 21.88, 22.86, 25.61, 27.56, 29.26, 30.80, 33.38, 33.93, 34.00, 34.39, 34.44, 34.76, 36.45, 47.08, 59.02, 172.81, 175.04, 175.14, 175.56, 176.64, 176.85, 177.28 ppm; ESI-MS C₂₀H₃₂N₂O₁₀ (M+H⁺ = 461.3; M+Na⁺ = 483.0; M-H⁻ = 459.5); IR (ATR) ν = 3338.5, 2944.2, 2834.3, 1708.7 (CO₂H), 1662.7, 1621.8 (Amid), 1537.6, 1410.4, 1198.4, 1019.5 cm⁻¹; HRMS von C₂₀H₃₂N₂O₁₀ [M+Na]⁺ ber. 483.19547 gef. 483.19500.
Divergenter Aufbau von Dendrimeren durch UGl-4CRs
Darstellung von Dendrimeren der ersten Generation

### Methylestergeschützte zweite Generation (1→2-Verzweigung) (60)

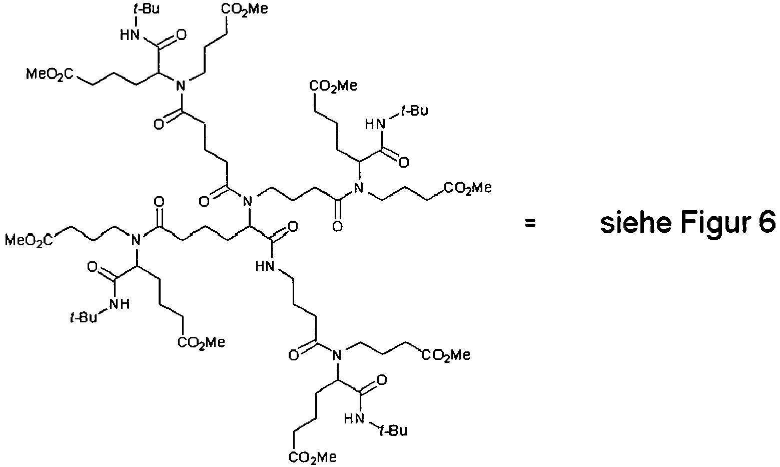

Die vierfache Ugi-4CR der Tetracarbonsäurekerneinheit **51** (0.84 g, 1.88 mmol) mit Überschüssen an Methyl 5-Oxopentanoat (2.94 g, 22.6 mmol), Methyl 4-Aminobutyrat-Hydrochlorid (3.47 g, 22.6 mmol) und *t*-Butylisonitril (1.88 g, 22.6 mmol) liefert die methylestergeschützte zweite Generation **60** nach säulenchromatographischer Aufreinigung (Ethylacetat/MeOH 19:1) als leicht gelbliches Öl (2.12 g, 66%). DC (Ethylacetat/MeOH 19:1) *R*_{f} = 0.64; ¹H NMR (CDCl₃, 300 MHz) δ = 1.24 - 1.37 (m, 36 H, 12 CH₃), 1.52 - 1.90 (m, 38 H, 19 CH₂), 2.26 - 2.63 (m, 24 H, 12 CH₂), 3.25 -3.41 (m, 10 H, 5 CH₂), 3.62 - 3.68 (m, 24 H, 8 CH₃), 4.70 - 4.83 (m, 5 H, 5 CH), 6.46 - 6.50 (m, 5 H, 5 NH) ppm; ¹³C NMR (CDCl₃, 75 MHz) δ = 21.00, 21.47, 24.74, 27.38, 28.50, 28.66, 30.83, 30.95, 33.50, 43.65, 50.89, 51.45, 51.64, 53.38, 56.98, 60.24, 169.90, 172.70, 173.25, 173.70 ppm; ESI-MS von C₈₃H₁₄₂N₁₀O₂₆ (M+H⁺ = 1697.4; M+Na⁺ = 1718.2; M+2Na²⁺ = 870.9; M-H⁻ = 1694.8; M+Cl⁻ = 1730.6); IR (ATR) ν = 3318.6, 2953.9, 2246.8, 1731.7 (CO₂Me), 1673.3 (Amid), 1624.1 (Amid), 1532.8, 1435.2, 1363.8, 1258.6, 1196.9, 1168.3, 1076.3, 918.0, 728.1 cm⁻¹; HRMS von C₈₃H₁₄₂N₁₀O₂₆ exakte Masse = 1695.00967 m/z (z = 2) [M+2Na]²⁺ ber. 870.49461, gef. 870.49746.

### Zweite Generation als Octacarbonsäure (61)

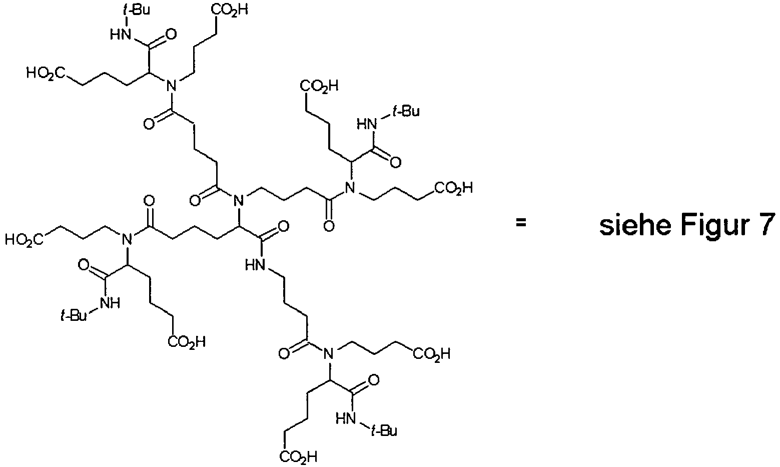

Die Verseifung der Methylestergruppen von der zweiten Generation **60** (2.00 g, 1.18 mmol) liefert das Octacarbonsäurederivat **61** als farblosen Feststoff (1.74 g, 93%). DC (Ethytacetat/MeOH/H₂O 2:2:1) *R*_{f} = 0.80; Smp. 75 - 76°C (Ethylacetat); ¹H NMR (CD₃OD, 300 MHz) δ = 1.21 - 1.39 (m, 36 H, 12 CH₃), 1.53 - 1.94 (m, 38 H, 19 CH₂), 2.24 - 2.54 (m, 24 H, 12 CH₂), 3.24 -3.39 (m, 10 H, 5 CH₂), 4.75 - 4.78 (m, 5 H, 5 CH) ppm; ¹³C NMR (CD₃OD, 75 MHz) δ = 22.76, 22.90, 25.28, 26.30, 28.85, 29.43, 31.77, 32.69, 33.87, 34.39, 45.51, 52.20, 52.52, 58.97, 61.78, 171.95, 176.05, 176.54, 176.67 ppm; ESI-MS von C₇₅H₁₂₆N₁₀O₂₆ (M+H⁺ = 1585.4; M+Na⁺ = 1606.0; M-H⁻ = 1582.1, M+2Na²⁺ = 814.7, M-H²⁻ = 791.0); IR (ATR) ν = 3335.3, 2964.2, 1713.2 (CO₂H), 1620.4 (Amid), 1538.9, 1455.3, 1417.4, 1365.9, 1218.4, 1027.2, 864.6, 754.3 cm⁻¹; HRMS von C₇₅H₁₂₆N₁₀O₂₆ exakte Masse = 1582.88447 m/z (z = 2) [M-2H]²⁻ ber. 790.43441, gef. 790.43280.

### Methylestergeschützte zweite Generation (1→2-Verzweigung) (62)

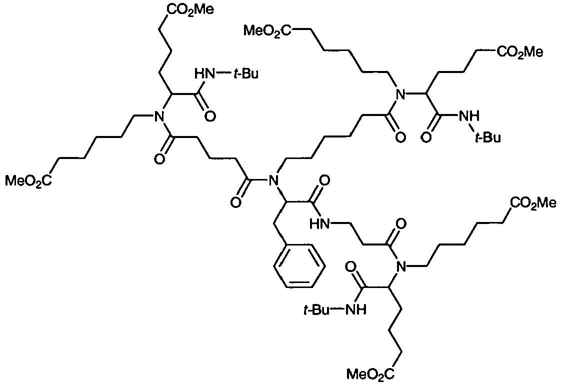

Die dreifache Ugi-4CR der Tricarbonsäurekerneinheit **53** (0.18 g, 0.38 mmol) mit Überschüssen an Methyl 5-Oxopentanoat (0.45 g, 3.42 mmol), Methyl 6-Aminohexanoat-Hydrochlorid (0.62 g, 3.42 mmol) und *t*-Butylisonitril (0.28 g, 3.42 mmol) liefert die methylestergeschützte zweite Generation **62** nach säulenchromatographischer Aufreinigung (Ethylacetat/MeOH 19:1) als gelbliches Öl (0.36 g, 64%). DC (Ethylacetat/MeOH 19:1) *R*_{f} = 0.64; ¹H NMR (CDCl₃, 300 MHz) δ = 1.24-1.94 (m, 65 H, 9 CH₃, 19 CH₂), 2.25-2.41 (m, 20 H, 10 CH₂), 3.19-3.30 (m, 12 H, 6 CH₂), 3.65, 3.66, 3.67 (3s, 18 H, 6 CH₃), 4.65 - 4.80 (m, 4 H, 4 CH), 6.40 - 6.62 (m, 4 H, 4 NH), 7.16 - 7.24 (m, 5 H, 5 CH) ppm; ¹³C NMR (CDCl₃, 75 MHz) δ = 21.49, 24.39, 24.58, 25.03, 26.54, 26.84, 27.35, 27.54, 28.55, 29.69, 32.68, 33.26, 33.51, 33.77, 44.57, 50.85, 50.91, 51.46, 53.39, 57.22, 128.17, 128.82, 169.68, 169.95, 173.30, 173.50, 173.78 ppm; ESI-MS von C₇₇H₁₂₈N₈O₂₀ (M+H⁺ = 1486.3; M+Na⁺ = 1509.2; M+2Na²⁺ = 766.2; M-H- = 1484.3); IR (ATR) ν = 3316.5, 2950.8, 2867.1, 1732.2 (CO₂Me), 1677.0 (Amid), 1622.5 (Amid), 1536.6, 1453.6, 1435.3, 1364.0, 1198.0, 1171.0, 1077.0, 1009.7, 883.2, 752.9, 702.3 cm⁻¹; HRMS von C₇₇H₁₂₈N₈O₂₀ exakte Masse = 1484.92449 m/z (z = 2) (M+2Na]²⁺ ber. 765.45202, gef. 765.45117.

### Zweite Generation als Hexacarbonsäure (63)

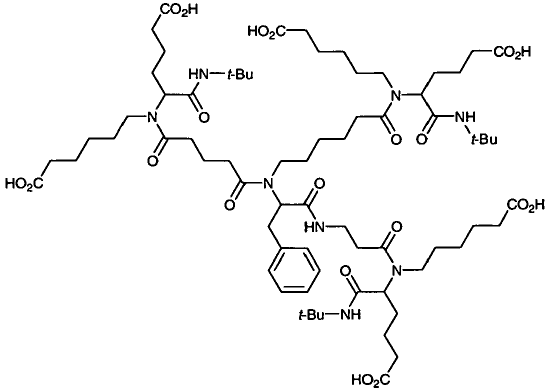

Die Verseifung der Methylestergruppen von der zweiten Generation **62** (0.18 g, 0.12 mmol) liefert das Hexacarbonsäurederivat **63** als farbloses Öl (0.15 g, 93%). DC (Ethylacetat/MeOH/H₂O 3:2:1) *R*_{f} = 0.81; ¹H NMR (CD₃OD, 300 MHz) δ = 1.25 - 1.88 (m, 65 H, 9 CH₃, 19 CH₂), 2.26 - 2.64 (m, 20 H, 10 CH₂), 3.13 - 3.54 (m, 12 H, 6 CH₂), 4.73 - 4.76 (m, 4 H, 4 CH), 7.16 - 7.24 (m, 5 H, 5 CH) ppm; ¹³C NMR (CD₃OD, 75 MHz) δ = 22.73, 24.21, 25.65, 25.77, 27.57, 27.84, 28.82, 29.38, 31.00, 34.39, 34.80, 46.25, 52.15, 59.06, 129.54, 130.38, 172.21, 176.75, 176.90, 177.25 ppm; ESI-MS von C₇₁H₁₁₆N₈O₂₀ (M+H⁺ = 1402.2; M+Na⁺ = 1424.4; M-H⁻ = 1400.3); IR (ATR) ν = 3331.5, 2941.6, 2831.5, 1713.0 (CO₂H), 1661.5 (Amid), 1621.0 (Amid), 1541.6, 1455.1, 1425.6, 1366.1, 1221.4, 1090.1, 1022.1 cm⁻¹; HRMS von C₇₁H₁₁₆N₈O₂₀ [M+Na]⁺ ber. 1423.82036, gef. 1423.81896.

### Methylestergeschützte zweite Generation (1→2-Verzweigung) (64)

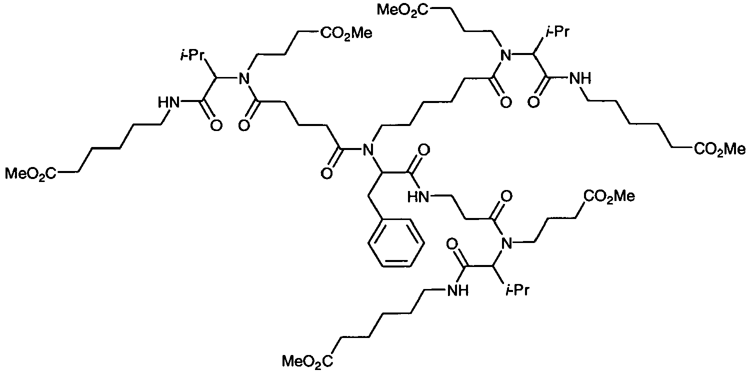

Die dreifache Ugi-4CR der Tricarbonsäurekerneinheit **53** (0.18 g, 0.38 mmol) mit Überschüssen an Isobutyraldehyd (0.25 g, 3.42 mmol), Methyl 4-Aminobutyrat-Hydrochlorid (0.53 g, 3.42 mmol) und Methyl 6-Isocyanohexanoat (0.53 g, 3.42 mmol) liefert die methylestergeschützte zweite Generation **64** nach säulenchromatographischer Aufreinigung (Ethylacetat/MeOH 19:1) als farbloses Öl (0.35 g, 63%). DC (Ethylacetat/MeOH 19:1) *R*_{f} = 0.49; ¹H NMR (CDCl₃, 300 MHz) δ = 0.74 - 0.99 (m, 18 H, 6 CH₃), 1.21 - 1.95 (m, 32 H, 16 CH₂), 2.27 - 2.65 (m, 23 H, 10 CH₂, 3 CH), 3.15 - 3.59 (m, 18 H, 9 CH₂), 3.65, 3.67, 3.68 (3s, 18 H, 6 CH₃), 4.06 - 4.40 (m, 4 H, 4 CH), 6.67 - 7.05 (m, 4 H, 4 NH), 7.17-7.26 (m, 5 H, 5 CH) ppm; ¹³C NMR (CDCl₃, 75 MHz) δ = 18.84, 19,77, 24.44, 26.29, 26.33, 29.03, 30.77, 30.92, 33.80, 38.92, 39.00, 51.39, 51.63, 128.15, 128.77, 172.64, 172.84, 173.63 ppm; ESI-MS von C₇₄H₁₂₂N₈O₂₀ (M+H⁺ = 1444.1; M+Na⁺ = 1466.3; M+2Na²⁺ = 744.6; M-H⁻ = 1442.3; M+Cl⁻ = 1478.6); IR (ATR) ν = 3307.2, 2949.9, 2870.9, 1732.1 (CO₂Me), 1621.1 (Amid), 1537.8, 1435.3, 1366.8, 1196.3, 1162.4, 1102.1, 1029.3, 924.9, 865.9, 731.9, 701.0 cm⁻¹; HRMS von C₇₄H₁₂₂N₈O₂₀ exakte Masse = 1442.87754 m/z (z = 2) [M+2Na]²⁺ ber. 744.42854, gef. 744.42727.

### Zweite Generation als Hexacarbonsäure (65)

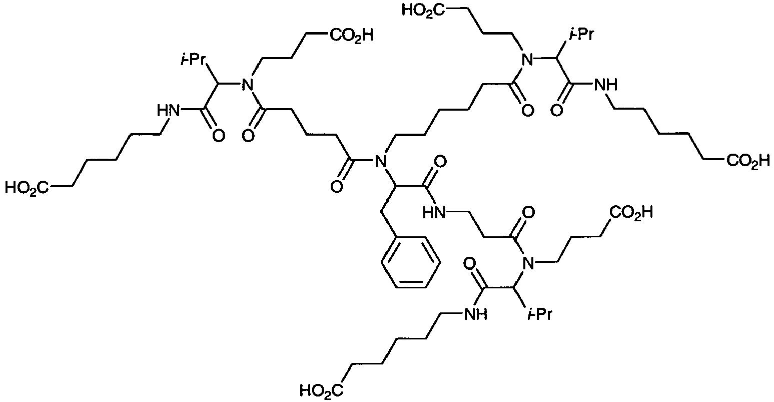

Die Verseifung der Methylestergruppen von der zweiten Generation **64** (0.20 g, 0.14 mmol) liefert das Hexacarbonsäurederivat **65** als farbloses Öl (0.17 g, 91%). DC (Ethylacetat/MeOH/H₂O 3:2:1) *R*_{f} = 0.76; ¹H NMR (CD₃OD, 300 MHz) δ = 0.80 - 0.97 (m, 18 H, 6 CH₃), 1.31 - 1.81 (m, 32 H, 16 CH₂), 2.22 - 2.67 (m, 23 H, 10 CH₂, 3 CH), 3.15 - 3.68 (m, 18 H, 9 CH₂), 4.49 - 4.51 (m, 4 H, 4 CH), 7.19-7.26 (m, 5 H, 5 CH) ppm; ¹³C NMR (CD₃OD, 75 MHz) δ = 19.19, 19.29, 20.01, 20.74, 24.21, 24.73, 25.66, 26.16, 26.49, 27.51, 28.17, 29.14, 29.86, 30.69, 31.93, 32.80, 33.54, 34.79, 37.01, 40.12, 43.69, 45.56, 64.81, 67.76, 127.69, 129.57, 130.37, 130.59, 171.64, 172.44, 172.56, 175.50, 176.18, 176.29, 176.75, 177.34 ppm; ESI-MS von C₆₈H₁₁₀N₈O₂₀ (M+H⁺ = 1359.9; M+Na⁺ = 1382.5; M-H- = 1358.6); IR (ATR) ν = 3306.9, 2940.2, 2831.6, 1712.4 (CO₂H), 1620.5 (Amid), 1549.0, 1418.0, 1372.9, 1197.7, 1165.3, 1088.8, 1022.4, 701.6 cm⁻¹; HRMS von C₆₈H₁₀N₈O₂₀ [M+Na]⁺ ber. 1381.77341, gef. 1381.77333.

### Methylestergeschützte zweite Generation (1→2-Verzweigung) (66)

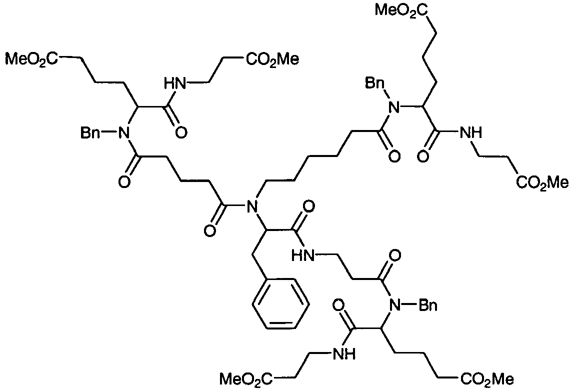

Die dreifache Ugi-4CR der Tricarbonsäurekerneinheit **53** (0.18 g, 0.39 mmol) mit Überschüssen an Methyl 5-Oxopentanoat (0.46 g, 3.51 mmol), Benzylamin (0.38 g, 3.51 mmol) und Methyl 3-Isocyanopropionat (0.40 g, 3.51 mmol) liefert die methylestergeschützte zweite Generation **66** nach säulenchromatographischer Aufreinigung (Ethylacetat/MeOH 19:1) als leicht gelbliches Öl (0.39 g, 68%). DC (Ethylacetat/MeOH 19:1) *R*_{f} = 0.47; ¹H NMR (CDCl₃, 300 MHz) δ = 1.13-2.57 (m, 40 H, 20 CH₂), 2.91 - 3.51 (m, 12 H, 6 CH₂), 3.60 - 3.69 (m, 18 H, 6 CH₃), 4.42 - 4.89 (m, 10 H, 3 CH₂, 4 CH), 6.83 - 6.98 (m, 4 H, 4 NH), 7.12 - 7.31 (m, 20 H, 20 CH) ppm; ¹³C NMR (CDCl₃, 75 MHz) δ = 21.66, 24.60, 27.86, 33.48, 33.72, 34.19, 34.90, 35.13, 48.25, 51.45, 51.66, 53.42, 57.18, 125.78, 125.86, 126.27, 127.19, 128.21, 128.53, 136.95, 137.23, 170.10, 172.08, 173.24, 174.53 ppm; ESI-MS von C₇₇H₁₀₄N₈O₂₀ (M+Na⁺ = 1484.0; M+2Na²⁺ = 754.3); IR (ATR) ν = 3314.6, 2949.9, 1731.8 (CO₂Me), 1625.5 (Amid), 1531.8, 1496.5, 1436.8, 1364.4, 1196.8, 1172.9, 1076.6, 1027.0, 918.3, 886.3, 729.6, 698.7 cm⁻¹; HRMS von C₇₇H₁₀₄N₈O₂₀ exakte Masse = 1460.73669 m/z (z = 2) [M+2Na]²⁺ ber. 753.35812, gef. 753.35629.

### Zweite Generation als Hexacarbonsäure (67)

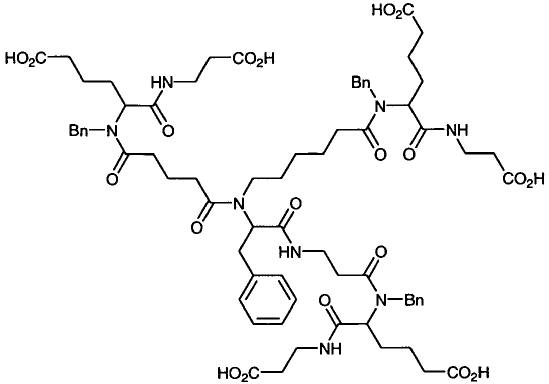

Die Verseifung der Methylestergruppen von der zweiten Generation **66** (0.25 g, 0.17 mmol) liefert das Hexacarbonsäurederivat **67** als farblosen Feststoff (0.21 g, 92%). DC (Ethylacetat/MeOH/H₂O 3:2:1) *R*_{f} = 0.72; Smp. 84 - 85°C (Ethylacetat); ¹H NMR (CD₃OD, 300 MHz) δ = 0.96 - 2.51 (m, 40 H, 20 CH₂), 2.96 - 3.48 (m, 12 H, 6 CH₂), 4.35 - 4.78 (m, 10 H, 3 CH₂, 4 CH), 7.12 - 7.33 (m, 20 H, 20 CH) ppm; ¹³C NMR (CD₃OD, 75 MHz) δ = 22.74, 22.81, 25.88, 27.39, 29.64, 29.84, 30.72, 33.97, 34.28, 34.34, 34.55, 36.32, 36.55, 36.79, 58.57, 61.92, 127.41, 127.49, 127.64, 127.91, 128.42, 128.54, 129.29, 129.51, 129.82, 130.37, 130.61, 138.76, 138.84, 139.12, 139.18, 172.45, 175.17, 175.51, 176.41, 176.71, 176.88 ppm; ESI-MS von C₇₁H₉₂N₈O₂₀ (M+H⁺ = 1378.1; M+Na⁺ = 1399.9; M-H⁻ = 1376.3); IR (ATR) ν = 2940.3, 1715.6 (CO₂H), 1622.1 (Amid), 1538.5, 1496.6, 1451.4, 1417.4, 1364.0, 1196.0, 1077.2, 1029.1, 862.2, 732.6, 700.5 cm⁻¹; HRMS von C₇₁H₉₂N₈O₂₀ [M+Na]⁺ ber. 1399.63256, gef. 1399.63316.

### Methylestergeschützte zweite Generation (1→2-Verzweigung) (68)

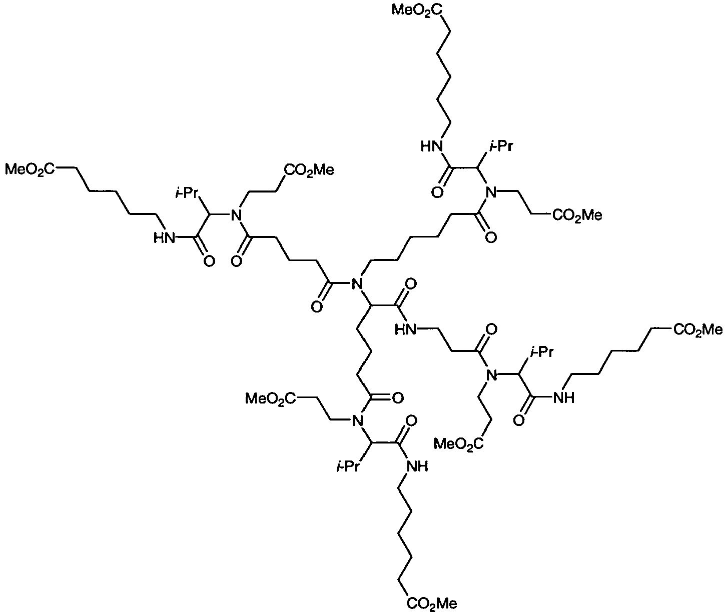

Die vierfache Ugi-4CR der Tetracarbonsäurekerneinheit **55** (0.18 g, 0.38 mmol) mit Überschüssen an Isobutyraldehyd (0.33 g, 4.56 mmol), Methyl 3-Aminopropionat-Hydrochlorid (0.64 g, 4.56 mmol) und Methyl 6-Isocyanohexanoat (0.71 g, 4.56 mmol) liefert die methylestergeschützte zweite Generation **68** nach säulenchromatographischer Aufreinigung (Ethylacetat/MeOH 19:1) als leicht gelbliches Öl (0.37 g, 57%). DC (Ethylacetat/MeOH 19:1) *R*_{f} = 0.46; ¹H NMR (CDCl₃, 300 MHz) δ = 0.79 - 0.96 (m, 24 H, 8 CH₃), 1.24 - 1.68 (m, 36 H, 18 CH₂), 1.97 - 2.74 (m, 30 H, 13 CH₂, 4 CH), 3.12 -3.59 (m, 20 H, 10 CH₂), 3.66, 3.68 (2s, 24 H, 8 CH₃), 4.08 - 4.41 (m, 5 H, 5 CH), 6.73 - 6.98 (m, 5 H, 5 NH) ppm; ¹³C NMR (CDCl₃, 75 MHz) δ = 18.80, 19.75, 24.45, 26.31, 29.05, 32.47, 33.57, 33.80, 39.00, 51.42, 51.74, 53.41, 170.46, 171.15, 173.63 ppm; ESI-MS von C₈₄H₁₄₄N₁₀O₂₆ (M+Na⁺ = 1732.6; M+2Na²⁺ = 877.6; M-H- = 1708.7; M+Cl⁻ = 1744.7); IR (ATR) ν = 3317.4, 2951.8, 2871.0, 1731.9 (CO₂Me), 1624.8 (Amid), 1537.4, 1434.7, 1370.2, 1196.2, 1163.3, 987.2, 850.7 cm⁻¹; HRMS von C₈₄H₁₄₄N₁₀O₂₆ exakte Masse = 1709.02532 m/z (z = 2) [M+2Na]²⁺ ber. 877.50243, gef. 877.50363.

### Zweite Generation als Octacarbonsäure (69)

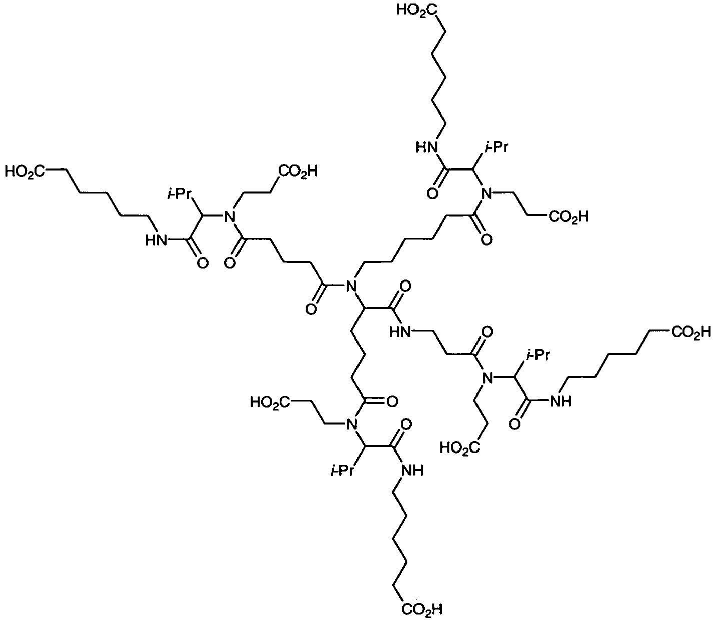

Die Verseifung der Methylestergruppen von der zweiten Generation **68** (0.25 g, 0.15 mmol) liefert das Octacarbonsäurederivat 69 als farblosen Feststoff (0.23 g, 96%). DC (Ethylacetat/MeOH/H₂O 3:2:1) *R*_{f} = 0.48; Smp. 70 - 71 °C (Ethylacetat); ¹H NMR (CD₃OD, 300 MHz) δ = 0.81 - 0.97 (m, 24 H, 8 CH₃), 1.21 - 1.99 (m, 36 H, 18 CH₂), 2.27 - 2.61 (m, 30 H, 13 CH₂, 4 CH), 3.10 - 3.94 (m, 20 H, 10 CH₂), 4.46 - 4.49 (m, 5 H, 5 CH) ppm; ¹³C NMR (CD₃OD, 75 MHz) δ = 19.36, 19.78, 20.10, 25.70, 27.52, 27.56, 28.22, 29.23, 29.88, 30.92, 33.30, 33.79, 34.19, 34.78, 40.16, 41.91, 64.88, 67.51, 171.42, 172.30, 174.13, 174.26, 175.26, 177.12 ppm; ESI-MS von C₇₆H₁₂₈N₁₀O₂₆ (M+H⁺ = 1597.3; M+Na⁺ = 1620.0; M+2Na²⁺ = 820.6; M-H- = 1596.5); IR (ATR) ν = 3344.5, 2942.1, 2833.4, 1712.1 (CO₂H), 1622.4 (Amid), 1556.0, 1422.2, 1202.4, 1117.7, 1021.0 cm⁻¹; HRMS von C₇₆H₁₂₈N₁₀O₂₆ [M+Na]⁺ ber. 1619.88990, gef. 1619.89041.

### Methylestergeschützte zweite Generation (1 →2-Verzweigung) (70)

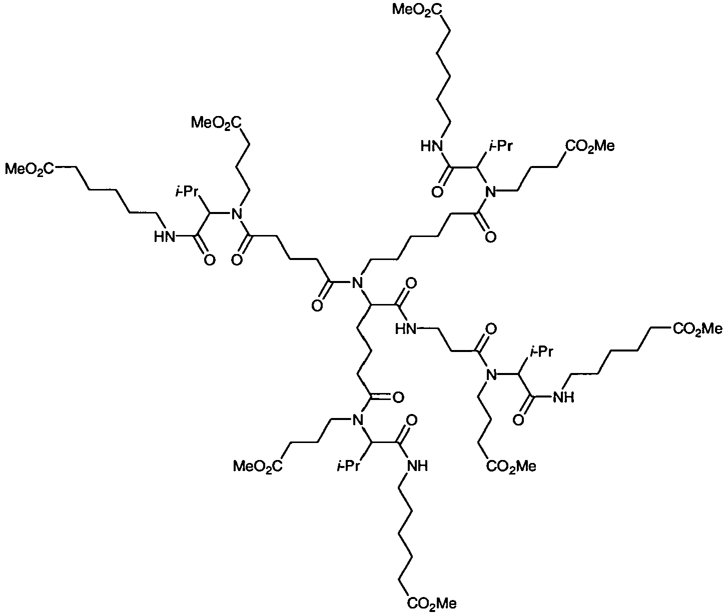

Die vierfache Ugi-4CR der Tetracarbonsäurekerneinheit **55** (0.18 g, 0.39 mmol) mit Überschüssen an Isobutyraldehyd (0.34 g, 4.68 mmol), Methyl 4-Aminobutyrat-Hydrochlorid (0.72 g, 4.68 mmol) und Methyl 6-Isocyanohexanoat (0.73 g, 4.68 mmol) liefert die methylestergeschützte zweite Generation **70** nach säulenchromatographischer Aufreinigung (Ethylacetat/MeOH 19:1) als leicht gelbliches Öl (0.47 g, 68%). DC (Ethylacetat/MeOH 19:1) *R*_{f} = 0.48; ¹H NMR (CDCl₃, 300 MHz) δ = 0.78 - 0.96 (m, 24 H, 8 CH₃), 1.24 - 2.05 (m, 44 H, 22 CH₂), 2.25 - 2.63 (m, 30 H, 13 CH₂, 4 CH), 3.12 -3.41 (m, 20 H, 10 CH₂), 3.66, 3.68 (2s, 24 H, 8 CH₃), 4.12 - 4.40 (m, 5 H, 5 CH), 6.75 - 6.98 (m, 5 H, 5 NH) ppm; ¹³C NMR (CDCl₃, 75 MHz) δ = 18.92, 19.82, 24.49, 26.35, 29.09, 30.95, 32.90, 33.86, 39.00, 51.44, 51.68, 53.42, 170.59, 172.91, 173.68 ppm; ESI-MS von C₈₈H₁₅₂N₁₀O₂₆ (M+H⁺ = 1767.1; M+Na⁺ = 1789.4; M+2Na²⁺ = 905.7; M-H- = 1764.5); IR (ATR) ν = 3308.2, 3072.4, 2951.6, 2871.1, 1731.9 (CO₂Me), 1621.0 (Amid), 1537.2, 1434.9, 1366.6, 1195.8, 1160.6, 1027.2, 923.6, 865.5, 731.1 cm⁻¹; HRMS von C₈₈H₁₅₂N₁₀O₂₆ exakte Masse = 1765.08793 m/z (z = 2) [M+2Na]²⁺ ber. 905.53373, gef. 905.53281.

### Zweite Generation als Octacarbonsäure (71)

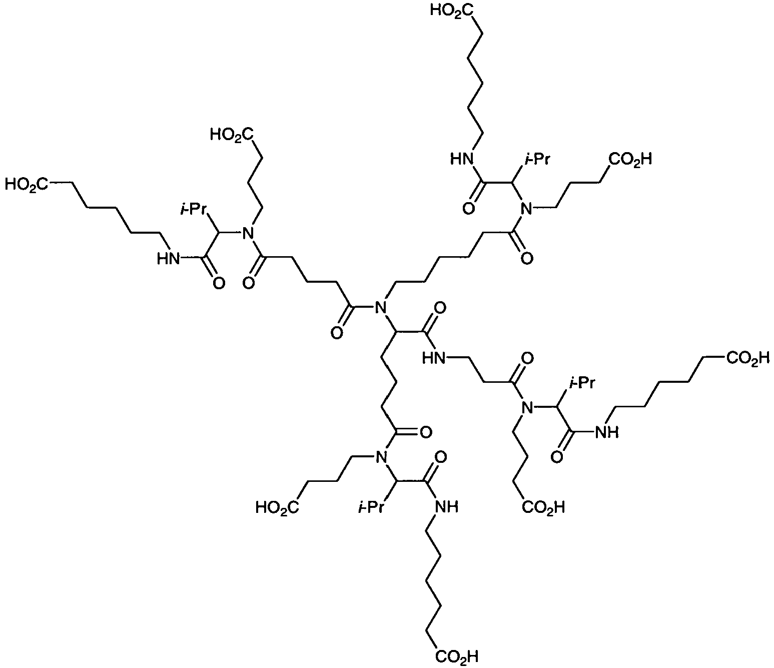

Die Verseifung der Methylestergruppen von der zweiten Generation **70** (0.36 g, 0.21 mmol) liefert das Octacarbonsäurederivat **71** als farblosen Feststoff (0.32 g, 94%). DC (Ethylacetat/MeOH/H₂O 3:2:1) *R*_{f} = 0.50; Smp. 67 - 68°C (Ethylacetat); ¹H NMR (CD₃OD, 300 MHz) δ = 0.80 - 1.05 (m, 24 H, 8 CH₃), 1.29 - 2.07 (m, 44 H, 22 CH₂), 2.22 - 2.73 (m, 30 H, 13 CH₂, 4 CH), 3.15 - 3.63 (m, 20 H, 10 CH₂), 4.49 - 4.52 (m, 5 H, 5 CH) ppm; ¹³C NMR (CD₃OD, 75 MHz) δ = 19.35, 19.86, 20.07, 24.25, 24.75, 25.69, 26.15, 27.51, 27.84, 28.19, 29.15, 29.88, 30.93, 31.84, 31.92, 32.81, 33.80, 34.13, 34.80, 40.13, 45.49, 64.66, 67.64, 171.41, 172.18, 172.29, 175.91, 176.05, 176.53, 177.08 ppm; ESI-MS von C₈₀H₁₃₆N₁₀O₂₆ (M+H⁺ = 1655.5; M+Na⁺ = 1676.0; M+2Na²⁺ = 847.3; M-H⁻ = 1653.8); IR (ATR) ν = 3326.8, 2939.2, 1712.4 (CO₂H), 1619.9 (Amid), 1552.5, 1417.8, 1373.1, 1273.5, 1197.4, 1164.9, 1088.5, 1022.4 cm⁻¹; HRMS von C₈₀H₁₃₆N₁₀O₂₆ [M+Na]⁺ ber. 1675.95249, gef. 1675.95060.

### Methylestergeschützte zweite Generation (1→2-Verzweigung) (72)

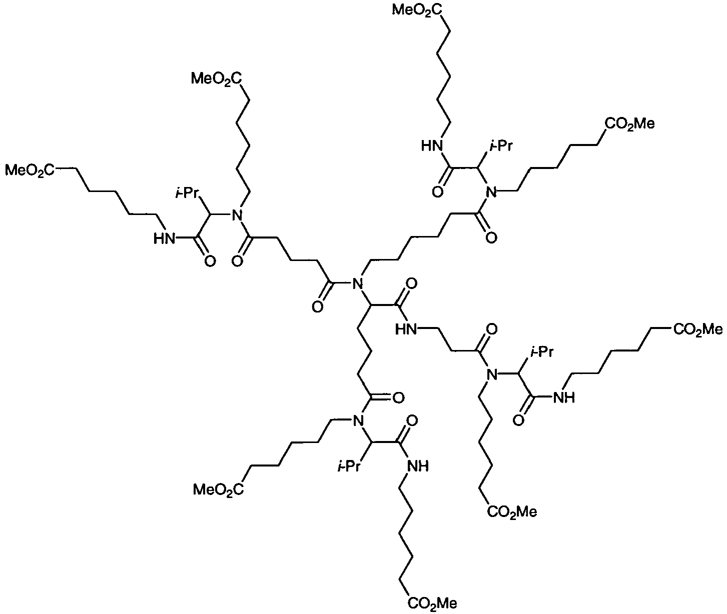

Die vierfache Ugi-4CR der Tetracarbonsäurekerneinheit **55** (0.18 g, 0.39 mmol) mit Überschüssen an Isobutyraldehyd (0.34 g, 4.68 mmol), Methyl 6-Aminohexanoat-Hydrochlorid (0.85 g, 4.68 mmol) und Methyl 6-Isocyanohexanoat (0.73 g, 4.68 mmol) liefert die methylestergeschützte zweite Generation **72** nach säulenchromatographischer Aufreinigung (Ethylacetat/MeOH 19:1) als leicht gelbliches Öl (0.49 g, 67%). DC (Ethylacetat/MeOH 19:1) *R*_{f} = 0.47; ¹H NMR (CDCl₃, 300 MHz) δ = 0.78 - 0.99 (m, 24 H, 8 CH₃), 1.21 - 1.65 (m, 58 H, 29 CH₂), 2.00 - 2.69 (m, 32 H, 14 CH₂, 4 CH), 3.12-3.42 (m, 20 H, 10 CH₂), 3.65, 3.66 (2s, 24 H, 8 CH₃), 4.08 - 4.20 (m, 5 H, 5 CH), 6.89 - 7.06 (m, 5 H, 5 NH) ppm; ¹³C NMR (CDCl₃, 75 MHz) δ = 17.55, 18.90, 19.59, 19.74, 24.32, 24.42, 24.61, 26.26, 26.38, 26.47, 26.61, 26.93, 29.01, 29.17, 29.40, 29.79, 31.11, 33.77, 38.43, 38.88, 49.24, 51.34, 51.39, 53.37, 68.38, 170.50, 173.47, 173.57 ppm; ESI-MS von C₉₆H₁₆₈N₁₀O₂₆ (M+H⁺ = 1878.5; M+Na⁺ = 1901.3; M+2Na²⁺ = 961.9; M+Cl⁻ = 1913.9); IR (ATR) ν = 3307.6, 2936.1, 2867.3, 1732.3 (CO₂Me), 1672.4, 1621.2 (Amid), 1537.2, 1434.9, 1367.3, 1196.1, 1163.0, 1100.7, 1010.2, 854.7, 731.7 cm⁻¹; HRMS von C₉₆H₁₆₈N₁₀O₂₆, exakte Masse = 1877.21313 m/z (z = 2) [M+2Na]²⁺ ber. 961.59633, gef. 961.59395.

### Zweite Generation als Octacarbonsäure (73)

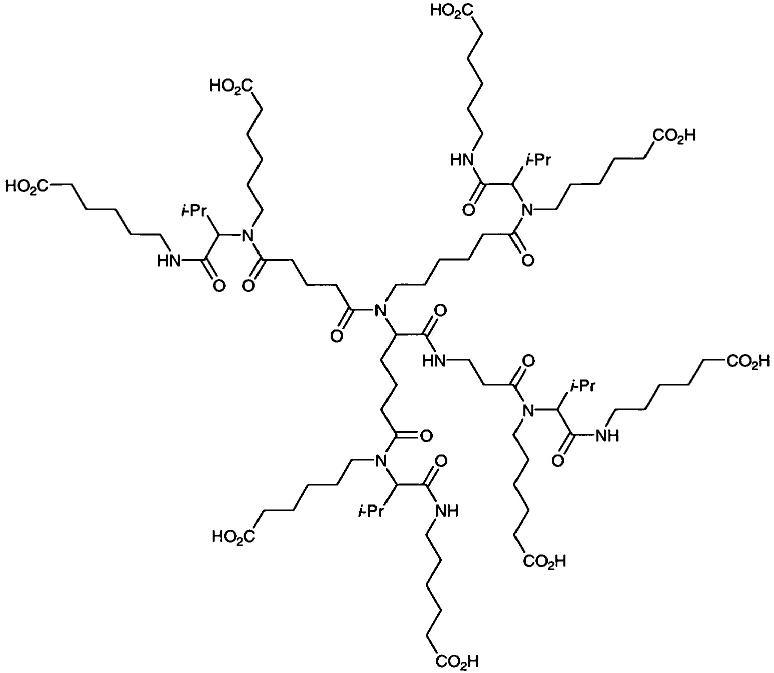

Die Verseifung der Methylestergruppen von der zweiten Generation **72** (0.38 g, 0.20 mmol) liefert das Octacarbonsäurederivat **73** als farbloses Öl (0.33 g, 94%). DC (Ethylacetat/MeOH/H₂O 3:2:1) *R*_{f} = 0.61; ¹H NMR (CD₃OD, 300 MHz) δ = 0.80 - 1.00 (m, 24 H, 8 CH₃), 1.13-1.99 (m, 60 H, 30 CH₂), 2.15-2.71 (m, 30 H, 13 CH₂, 4 CH), 3.15 - 3.82 (m, 20 H, 10 CH₂), 4.47 - 4.50 (m, 5 H, 5 CH) ppm; ¹³C NMR (CD₃OD, 75 MHz) δ = 19.30, 19.82, 20.04, 25.57, 25.67, 25.76, 26.48, 27.53, 27.82, 28.19, 28.66, 29.11, 29.88, 30.65, 33.83, 34.26, 34.79, 40.13, 46.20, 54.82, 64.94, 67.68, 172.42, 172.56, 176.02, 177.27 ppm; ESI-MS von C₈₈H₁₅₂N₁₀O₂₆ (M+H⁺ = 1766.2; M+Na⁺ = 1788.4; M+2Na²⁺ = 905.8; M-H⁻ = 1764.5); IR (ATR) ν = 3330.6, 2937.1, 2870.1, 1712.5 (CO₂H), 1615.1 (Amid), 1548.6, 1421.5, 1372.2, 1232.2, 1089.2, 1024.0, 850.3, 731.0 cm⁻¹; HRMS von C₈₈H₁₅₂N₁₀O₂₆ [M+Na]⁺ ber. 1788.07770, gef. 1788.07978.
Darstellung von Dendrimeren der dritten Generation

### Methylestergeschützte dritte Generation (80)

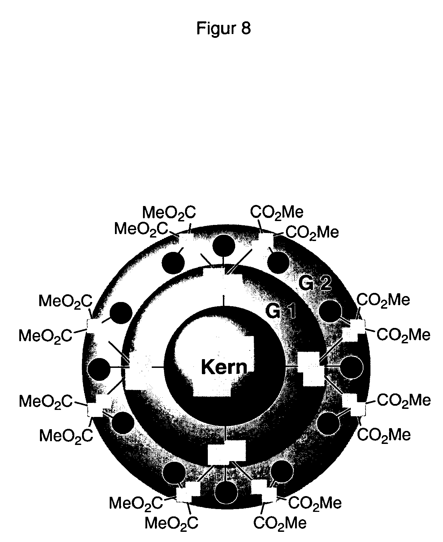

### siehe Figur 8

Die achtfache Ugi-4CR der Octacarbonsäure als zweite Generation **53** (0.40 g, 0.25 mmol) mit Überschüssen an Methyl 5-Oxopentanoat (1.31 g, 10.1 mmol), Methyl 4-Aminobutyrat-Hydrochlorid (1.55 g, 10.1 mmol) und *t*-Butylisonitril (0.84 g, 10.1 mmol) liefert die methylestergeschützte dritte Generation **80** nach säulenchromatographischer Aufreinigung (Ethylacetat/MeOH 19:1) als leicht bräunliches Öl (1.00 g, 97%). DC (Ethylacetat/MeOH 9:1) *R*_{f} = 0.79; ¹H NMR (CDCl₃, 300 MHz) δ = 1.23 - 1.35 (m, 108 H, 36 CH₃), 1.54 - 2.51 (m, 142 H, 71 CH₂), 3.28 - 3.36 (m, 26 H, 13 CH₂), 3.66 - 3.68 (m, 48 H, 16 CH₃), 4.67 - 4.88 (m, 13 H, 13 CH), 6.40 - 6.77 (m, 13 H, 13 NH) ppm; ¹³C NMR (CDCl₃, 75 MHz) δ = 20.97, 21.42, 22.14, 24.70, 25.34, 27.29, 28.45, 28.60, 30.29, 30.80, 31.85, 32.88, 32.99, 33.44, 43.62, 47.97, 50.20, 50.80, 51.38, 51.58, 53.36, 56.94, 169.86, 172.61, 172.72, 173.15, 173.38, 173.46, 173.68 ppm; ESI-MS von C₂₀₃H₃₅₀N₂₆O₅₈ (M+2Na²⁺ = 2064.8; M+3Na³⁺ = 1384.8; M+2Cl²⁻ = 2077.1); IR (ATR) ν = 3323.0, 2954.8, 2248.1, 1731.7 (CO₂Me), 1673.0 (Amid), 1624.8 (Amid), 1536.6, 1453.6, 1435.5, 1416.1, 1364.0, 1258.7, 1197.9, 1170.1, 1073.8, 914.1, 726.6 cm⁻¹; HRMS von C₂₀₃H₃₅₀N₂₆O₅₈ exakte Masse = 4080.52373 m/z (z = 3) [M+4H]³⁺ ber. 1361.51835 gef. 1361.51388; MALDITOF-MS von C₂₀₃H₃₅₀N₂₆O₅₈ [M+Na]⁺ ber. 4103.514 gef. 4102.949; [M+K]⁺ ber. 4119.487 gef. 4118.936.

### Dritte Generation als Polycarbonsäure (81)

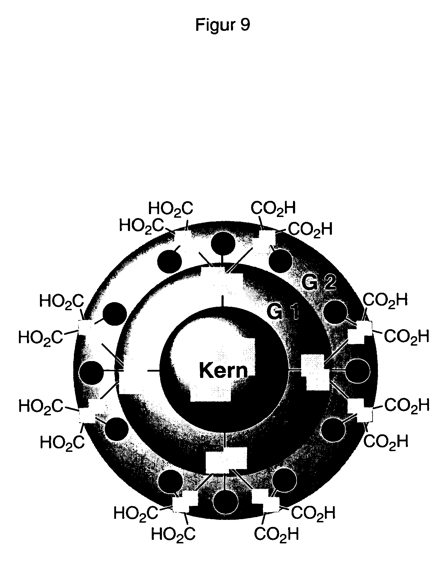

### siehe Figur 9

Die Verseifung der Methylestergruppen von der dritten Generation **80** (0.86 g, 0.21 mmol) liefert das Polycarbonsäurederivat **81** als farblosen Feststoff (0.75 g, 92%). DC (Ethylacetat/MeOH/H₂O 2:2:1) *R*_{f} = 0.85; Smp. 131 - 132°C (Ethylacetat); ¹H NMR (CD₃OD, 300 MHz) δ = 1.22 - 1.40 (m, 108 H, 36 CH₃), 1.52 - 2.56 (m, 142 H, 71 CH₂), 3.29 - 3.41 (m, 26 H, 13 CH₂), 4.70 - 4.85 (m, 13 H, 13 CH) ppm; ¹³C NMR (CD₃OD, 75 MHz) δ = 20.85, 22.73, 22.91, 23.48, 25.32, 26.36, 28.87, 28.97, 29.36, 29.89, 31.14, 31.44, 31.78, 32.72, 33.86, 34.39, 45.51, 52.14, 52.50, 58.96, 61.73, 171.92, 175.22, 176.02, 176.49, 176.60 ppm; ESI-MS von C₁₈₇H₃₁₈N₂₆O₅₈ (M+2Na²⁺ = 1952.0; M+3Na³⁺ = 1307.3; M-2H²⁻ = 1928.6); IR (ATR) ν = 3342.2, 2965.1, 1712.6 (CO₂H), 1659.7 (Amid), 1614.5 (Amid), 1538.7, 1454.8, 1417.5, 1393.5, 1365.1, 1264.2, 1218.3, 1023.9, 865.3 cm⁻¹; HRMS von C₁₈₇H₃₁₈N₂₆O₅₈ exakte Masse = 3856.27333 m/z (z = 3) [M-3H]³⁻ ber. 1284.41662 gef. 1284.42075; MALDITOF-MS von C₁₈₇H₃₁₈N₂₆O₅₈ [M+Na]⁺ ber. 3879.263 gef. 3879.021; [M+K]⁺ ber. 3895.237 gef. 3895.102.

### Methylestergeschütztes linear verlängertes Produkt der dritten Generation (82)

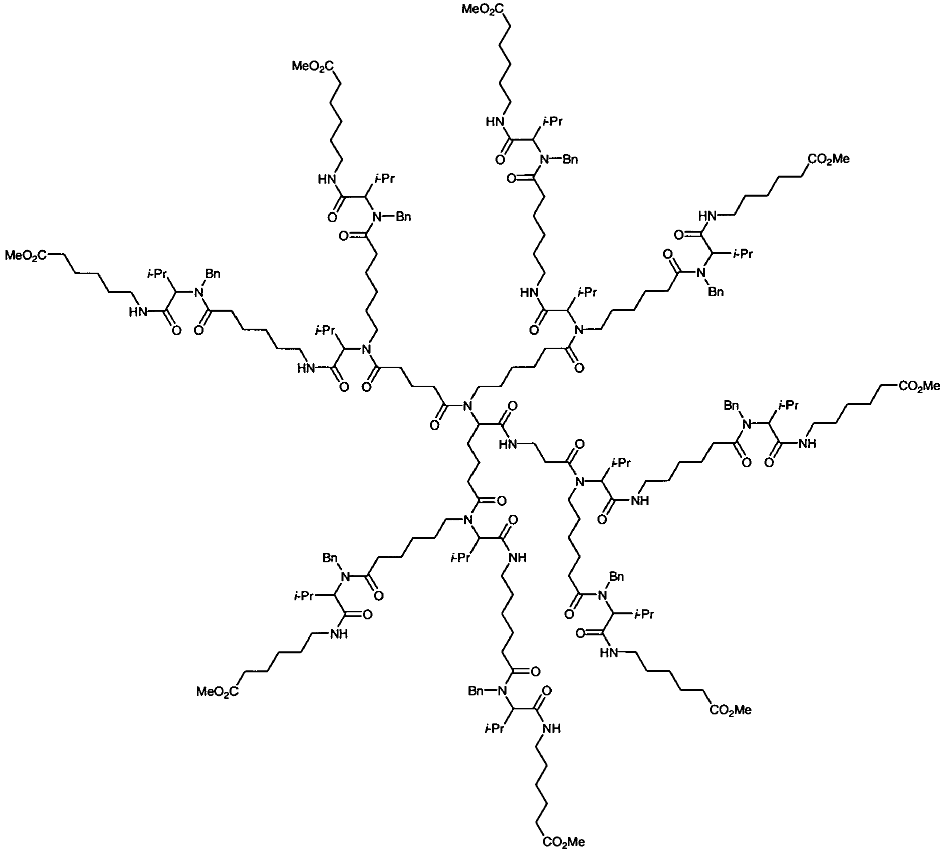

Die achtfache Ugi-4CR der Octacarbonsäure **73** (0.08 g, 0.05 mmol) mit Überschüssen an Isobutyraldehyd (0.14 g, 1.88 mmol), Benzylamin (0.20 g, 1.88 mmol) und Methyl 6-Isocyanohexanoat (0.29 g, 1.88 mmol) liefert die unverzweigte methylestergeschützte dritte Generation **82** nach säulenchromatographischer Aufreinigung (Ethylacetat/MeOH 19:1) als leicht gelbliches Öl (0.17 g, 86%). DC (Ethylacetat/MeOH 19:1) *R*_{f} = 0.52; ¹H NMR (CDCl₃, 500 MHz) δ = 0.74 - 0.94 (m, 72 H, 24 CH₃), 1.18 - 1.64 (m, 108 H, 54 CH₂), 2.13 - 2.40 (m, 54 H, 21 CH₂, 12 CH), 2.95 - 3.18 (m, 36 H, 18 CH₂), 3.65 (s, 24 H, 8 CH₃), 4.53 - 4.59 (m, 16 H, 8 CH₂), 4.75 - 4.78 (m, 13 H, 13 CH), 6.82 - 7.05 (m, 13 H, 13 NH), 7.11 - 7.34 (m, 40 H, 40 CH), ppm; ¹³C NMR (CDCl₃, 125 MHz) δ = 18.73, 19.51, 24.14, 24.29, 24.45, 24.53, 26.19, 26.48, 26.85, 28.68, 28.83, 29.09, 29.25, 33.65, 33.86, 38.83, 38.96, 48.49, 51.27, 125.87, 126.93, 127.81, 128.34, 137.45, 169.71, 173.74, 174.93, 175.12 ppm; ESI-MS von C₂₄₀H₃₇₆N₂₆O₄₂ (M+2Na²⁺ = 2171.5; M+3Na³⁺ = 1455.3); IR (ATR) ν = 3307.8, 2936.4, 2869.7, 1736.8 (CO₂Me), 1626.5 (Amid), 1541.4, 1452.3, 1368.8, 1234.2, 1203.4, 1168.0, 1102.5, 1029.7, 971.3, 854.3, 731.5, 696.8 cm⁻¹; HRMS von C₂₄₀H₃₇₆N₂₆O₄₂ exakte Masse = 4294.80855 m/z (z = 3) [M+3Na]³⁺ ber. 1454.59262 gef. 1454.58922.

### Methylestergeschützte dritte Generation (1→2-Verzweigung) (83)

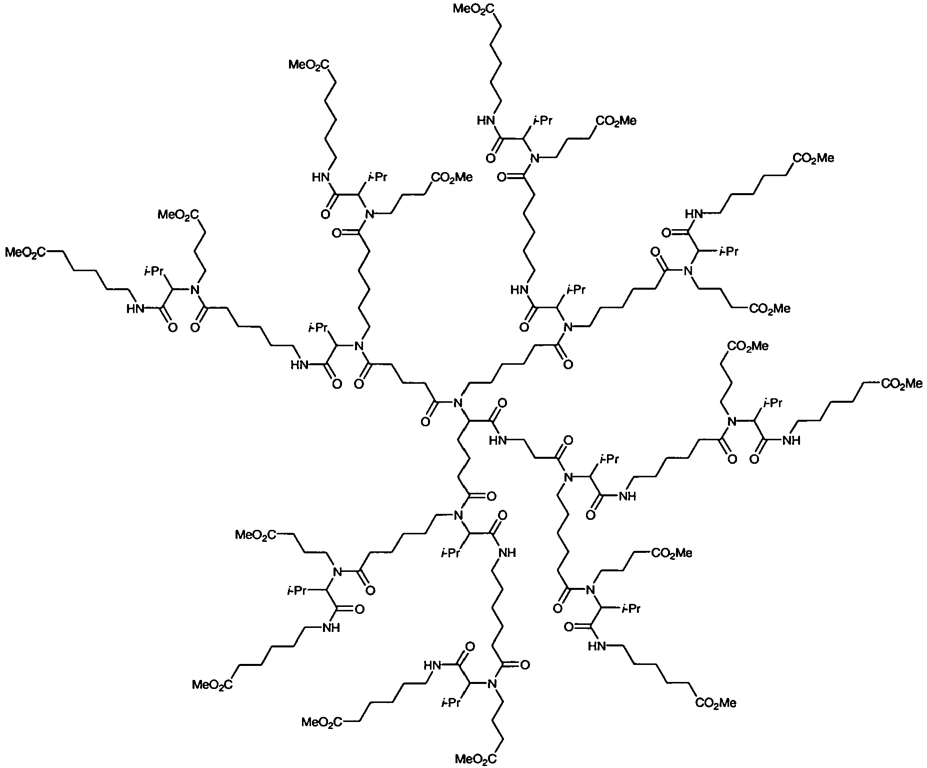

Die achtfache Ugi-4CR der Octacarbonsäure 73 (0.076 g, 0.04 mmol) mit Überschüssen an Isobutyraldehyd (0.12 g, 1.72 mmol), Methyl 4-Aminobutyrat-Hydrochlorid (0.26 g, 1.72 mmol) und Methyl 6-Isocyanohexanoat (0.27 g, 1.72 mmol) liefert die zweifach verzweigte methylestergeschützte dritte Generation **83** nach säulen-chromatographischer Aufreinigung (Ethylacetat/MeOH 19:1) als farbloses Öl (0.15 g, 81%). DC (Ethyl-acetat/MeOH 19:1) *R*_{f} = 0.38; ¹H NMR (CDCl₃, 500 MHz) δ = 0.79 - 1.01 (m, 72 H, 24 CH₃), 1.22 - 1.89 (m, 124 H, 62 CH₂), 2.25 - 2.43 (m, 70 H, 29 CH₂, 12 CH), 2.91 -3.41 (m, 52 H, 26 CH₂), 3.66, 3.67, 3.68 (3s, 48 H, 16 CH₃), 4.20 - 4.26 (m, 13 H, 13 CH), 6.83 - 7.05 (m, 13 H, 13 NH) ppm; ¹³C NMR (CDCl₃, 125 MHz) δ = 18.68, 19.40, 19.57, 24.25, 24.31, 24.55, 24.88, 26.12, 26.25, 26.40, 26.67, 28.83, 29.03, 29.21, 29.34, 29.42, 30.68, 31.10, 33.05, 33.57, 33.64, 38.78, 39.08, 51.25, 51.39, 51.49, 68.41, 170.66, 170.72, 172.91, 173.51, 173.71, 173.83, 174.31, 174.46 ppm; ESI-MS von C₂₂₄H₃₉₂N₂₆O₅₈ (M+2Na²⁺ = 2211.8; M+3Na³⁺ = 1482.9); IR (ATR) ν = 3305.9, 2935.6, 2870.4, 1732.5 (CO₂Me), 1620.5 (Amid), 1538.8, 1435.1, 1367.1, 1196.3, 1161.2, 1101.7, 1030.0, 854.8 cm⁻¹; HRMS von C₂₂₄H₃₉₂N₂₆O₅₈ exakte Masse = 4374.85238 m/z (z = 3) [M+3Na]³⁺ ber. 1481.27390 gef. 1481.27444.

### Methylestergeschützte Generation 3 (1→3-Verzweigung) (84)

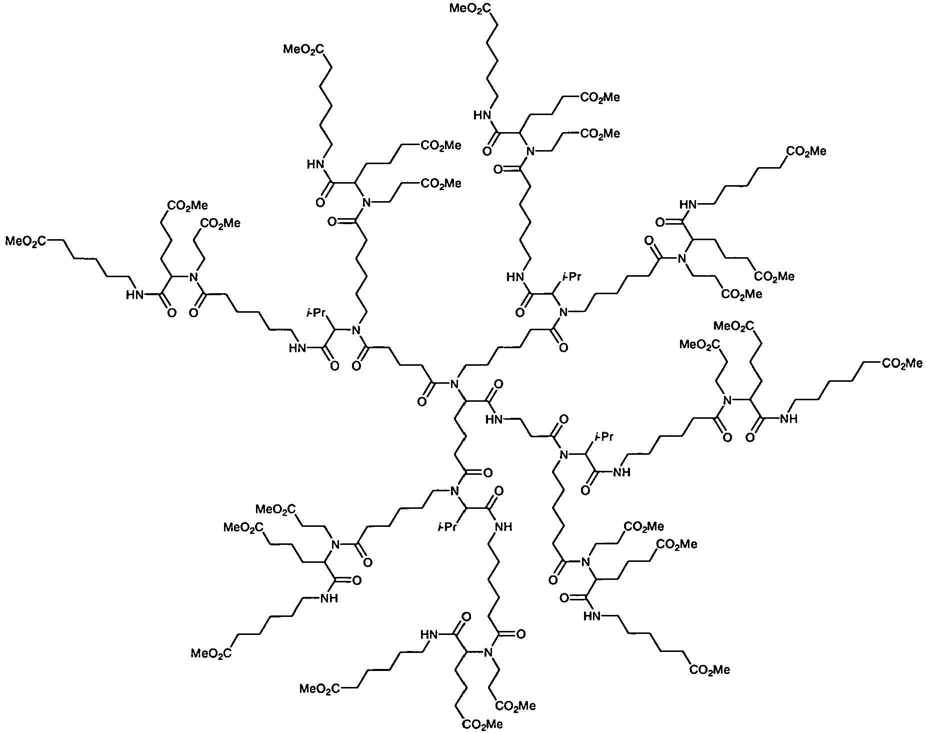

Die achtfache Ugi-4CR der Octacarbonsäure **73** (0.08 g, 0.05 mmol) mit Überschüssen an Methyl 5-Oxopentanoat (0.24 g, 1.86 mmol), Methyl 3-Aminopropionat-Hydrochlorid (0.26 g, 1.86 mmol) und Methyl 6-Isocyanohexanoat (0.29 g, 1.86 mmol) liefert die dreifach verzweigte methylestergeschützte dritte Generation **84** nach säulenchromatographischer Aufreinigung (Ethylacetat/MeOH 19:1) als leicht gelbliches Öl (0.19 g, 89%). DC (Ethylacetat/MeOH 9:1) *R*_{f} = 0.36; ¹H NMR (CDCl₃, 300 MHz) δ = 0.79 - 0.94 (m, 24 H, 8 CH₃), 1.23 - 2.00 (m, 140 H, 70 CH₂), 2.28 - 2.91 (m, 78 H, 37 CH₂, 4 CH), 3.10 -3.41 (m, 52 H, 26 CH₂), 3.66, 3.68 (2s, 72 H, 24 CH₃), 4.68 - 4.83 (m, 13 H, 13 CH), 6.80 - 6.97 (m, 13 H, 13 NH) ppm; ¹³C NMR (CDCl₃, 125 MHz) δ = 18.63, 19.49, 20.89, 21.26, 21.52, 21.59, 24.04, 24.19, 24.26, 24.44, 24.59, 26.00, 26.07, 26.14, 26.34, 26.53, 27.31, 28.76, 28.88, 28.97, 29.02, 29.22, 32.58, 32.69, 33.02, 33.10, 33.18, 33.30, 33.55, 34.01, 38.43, 38.88, 38.90, 39.25, 40.19, 43.45, 51.20, 51.31, 51.38, 51.44, 51.48, 51.56, 56.73, 169.46, 170.65, 171.13, 173.18, 173.26, 173.34, 173.45, 173.67, 174.01, 174.18 ppm; ESI-MS von C₂₃₂H₃₉₂N₂₆O₇₄ (M+2Na²⁺ = 2386.9; M+3Na³⁺ = 1600.1); IR (ATR) ν = 3307.7, 2948.1, 2865.7, 1731.3 (CO₂Me), 1625.3 (Amid), 1537.4, 1434.9, 1368.8, 1196.8, 1166.9, 1104.2, 1058.6, 1010.7, 849.2 cm⁻¹; HRMS von C₂₃₂H₃₉₂N₂₆O₇₄ exakte Masse = 4726.77102 m/z (z = 3) [M+3Na]³⁺ ber. 1598.58011 gef. 1598.59495.

### Methylestergeschützte dritte Generation (1→3-Verzweigung) (85)

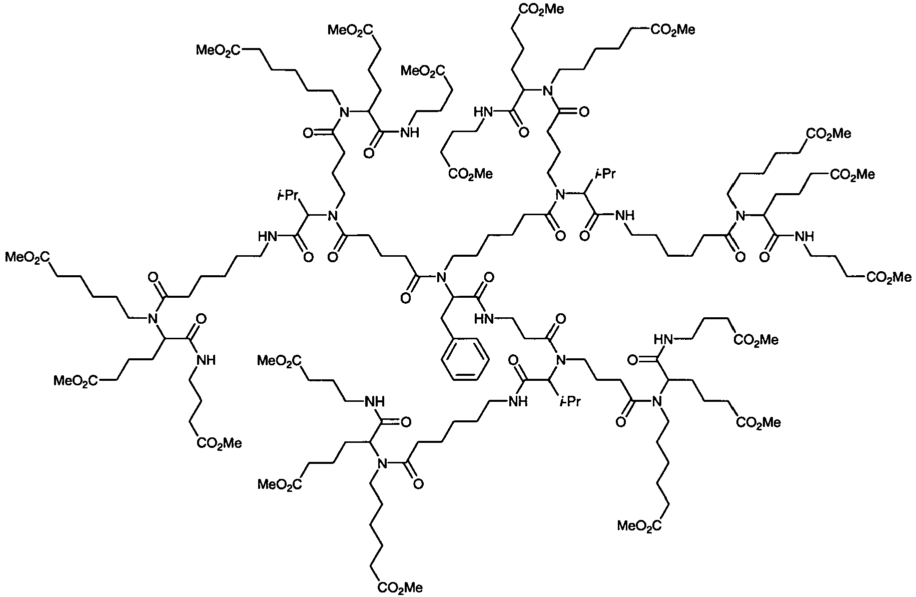

Die sechsfache Ugi-4CR der Hexacarbonsäure **65** (0.10 g, 0.07 mmol) mit Überschüssen an Methyl 5-Oxopentanoat (0.27 g, 2.10 mmol), Methyl 6-Aminohexanoat-Hydrochlorid (0.38 g, 2.10 mmol) und Methyl 4-Isocyanobutyrat (0.27 g, 2.10 mmol) liefert die dreifach verzweigte methylestergeschützte dritte Generation **85** nach säulenchromatographischer Aufreinigung (Ethylacetat/MeOH 9:1) als bräunliches Öl (0.22 g, 84%). DC (Ethylacetat/MeOH 9:1) *R*_{f} = 0.59; ¹H NMR (CDCl₃, 300 MHz) δ = 0.79 - 0.95 (m, 18 H, 6 CH₃), 1.18 - 1.94 (m, 104 H, 52 CH₂), 2.11 - 2.65 (m, 59 H, 28 CH₂, 3 CH), 2.86 - 3.57 (m, 42 H, 21 CH₂), 3.66, 3.67 (2s, 54 H, 18 CH₃), 4.75 - 4.89 (m, 10 H, 10 CH), 6.91 - 7.02 (m, 10 H, 10 NH), 7.19 - 7.24 (m, 5 H, 5 CH) ppm; ¹³C NMR (CDCl₃, 125 MHz) δ = 18.48, 19.38, 20.87, 21.21, 21.54, 24.03, 24.20, 24.24, 24.30, 24.42, 24.49, 24.58, 25.28, 25.89, 25.92, 25.94, 26.01, 26.13, 26.18, 26.24, 26.29, 26.47, 27.20, 27.33, 27.51, 28.80, 28.87, 29.08, 29.33, 29.44, 30.93, 30.95, 30.98, 31.06, 31.15, 31.21, 32.68, 32.97, 33.15, 33.23, 33.31, 33.42, 33.48, 33.53, 37.85, 38.23, 38.27, 38.36, 38.80, 40.29, 44.80, 45.39, 45.43, 48.28, 51.11, 51.12, 51.17, 51.20, 51.28, 51.38, 128.05, 128.68, 170.86, 170.90, 173.10, 173.13, 173.17, 173.21, 173.32, 173.37, 173.44, 173.59, 173.67, 173.69, 173.73, 173.95, 174.00 ppm; ESI-MS von C₁₈₂H₃₀₂N₂₀O₅₆ (M+2Na²⁺ = 1856.2; M+3Na³⁺ = 1242.2); IR (ATR) ν = 3308.1, 2949.7, 1731.4 (CO₂Me) 1626.3 (Amid), 1532.0, 1435.4, 1366.5, 1196.4, 1168.6, 1095.0, 1004.0, 883.6, 703.1 cm⁻¹; HRMS von C₁₈₂H₃₀₂N₂₀O₅₆ exakte Masse = 3664.13986 m/z (z = 3) [M+3Na]³⁺ ber. 1244.36972 gef. 1244.37251.

### Methylestergeschützte dritte Generation (1→2-Verzweigung) (86)

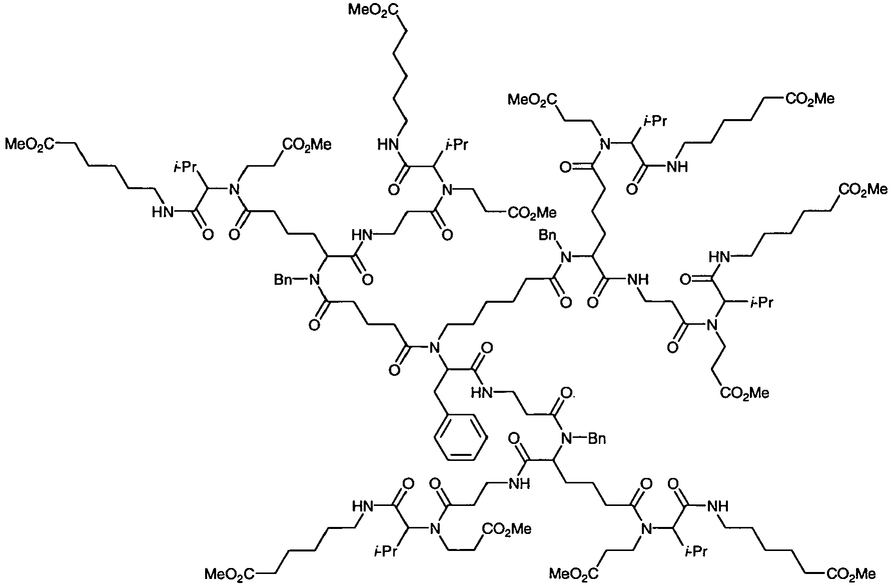

Die sechsfache Ugi-4CR der Hexacarbonsäure **67** (0.15 g, 0.11 mmol) mit Überschüssen an Isobutyraldehyd (0.23 g, 3.24 mmol), Methyl 3-Aminopropionat-Hydrochlorid (0.45 g, 3.24 mmol) und Methyl 6-Isocyanohexanoat (0.50 g, 3.24 mmol) liefert die zweifach verzweigte methylestergeschützte dritte Generation **86** nach säulenchromatographischer Aufreinigung (Ethylacetat/MeOH 9:1) als leicht gelbliches Öl (0.26 g, 74%). DC (Ethylacetat/MeOH 9:1) *R*_{f} = 0.48; ¹H NMR (CDCl₃, 300 MHz) δ = 0.76 - 0.94 (m, 36 H, 12 CH₃), 1.31 - 1.95 (m, 56 H, 28 CH₂), 2.27 - 2.68 (m, 50 H, 22 CH₂, 6 CH), 3.11 - 3.85 (m, 36 H, 18 CH₂), 3.65, 3.66 (2s, 36 H, 12 CH₃), 4.18 - 5.03 (m, 16 H, 3 CH₂, 10 CH), 6.83 - 7.04 (m, 10 H, 10 NH), 7.24 - 7.38 (m, 20 H, 20 CH) ppm; ¹³C NMR (CDCl₃, 125 MHz) δ = 18.48, 19.41, 20.59, 21.67, 21.77, 24.14, 24.29, 24.41, 26.02, 26.53, 27.61, 27.96, 28.09, 28.48, 28.71, 29.03, 29.31, 32.02, 32.16, 32.56, 33.26, 33.51, 33.90, 35.27, 38.43, 38.73, 38.78, 38.95, 40.01, 40.56, 48.07, 51.15, 51.45, 57.03, 66.41, 125.65, 125.75, 126.22, 127.02, 127.20, 127.54, 128.06, 128.23, 128.41, 128.50, 136.91, 137.08, 137.37, 168.96, 170.17, 170.28, 171.09, 172.03, 172.41, 173.05, 173.47, 173.61, 173.93, 174.35 ppm; ESI-MS von C₁₆₇H₂₆₀N₂₀O₄₄ (M+2Na²⁺ = 1649.1; M+3Na³⁺ = 1107.1); IR (ATR) ν = 3306.9, 2941.2, 2874.2, 2828.6, 1732.5 (CO₂Me), 1625.5 (Amid), 1539.3, 1435.7, 1369.1, 1198.6, 1166.2, 1104.8, 1026.0, 731.2, 699.1 cm⁻¹; HRMS von C₁₆₇H₂₆₀N₂₀O₄₄ exakte Masse = 3249.87223 m/z (z = 3) [M+3Na]³⁺ ber. 1106.28051 gef. 1106.28024.
Darstellung eines Dendrimers der vierten Generation

### Methylestergeschützte vierte Generation (90)

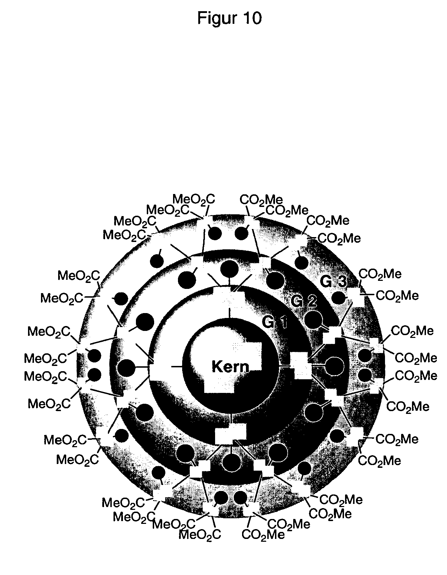

### siehe Figur 10

Die sechzehnfache Ugi-4CR der Polycarbonsäure **81** (0.30 g, 0.08 mmol) mit Überschüssen an Methyl 5-Oxopentanoat (0.81 g, 6.22 mmol), Methyl 4-Aminobutyrat-Hydrochlorid (0.96 g, 6.22 mmol) und *t*-Butylisonitril (0.52 g, 6.22 mmol) liefert die methylestergeschützte vierte Generation **90** nach säulenchromatographischer Aufreinigung (Ethylacetat/MeOH 9:1) als leicht gelbliches Öl (0.61 g, 88%). DC (Ethylacetat/MeOH 19:1) *R*_{f} = 0.80; ¹H NMR (CDCl₃, 300 MHz) δ = 1.28 - 1.34 (m, 252 H, 84 CH₃), 1.54 - 2.46 (m, 302 H, 151 CH₂), 3.27 -3.43 (m, 58 H, 29 CH₂), 3.65 - 3.67 (m, 96 H, 32 CH₃), 4.65 - 4.93 (m, 29 H, 29 CH), 6.45 - 6.81 (m, 29 H, 29 NH) ppm; ¹³C NMR (CDCl₃, 75 MHz) δ = 20.78, 21.43, 22.13, 24.76, 27.38, 28.47, 29.85, 30.84, 32.86, 33.47, 42.07, 43.63, 50.86, 51.43, 51.63, 53.38, 56.95, 169.85, 172.70, 172.86, 173.22, 173.71 ppm; ESI-MS von C₄₄₃H₇₆₆N₅₈O₁₂₂ (M+4Na⁴⁺ = 2237.7); IR (ATR) ν = 3315.9, 2958.8, 1732.4 (CO₂Me), 1673.7 (Amid), 1621.4 (Amid), 1537.1, 1453.4, 1434.8, 1391.9, 1363.8, 1259.3, 1223.0, 1198.1, 1170.3, 1072.6, 885.1 cm⁻¹; HRMS von C₄₄₃H₇₆₆N₅₈O₁₂₂ exakte Masse = 8851.55185 m/z (z = 4) [M+4Na]⁴⁺ ber. 2235.87773 exakte Masse konnte nicht identifiziert werden; MALDITOF-MS von C₄₄₃H₇₆₆N₅₈O₁₂₂ [M+Na]⁺ ber. 8874.541 gef. 8880.963; [M+K]⁺ ber. 8896.479 gef. 8890.516.

### Vierte Generation als Polycarbonsäure (91)

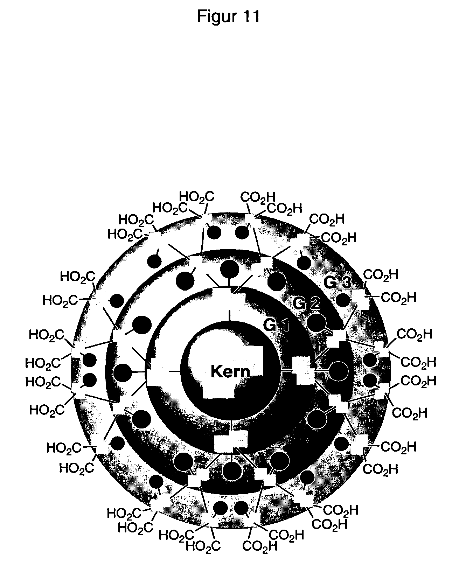

### siehe Figur 11

Die Verseifung der Methylestergruppen von der vierten Generation **90** (0.49 g, 0.06 mmol) liefert das Polycarbonsäurederivat **91** als farblosen Feststoff (0.37 g, 80%). DC (Ethylacetat/MeOH/H₂O 2:2:1) *R*_{f} = 0.91; Smp. 109 - 110°C (Ethylacetat); ¹H NMR (CD₃OD, 300 MHz) δ = 1.21 - 1.35 (m, 252 H, 84 CH₃), 1.53 - 2.56 (m, 302 H, 151 CH₂), 3.30 - 3.42 (m, 58 H, 29 CH₂), 4.68 - 4.84 (m, 29 H, 29 CH) ppm; ¹³C NMR (CD₃OD, 75 MHz) δ = 20.83, 22.77, 22.93, 23.56, 26.41, 28.95, 29.38, 29.89, 30.74, 30.92, 31.20, 31.50, 31.82, 32.21, 32.76, 33.89, 34.43, 45.55, 52.16, 52.52, 58.96, 61.78, 171.94, 174.93, 175.23, 175.94, 176.55 ppm; ESI-MS von C₄₁₁H₇₀₂N₅₈O₁₂₂ (M-4H⁴⁻ = 2101.4); IR (ATR) ν = 3335.8, 2964.9, 1716.1 (CO₂H), 1620.4 (Amid), 1541.6, 1455.3, 1422.0, 1393.7, 1365.7, 1221.5, 1071.6, 871.1, 753.2 cm⁻¹; MALDITOF-MS von C₄₁₁H₇₀₂N₅₈O₁₂₂ [M+Na]⁺ ber. 8426.041 gef. 8431.544; [M+K]⁺ ber. 8442.015 gef. 8446.877.
Oberflächenderivatisierung eines Dendrimers mit konvertierbarem Isonitril durch konvergente Synthese (zu vier Generationen Ugi-Verzweigung)

### Benzyl {9-[4-(2,2-dimethoxyethyl)-3-(formylamino)benzoyl]-10-isopropyl-13,13-dimethyl-11-oxo-3,6-dioxa-9,12-diazatetradec-1-yl}carbamat (48)

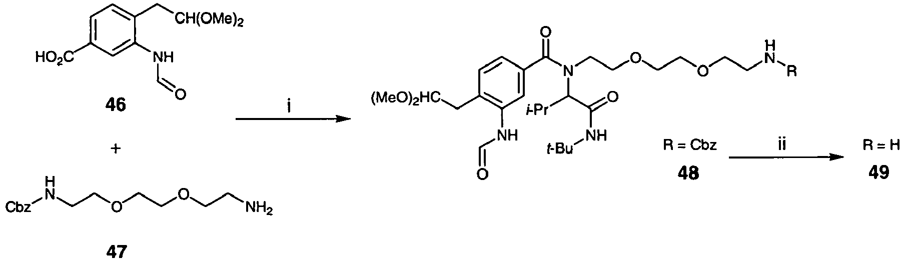

Die Ugi-4CR des Formamids **46** (1.19 g, 4.70 mmol) mit Isobutyraldehyd (0.34 g, 4.70 mmol), dem Aminderivat **47** (1.33 g, 4.70 mmol) und *t*-Butylisonitril (0.39 g, 4.70 mmol) liefert das Cbz-geschützte Aminderivat **48** nach säulenchromatographischer Aufreinigung (Ethylacetat/MeOH 19:1) als leicht gelbliches Öl (1.93 g, 68%). DC (Ethylacetat) *R*_{f} = 0.38; ¹H NMR (CDCl₃, 300 MHz, s-cis (Minder)- und s-trans (Haupt)-Isomer) δ = 0.73, 0.95 - 1.01 (t, J = 6.0 Hz, m, 6 H, 2 CH₃), 1.35, 1.39 (2s, 9 H, 3 CH₃), 2.56 - 2.77 (m, 1 H, CH), 2.93 (t, *J* = 4.9 Hz, 2 H, CH₂), 3.39, 3.41 (2s, 6 H, 2 CH₃), 3.45 - 3.96 (m, 13 H, 6 CH₂, CH), 4.42 - 4.47 (m, 1 H, CH), 5.08 (s, 2 H, CH₂), 5.67, 5.87 (br, 2s, 1 H, NH), 7.14 - 7.33 (m, 8 H, 8 CH), 7.70, 8.02 (2s, 1 H, NH), 8.38, 8.53 (s, d, J = 11.3 Hz, 1 H, CHO), 8.76 - 8.90 (m, 1 H, NH) ppm; ¹³C NMR (CDCl₃, 75 MHz, s-cis (Minder)- und s-trans (Haupt)-Isomer) δ = 18.99, 19.72, 19.80, 26.46, 28.50, 36.32, 36.75, 40.78, 41.30, 50.81, 51.83, 53.34, 54.03, 54.49, 66.36, 67.91, 68.22, 69.94, 70.14, 105.69, 106.45, 119.88, 122.26, 123.59, 127.89, 128.31, 129.01, 130.07, 131.29, 131.89, 135.73, 136.59, 156.44, 159.09, 163.05, 168.51, 169.78, 169.97, 172.75, 173.15 ppm; ESI-MS von C₃₅H₅₂N₄O₉ (M+H⁺ = 673.7; M+Na⁺ = 695.4; 2M+Na⁺ = 1367.8; M-H- = 671.8); IR (ATR) ν = 3314.9, 2963.6, 1668.0 (Amid), 1612.8 (Amid), 1573.5, 1531.2, 1454.5, 1416.9, 1364.0, 1250.3, 1114.9, 1067.6, 1026.3, 924.6, 823.8, 736.9, 697.0 cm⁻¹; HRMS von C₃₅H₅₂N₄O₉ [M+Na]⁺ ber. 695.36320 gef. 695.36354.

### N-{2-[2-(2-Aminoethoxy)ethoxy]ethyl}-N-{1-[(tert-butylamino)carbonyl]-2-methylpropyl}-4-(2,2-dimethoxyethyl)-3-(formylamino)benzamid (49)

(Formamid-Dendron Amino-URG, Vorläufer des konvertierbaren Isonitrils)

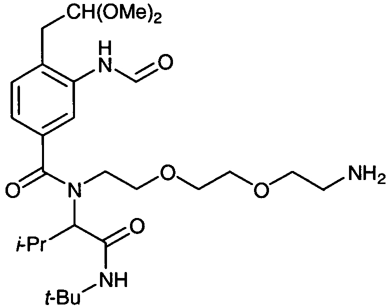

Das Cbz-geschützte Aminderivat **48** (1.93 g, 2.87 mmol) in MeOH (50 mL) wird mit einer Spatelspitze Pd(OH)₂ (20% auf Aktivkohle) versetzt. Unter kräftigem Rühren lässt man für längere Zeit unter H₂-Atmosphäre bei Raumtemperatur reagieren. Nach drei Stunden zeigt die DC-Kontrolle (Ethylacetat) eine komplette Abspaltung der Cbz-Schutzgruppe an. Der Katalysator wird daraufhin über Celite^{®} abfiltriert und die farblose Lösung im Vakuum zur Trockene eingeengt. Das Aminderivat **49** wird als leicht gelbliches Öl erhalten (1.44 g, 93%). DC (Ethylacetat/MeOH/H₂O 2:2:1) *R*_{f} = 0.38; ¹H NMR (CDCl₃, 500 MHz, s-cis (Minder)- und s-trans (Haupt)- Isomer) δ = 0.76, 0.99 - 1.01 (t, J = 6.1 Hz, m, 6 H, 2 CH₃), 1.37, 1.40 (2s, 9 H, 3 CH₃), 1.82 (br, s, 2 H, NH₂), 2.64 - 2.89 (m, 2 H, CH₂), 2.94 - 2.97(m, 1 H, CH), 3.40, 3.42 (2s, 6 H, 2 CH₃), 3.43 - 4.03 (m, 13 H, 6 CH₂, CH), 4.42 - 4.49 (m, 1 H, CH), 7.17 - 7.32(m, 3 H, 3 CH), 7.70, 8.01 (2s, 1 H, NH), 8.42, 8.52 (2s, 1 H, CHO), 8.68, 8.95 (2s, 1 H, NH) ppm; ¹³C NMR (CDCl₃, 125 MHz, s-cis (Minder)- und s-trans (Haupt)-Isomer) δ = 18.62, 18.98, 19.77, 19.84, 26.47, 26.55, 28.49, 28.76, 36.28, 36.72, 41.29, 41.53, 41.57, 50.79, 50.82, 51.81, 53.34, 53.81, 53.94, 54.01, 54.44, 67.82, 67.98, 68.13, 68.27, 69.85, 69.97, 70.05, 70.12, 73.05, 73.15, 105.64, 106.37, 106.74, 119.97, 122.21, 122.82, 123.67, 123.95, 125.39, 129.09, 129.77, 130.29, 131.28, 131.83, 131.95, 134.23, 135.23, 135.62, 135.72, 136.37, 136.64, 159.05, 159.29, 162.80, 168.49, 169.74, 169.93, 171.73, 172.72, 173.12 ppm; ESI-MS von C₂₇H₄₆N₄O₇ (M+H⁺ = 539.0; M+Na⁺ = 561.1; M-H⁻ = 537.8); IR (ATR) ν = 3309.1, 2963.7, 2933.0, 2871.5, 2830.9, 2358.8, 2338.3, 1668.1 (Amid), 1613.1 (Amid), 1573.4, 1530.9, 1454.1, 1417.1, 1388.9, 1362.7, 1307.2, 1295.7, 1270.6, 1245.2, 1224.9, 1189.6, 1168.9, 1115.1, 1067.1, 1038.6, 1002.4, 978.2, 919.0, 859.0, 823.5, 793.9, 750.5, 729.0, 665.0 cm⁻¹; HRMS von C₂₇H₄₆N₄O₇ [M+Na]⁺ ber. 539.34448 gef. 539.34383.

### Formamid-Dendron-modifizierte Dendrimeroberfläche der ersten Generation (95)

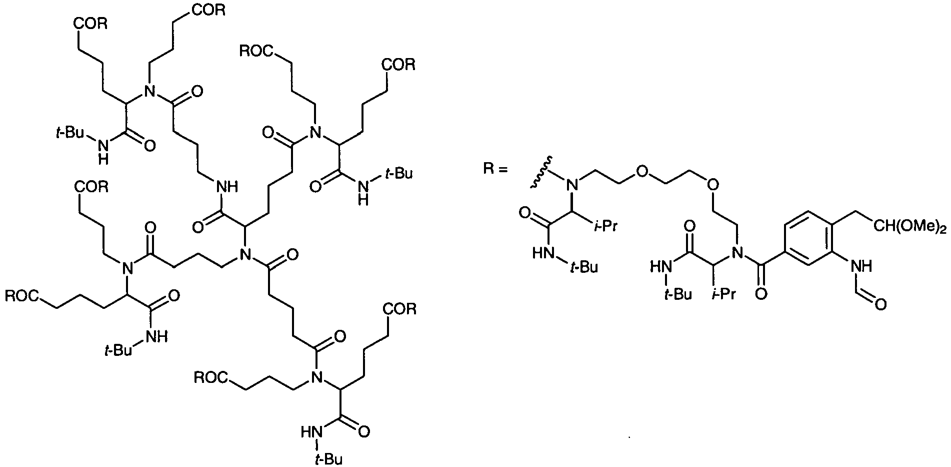

Die achtfache Ugi-4CR der Octacarbonsäure **61** (0.10 g, 0.06 mmol) mit Überschüssen an Isobutyraldehyd (0.11 g, 1.52 mmol), dem Aminderivat **49** (0.82 g, 1.52 mmol) und *t*-Butylisonitril (0.13 g, 1.52 mmol) liefert das als Formamid oberflächenderivatisierte Dendrimer **95** (vier Generationen an Ugi-Verzweigungspunkten) nach säulenchromatographischer Aufreinigung (Ethylacetat/MeOH 4:1) als farblosen Feststoff (0.32 g, 72%). DC (Ethylacetat/MeOH 4:1) *R*_{f} = 0.68; ¹H NMR (CDCl₃, 300 MHz) δ = 0.75 - 1.03 (m, 96 H, 32 CH₃), 1.24 - 1.39 (m, 180 H, 60 CH₃), 1.57 - 1.89 (m, 34 H, 17 CH₂), 2.41 - 2.63 (m, 40 H, 12 CH₂, 16 CH), 2.86 - 3.00 (m, 18 H, 9 CH₂), 3.31 - 4.50 (m, 185 H, 16 CH₃, 54 CH₂, 29 CH), 6.42 - 7.05 (m, 21 H, 21 NH), 7.16 - 7.32 (m, 24 H, 24 CH), 8.42, 8.50 (s, d, *J* = 11.4 Hz, 8 H, 8 CHO), 8.87 - 8.94 (m, 8 H, 8 NH), ppm; ¹³C NMR (CDCl₃, 125 MHz) δ = 18.63, 19.01, 19.64, 19.77, 19.85, 22.12, 25.17, 26.46, 26.55, 28.41, 28.49, 28.74, 29.52, 30.34, 33.11, 36.20, 36.64, 41.20, 43.96, 48.16, 50.74, 50.79, 51.80, 53.79, 54.00, 54.42, 55.84, 57.27, 67.74, 67.99, 68.13, 68.23, 68.81, 69.68, 69.84, 70.15, 70.29, 76.57, 77.20, 105.62, 106.32, 106.70, 119.74, 122.17, 122.84, 123.60, 124.00, 125.41, 129.20, 129.82, 130.43, 131.36, 131.96, 134.19, 135.21, 135.51, 135.72, 136.25, 136.63, 159.13, 159.30, 162.69, 168.45, 168.88, 169.69, 169.89, 170.02, 170.99, 171.66, 172.64, 173.09, 173.47, 174.33 ppm; ESI-MS von C₃₆₃H₆₁₄N₅₀O₈₂ (M+3Na³⁺ = 2353.5; M+4Na⁴⁺ = 1771.0; M+5Na⁵⁺ = 1421.3); IR (ATR) ν = 3306.4, 3076.1, 2965.4, 2937.6, 2876.3, 2830.2, 2359.9, 2340.5, 1660.9 (Amid), 1621.5 (Amid), 1572.5, 1543.8, 1455.1, 1418.3, 1391.4, 1364.9, 1313.5, 1297.6, 1273.8, 1248.4, 1223.2, 1191.1, 1170.5, 1116.4, 1070.1, 1025.0, 928.6, 820.9, 795.5, 733.5, 668.3 cm⁻¹; HRMS von C₃₆₃H₆₁₄N₅₀O₈₂ exakte Masse = 6986.54127 m/z (z = 4) [M+4Na]⁴⁺ ber. 1769.62509 exakte Masse konnte nicht identifiziert werden; MALDITOF-MS von C₃₆₃H₆₁₄N₅₀O₈₂ [M+Na]⁺ ber. 7009.531 gef. 7015.103; [M+K]⁺ ber. 7025.505 gef. 7030.729.

### Synthese von hochdiversen Janus-Dendrimeren

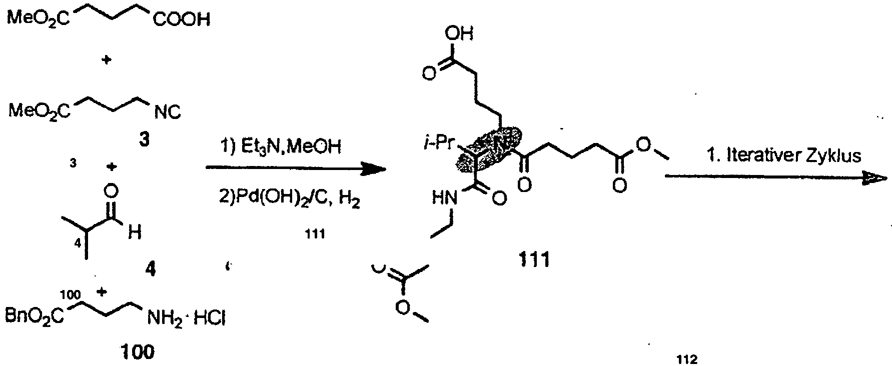

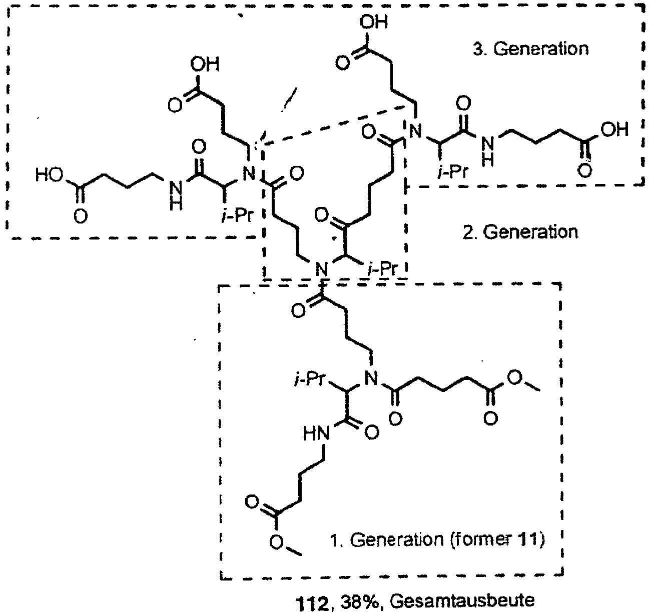

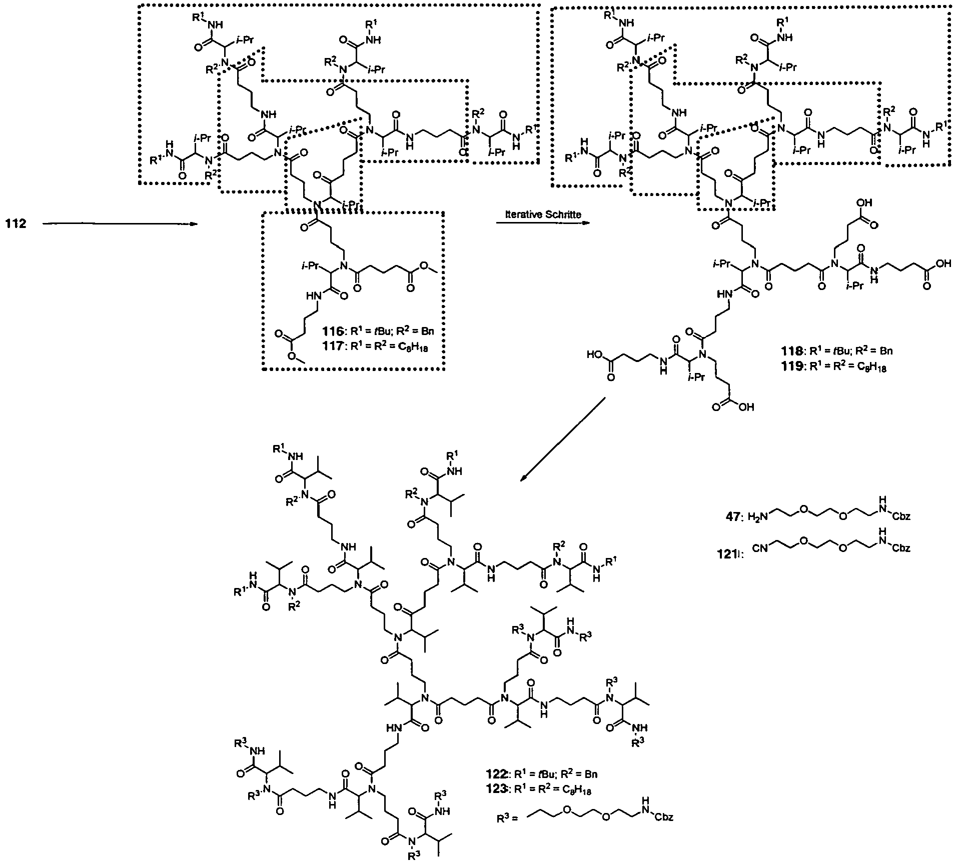

### Erste Generation (geschützt) 111a

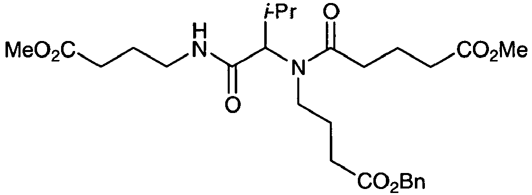

Die Ugi-4CR mit *mono*-Methylglutarat (0.95 g, 6.50 mmol), Benzyl-4-aminobutyrat Hydrochlorid **100** (1.50 g, 6.50 mmol), Isobutyraldehyd **4** (0.47 g, 6.50 mmol), Methyl-4-isocyanobutyrat (0.83 g, 6.50 mmol) and Triethylamin (0.66 g, 6.50 mmol) lieferte die geschützte 1. Generation **111a** nach säulenchromatographischer Aufreinigung (MeOH / EE, 1 : 5) als farbloses Öl (2.16 g, 64%).
ESI-MS: C₂₇H₄₀N₂O₈ (M+H⁺= 521.4).

### Erste Generation (funktionalisiert) 111

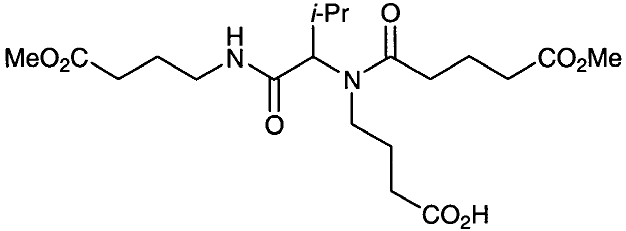

Die Spaltung der benzylestergeschützten ersten Generation **111a** (2.07 g, 3.97 mmol) lieferte 11 als farbloses Öl (1.70 g, 99%).
ESI-MS of C₂₀H₃₅N₂O₈ (M+H⁺= 431.4).

### Zweite Generation (geschützt) 112a

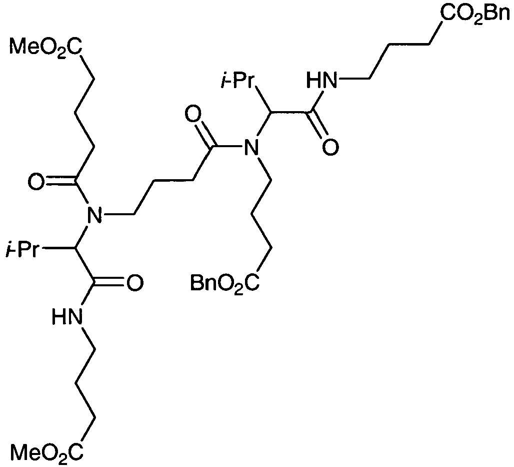

Die Ugi-4CR mit 111 (0.66 g, 1.50 mmol) mit Benzyl-4-aminobutyrat Hydrochlorid **100** (0.66 g, 1.50 mmol), Isobutyraldehyd **4** (0.11 g, 1.50 mmol), Benzyl-4-isocyanobutyrat (0.31 g, 1.50 mmol) und Triethylamin (0.15 g, 1.50 mmol) lieferte den Dibenzylester **112a** nach säulenchromatographischer Aufreinigung (EE/ Hexan, 2:1) als farbloses Öl (0.81 g, 64%).
ESI-MS: C₄₇H₆₈N₄O₁₂ (M+Na⁺= 903.6).

### Zweite Generation (funktionalisiert) 112b

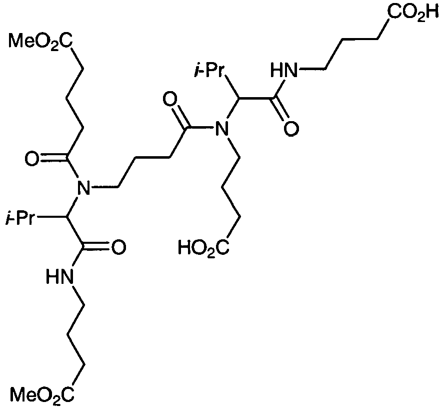

Die Spaltung des Dibenzylesters **112a** (0.69 g, 0.79 mmol) lieferte **112b** als farbloses Öl (0.55 g, 99%).
ESI-MS: C₃₃H₅₆N₄O₁₂ (M+Na⁺= 723.2).

### 3. Generation 112c

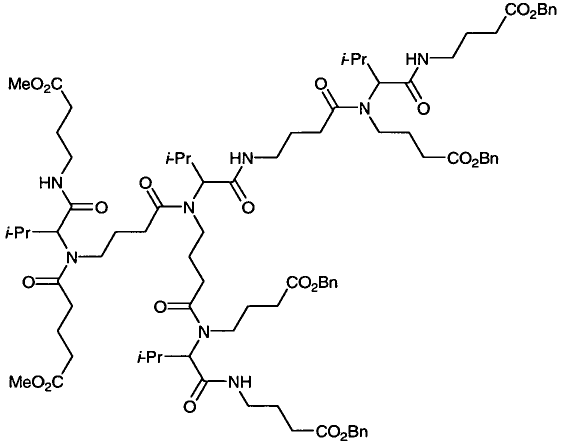

Die zweifache Ugi-4CR der Dicarbonsäure **112b** (0.46 g, 0.65 mmol) mit Benzyl-4-aminobutyrat Hydrochlorid **100** (0.46 g, 2.00 mmol), Isobutyraldehyd 4 (0.14 g, 2.00 mmol), Benzyl-4-isocyanobutyrat (0.41 g, 2.00 mmol) und Triethylamin (0.20 g, 2.00 mmol) lieferte den Tetrabenzylester **112c** nach säulenchromatographischer Aufreinigung (EE / Hexan, 6 : 1) als farbloses Öl (0.76 g, 73%).
ESI-MS: C₈₇H₁₂₄N₈O₂₀ (M+Na⁺= 1625.5).

### Tetracarbonsäure 112 (HMI 218)

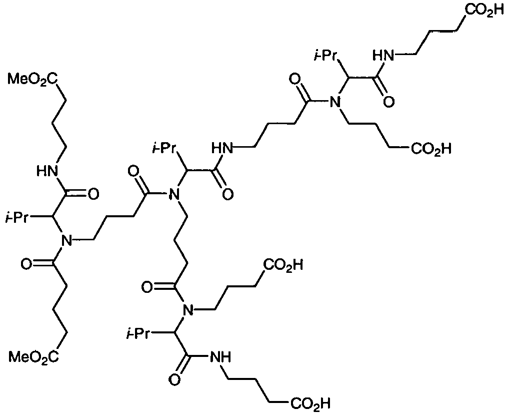

Die Spaltung des Tetrabenzylesters **112c** (0.67 g, 0.42 mmol) lieferte die Tetracarbonsäure **112** als farbloses Öl (0.48 g, 92%).
ESI-MS: C₅₉H₁₀₀N₈O₂₀ (M+Na⁺= 1264.1).

### Lipophiles Janusdendrimer (geschützt) 116

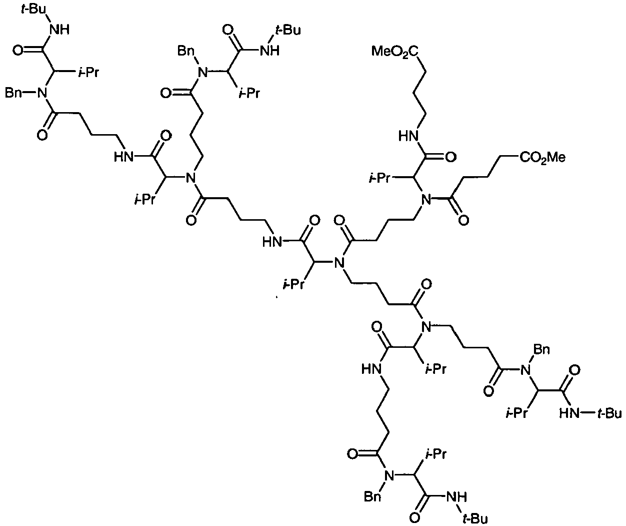

Die vierfache Ugi-4CR der Tetracarbonsäure **112** (0.37 g, 0.30 mmol) mit Benzylamin (0.64 g, 6.00 mmol), Isobutyraldehyd 4 (0.43 g, 6.00 mmol) und *t*-Butylisonitril (0.50 g, 6.00 mmol) lieferte den Dimethylester **116** nach säulenchromatographischer Aufreinigung (MeOH / EE, 1:40) als farbloses Öl (0.42 g, 63%).
ESI-MS: C₁₂₃H₁₉₆N₁₆O₂₀ (M+2Na²⁺ = 1132.2).

### Janusdendrimer (funktionalisiert) 118a

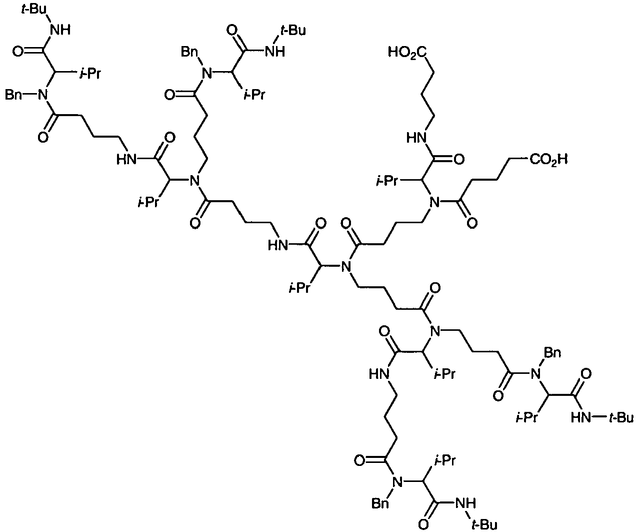

Die Verseifung des Dimethylesters **116** (0.42 g, 0.19 mmol) mit Lithiumhydroxid Monohydrat (0.04 g, 0.95 mmol) lieferte die Dicarbonsäure **118a** als farbloses Öl (0.41 g, 99%).
ESI: C₁₂₁H₁₉₂N₁₆O₂₀ (M+2Na²⁺= 1118.5).

### Janusdendrimer (geschützt) 118b

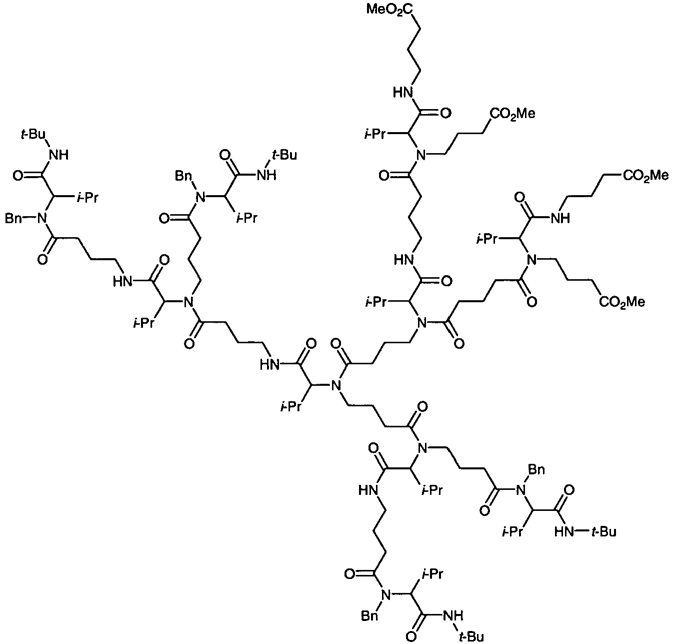

Die zweifache Ugi-4CR der Dicarbonsäure **118a** (0.41 g, 0.19 mmol) mit Methyl-4-aminobutyrat Hydrochlorid (0.09 g, 0.56 mmol), Isobutyraldehyd **4** (0.04 g, 0.56 mmol), Methyl-4-isocyanobutyrat **3** (0.07 g, 0.56 mmol) und Triethylamin (0.06 g, 0.56 mmol) lieferte den Tetramethylester **118b** nach säulenchromatographischer Aufreinigung (MeOH / EE, 1:40) als farbloses Öl (0.22 g, 71%).
ESI-MS: C₁₅₁H₂₄₄N₂₀O₂₈ (M+2Na²⁺ = 1415.9).

### Janusdendrimer (funktionalisiert) 118

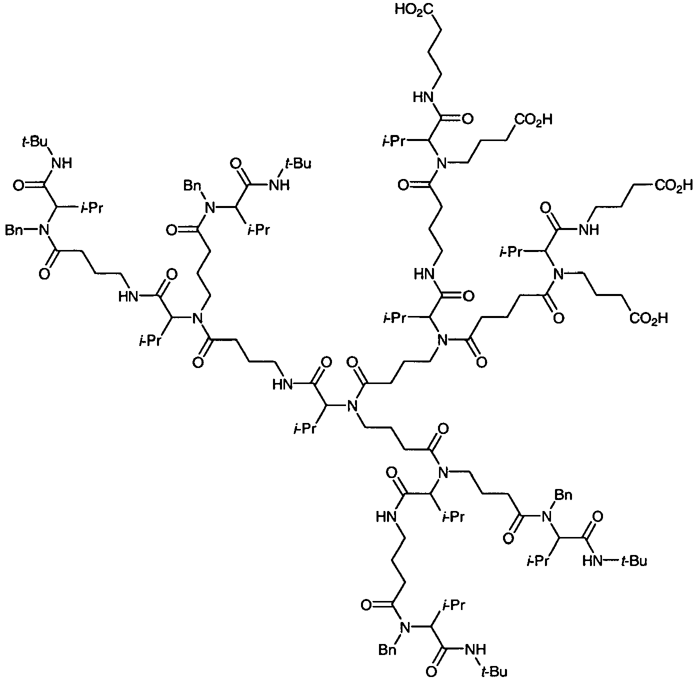

Die Verseifung des Tetramethylesters **118b** (0.1 g, 0.04 mmol) mit Lithiumhydroxid Monohydrat (0.02 g, 0.38 mmol) lieferte die Tetracarbonsäure **118** als farbloses Öl (0.09 g, 91%). ESI-MS: C₁₄₇H₂₃₆N₂₀O₂₈ (M+2Na²⁺ = 1388.5).

### Janusdendrimer (122)

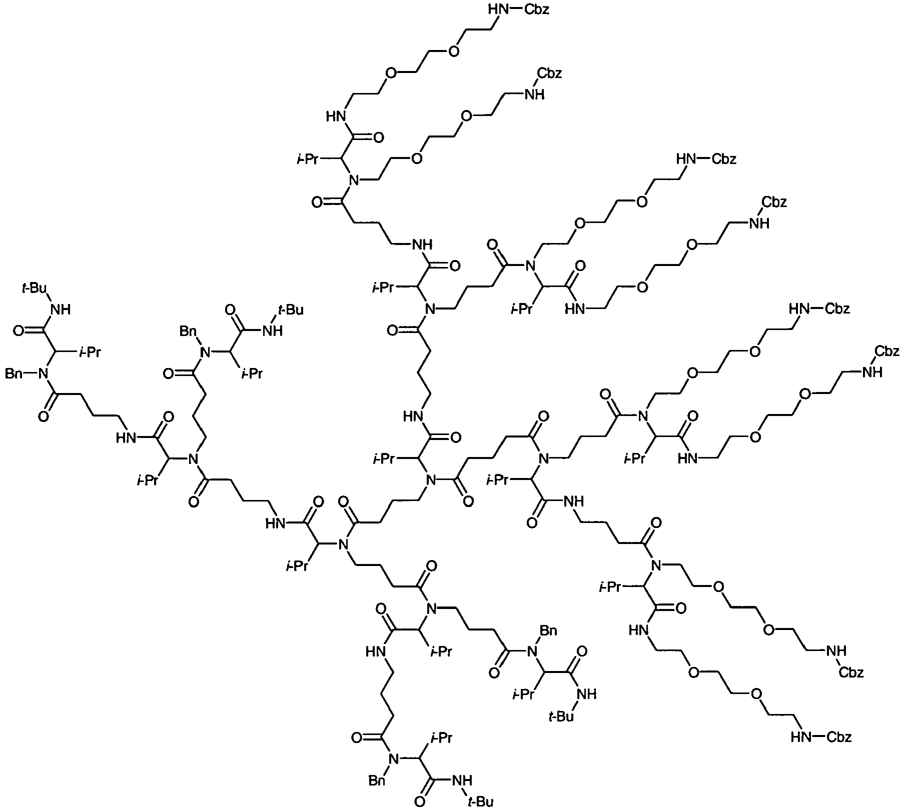

Die vierfache Ugi-4CR der Tetracarbonsäure **118** (0.09 g, 0.03 mmol) mit Benzyl-{2-[2-(2-aminoethoxy)ethoxy]ethyl}carbamat **47** (0.08 g, 0.27 mmol), Isobutyraldehyd **4** (0.02 g, 0.27 mmol) und Benzyl-{2-[2-(2-isocyanoethoxy)ethoxy]ethyl}carbamat **121** (0.08 g, 0.27 mmol) lieferte das Janusdendrimer **122** nach säulenchromatographischer Aufreinigung (MeOH / EE, 1:20) als farbloses Öl (0.12 g, 66%). ESI-MS: C₂₇₉H₄₂₈N₃₆O₆₀ (M+3Na³⁺ = 1771.5).

### Janusdendrimer (geschützt) 117

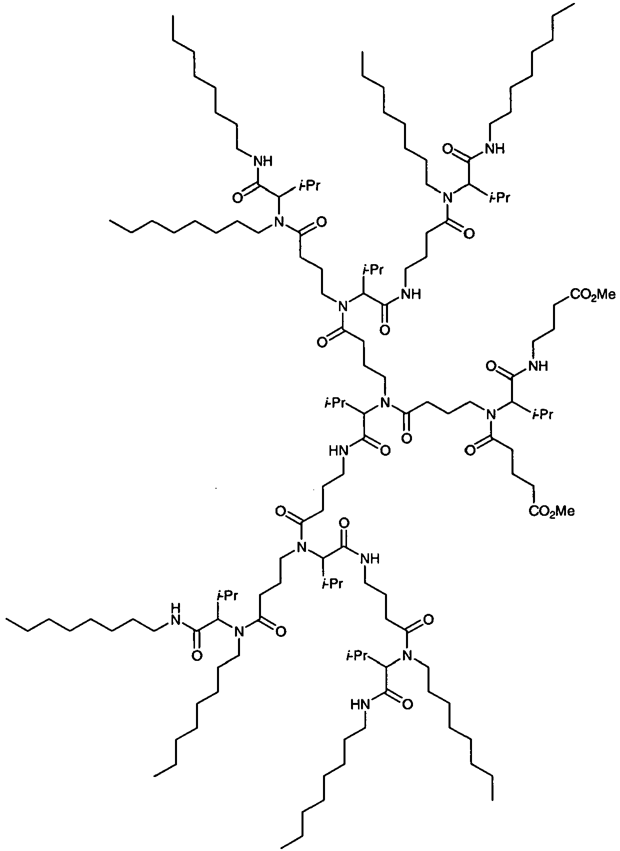

Die veirfache Ugi-4CR der Tetracarbonsäure **112** (0.20 g, 0.16 mmol) mit *n*-Octylamin (0.13 g, 0.97 mmol), Isobutyraldehyd **4** (0.07 g, 0.97 mmol) und *n*-Octylisonitril (0.14 g, 0.97 mmol) lieferte den Dimethylester **117** nach säulenchromatographischer Aufreinigung (MeOH / EE, 1:20) als farbloses Öl (0.35 g, 86%).
ESI-MS: C₁₄₃H₂₆₈N₁₆O₂₀ (M+2Na²⁺ = 1289.1).

### Janusdendrimer (funktionalisiert) 119a

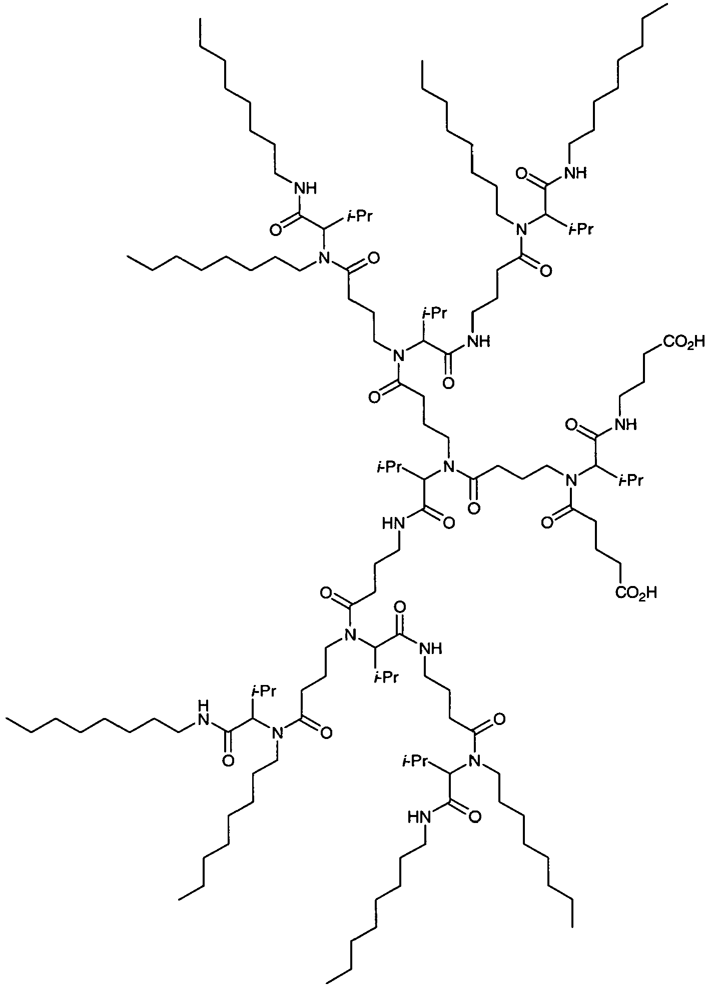

Die Verseifung des Tetramethylesters **117** (0.31 g, 0.12 mmol) mit Lithiumhydroxid Monohydrat (0.03 g, 0.62 mmol) lieferte die Dicarbonsäure **119a** als farbloses Öl (0.31 g, 98%). ESI-MS: C₁₄₁H₂₆₄N₁₆O₂₀ (M-2H²⁻ = 1251.0).

### Janusdendrimer (geschützt) 119b

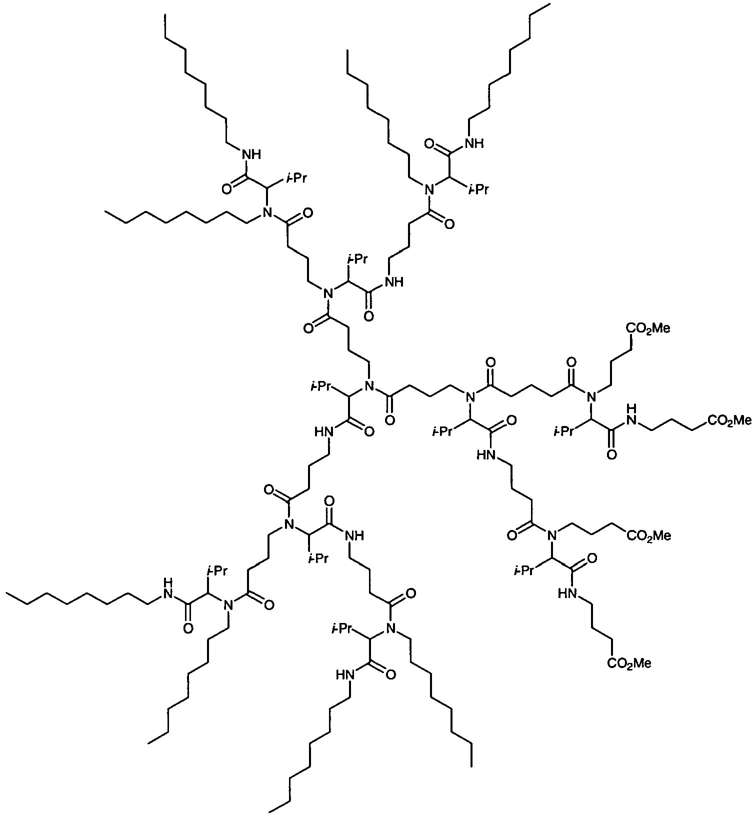

Die zweifache Ugi-4CR der Dicarbonsäure **119a** (0.26 g, 0.10 mmol) mit Methyl-4-aminobutyrat Hydrochlorid (0.05 g, 0.31 mmol), Isobutyraldehyd **4** (0.02 g, 0.31 mmol), Methyl-4-isocyanobutyrat 3 (0.04 g, 0.31 mmol) und Triethylamin (0.03 g, 0.31 mmol) lieferte den Tetramethylester **119b** nach säulenchromatographischer Aufreinigung (MeOH / EE, 1:20)als farbloses Öl (0.23 g, 70%).
ESI-MS: C₁₇₁H₃₁₆N₂₀O₂₈ (M+2Na²⁺ = 1573.4).

### Janusdendrimer (funktionalisiert) 119

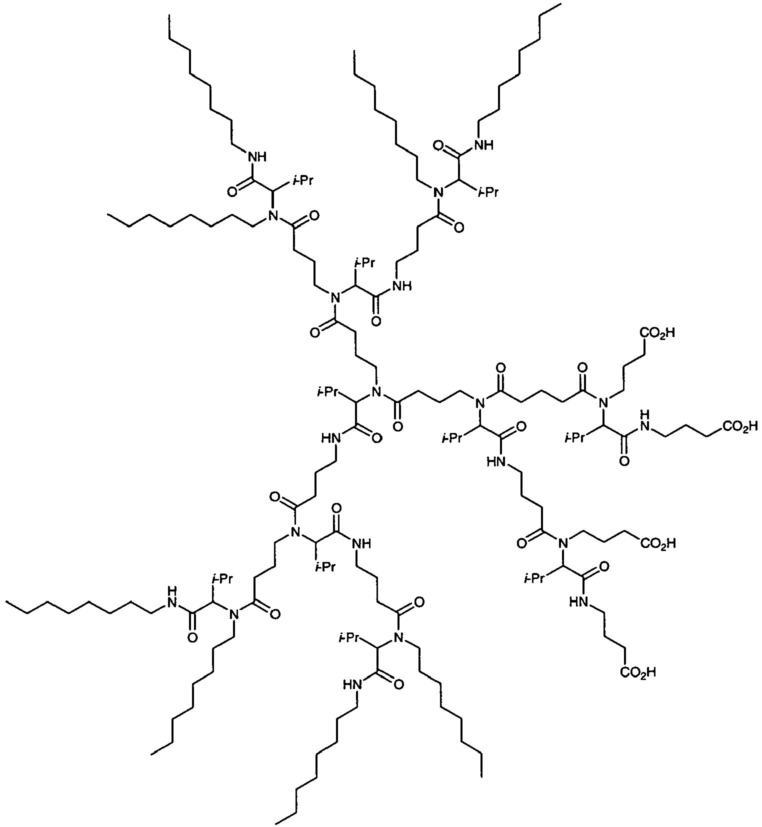

Die Verseifung des Tetramethylesters **119b** (0.22 g, 0.07 mmol) mit Lithiumhydroxid Monohydrat (0.03 g, 0.71 mmol) lieferte die Tetracarbonsäure **119** als gelbes Öl (0.21 g, 98%).
ESI-MS: C₁₆₇H₃₀₈N₂₀O₂₈ (M+2Na²⁺ = 1545.7).

### Janusdendrimer (123)

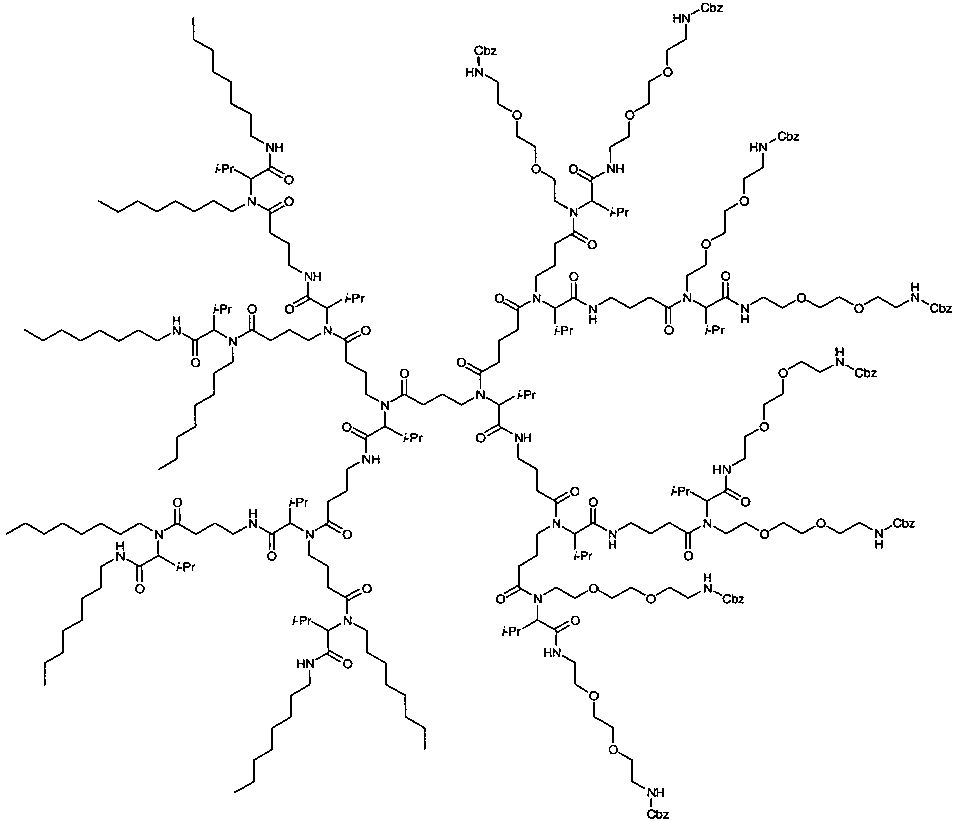

Die vierfache Ugi-4CR der Tetracarbonsäure **119** (0.10 g, 0.03 mmol) mit Benzyl-{2-[2-(2-aminoethoxy)ethoxy]ethyl}carbamat **47** (0.06 g, 0.20 mmol), Isobutyraldehyd 4 (0.01 g, 0.20 mmol) und Benzyl-{2-[2-(2-isocyanoethoxy)ethoxy]ethyl}carbamat **121** (0.06 g, 0.20 mmol) lieferte das Janusdendrimer **123** nach säulenchromatographischer Aufreinigung (MeOH / EE, 1:5) als gelbes Öl (0.15 g, 82%). ESI-MS: C₂₉₉H₅₀₀N₃₆O₆₀ (M+3Na³⁺ = 1876.0)

### Dendrimere (127), (128), (129), (130) und (131), synthetisiert durch multiple Passerini-3-Komponentenreaktion

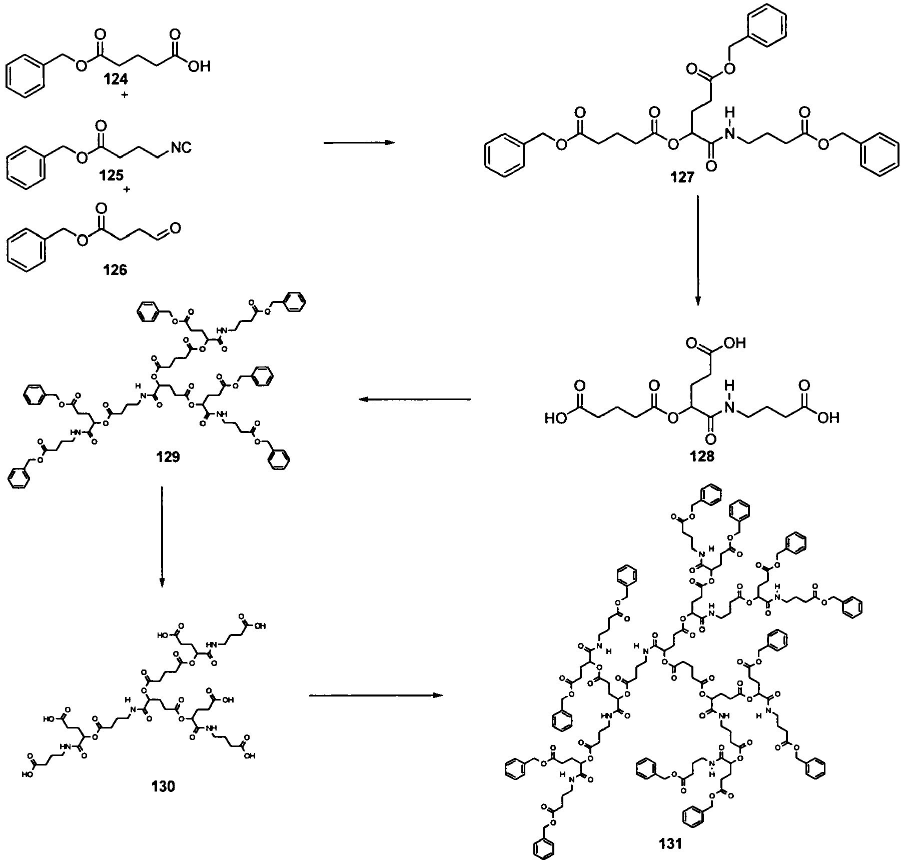

### Passerini-Dendrimer der 1. Generation - Benzylester-geschützt 127 (Benzyl 5-(benzyloxy)-1-(4-(benzyloxy)-4-oxobutylamino)-1,5-dioxopentan-2-yl glutarat)

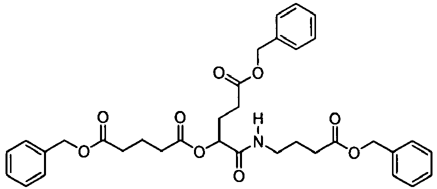

Benzyl 4-isocyanobutyrat **125** (0.41 g, 2.00 mmol), Benzyl 4-oxobutyrat **126** (0.38 g, 2.00 mmol), hergestellt gemäß Ghosez et al. (Tetrahedron, 60, 2004, 7591), und 5-(Benzyloxy)-5-Oxopentansäure **124** (0.44 g, 2.00 mmol), hergestellt gemäß Li et al. (JACS, 117, 1995, 2123), werden in CH₂Cl₂ gelöst und bei Raumtemperatur 24 Stunden gerührt. Das Lösungsmittel wird im Rotationsverdampfer entfernt und der Rückstand säulenchromatographisch aufgereinigt (Hexan: Ethylacetat, 2:1). Das Produkt wird als gelbes Öl erhalten (0.99 g, 80%). ESI-MS C₃₅H₃₉NO₉ (M+H⁺ = 618.13; M+Na⁺ = 640.3); HRMS C₃₅H₃₉NO₉ [M+Na]⁺ ber. 640.2523, gef. 640.2517.

### Passerini-Dendrimer der 1. Generation - Carbonsäure-funktionalisiert 128 (5-(Benzyloxy)-1-(4-(benzyloxy)-4-oxobutylamino)-1,5-dioxopentan-2-yl 9,22-bis(3-(benzyloxy)-3-oxopropyl)-3,8,11,16,20,23,28-heptaoxo-1,30-diphenyl-2,10,21,29-tetraoxa-7,15,24-triazatriacontan-17-yl glutarat)

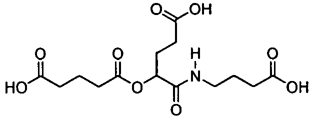

Dendrimer **127** (0.56 g, 1.60 mmol) wird in THF (40 mL) gelöst. Eine katalytische Menge an Pd(OH)₂/C (50 mg) wird zugegeben und die Reaktionlösung wird bei Raumtemperatur über Nacht gerührt. Die Reaktionslösung wird über Celite^{®} filtiriert und das Filtrat wird im Rotationsverdampfer eingeengt. Das Produkt wird als farbloses Öl in einer Ausbeute von 95% erhalten. ESI-MS C₁₄H₂₁NO₉ (M+Na⁺ = 369.8, M-H⁺ = 346.0); HRMS C₁₄H₂₁NO₉ [M+Na]⁺ ber. 370.1114; gef. 370.1109.

### Passerini-Dendrimer der 2. Generation - Benzylester-geschützt 129 (5-(Benzyloxy)-1-(4-(benzyloxy)-4-oxobutylamino)-1,5-dioxopentan-2-yl 9,22-bis(3-(benzyloxy)-3-oxopropyl)-3,8,11,16,20,23,28-heptaoxo-1,30-diphenyl-2,10,21,29-tetraoxa-7,15,24-triazatriacontan-17-yl glutarat)

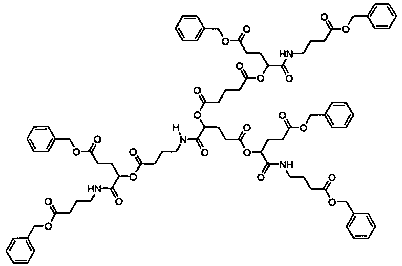

Benzyl 4-isocyanobutyrat **125** (1.16 g, 5.70 mmol), Benzyl 4-oxobutyrat **126** (1.03 g, 5.70 mmol), und **128** (0.44 g, 2.00 mmol) werden in CH₂Cl₂ gelöst und bei Raumtemperatur 24 Stunden gerührt. Das Lösungsmittel wird im Rotationsverdampfer entfernt und der Rückstand säulenchromatographisch aufgereinigt (Hexan : Ethylacetat, 1 : 1). Das Produkt wird als gelbes Öl erhalten (1.73 g, 72%). ESI-MS C₈₃H₉₆N₄O₂₄ (M+Na⁺ = 1556.2); HRMS C₈₃H₉₆N₄O₂₄ [M+Na]⁺ ber. 1555.6312, gef. 1555.6307.

### Passerini-Dendrimer der 2. Generation - Carbonsäure-funktionalisiert 130 (15-(3-(4-Carboxy-1-(3-carboxypropylamino)-1-oxobutan-2-yloxy)-3-oxopropyl)-7,23-bis(2-carboxyethyl)-6,9,14,17,21,24-hexaoxo-8,16,22-trioxa-5,13,25-triazanonacosan-1,29-dioic acid)

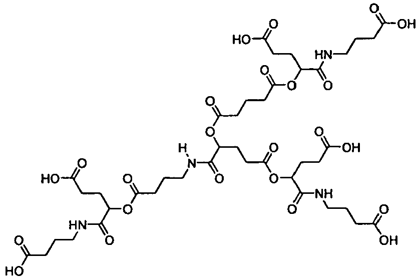

Dendrimer **129** (1.09 g, 1.10 mmol) wird in THF (40 mL) gelöst. Eine katalytische Menge an Pd(OH)₂/C (50 mg) wird zugegeben und die Reaktionlösung wird bei Raumtemperatur über Nacht gerührt. Die Reaktionslösung wird über Celite^{®} filtiriert und das Filtrat wird im Rotationsverdampfer eingeengt. Das Produkt wird als farbloses Öl in einer Ausbeute von 95% erhalten. ESI-MS C₄₁H₆₀N₄O₂₄ (M-H⁺ = 991.5); HRMS of C₄₁H₆₀N₄O₂₄ [M-H]⁺ ber. 991.3519, gef. 991.3524

### Passerini-Dendrimer der 3. Generation - Benzylester-geschützt 131 (17,30-Bis(3-(5-(benzyloxy)-1-(4-(benzyloxy)-4-oxobutylamino)-1,5-dioxopentan-2-yloxy)-3-oxopropyl)-9,38-bis(3-(benzyloxy)-3-oxopropyl)-3,8,11,16,19,24,28,31,36,39,44-undecaoxo-1,46-diphenyl-2,10,18,29,37,45-hexaoxa-7,15,23,32,40-pentaazahexatetracontan-25-yl 9,22-bis(3-(benzyloxy)-3-oxopropyl)-3,8,11,16,20,23,28-heptaoxo-1,30-diphenyl-2,10,21,29-tetraoxa-7,15,24-triazatriacontan-17-yl glutarat)

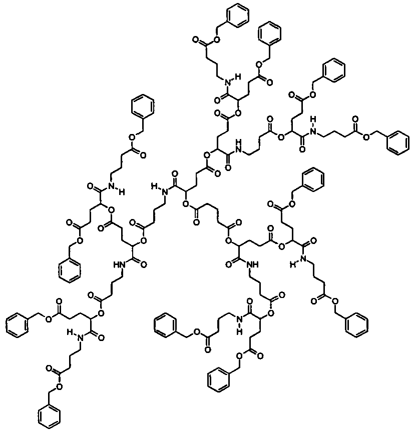

Benzyl 4-isocyanobutyrat **125** (0.73 g, 3.60 mmol), Benzyl 4-oxobutyrat 126 (0.69 g, 3.60 mmol), und **129** (0.50 g, 0.50 mmol) werden in CH₂Cl₂ gelöst und bei Raumtemperatur 24 Stunden gerührt. Das Lösungsmittel wird im Rotationsverdampfer entfernt und der Rückstand säulenchromatographisch aufgereinigt (Hexan : Ethylacetat, 1 : 1). Das Produkt wird als gelbes Öl erhalten (0.84 g, 51%). HRMS C₁₇₉H₂₁₀N₁₀O₅₄ [M+2Na]²⁺ ber. 1704.6895, gef. 1704.6788.

## Patentansprüche

1. Verfahren zur Herstellung von chimären peptid-peptoidischen Dendrimeren durch multiple iterative Mehrkomponentenreaktionen, umfassend
das Umsetzen einer polyfunktionalisierten Kerneinheit mit zwei bis sechs weiteren Komponenten, die jeweils verschiedene Funktionalitäten mit unterschiedlichen Reaktivitäten bzw. Schutzgruppen aufweisen, wobei diese Mehrkomponentenreaktion über reaktive Verzweigungen der 3 bis 7 Komponenten zu einer verzweigten Verbindung führt,
das Aktivieren der weniger reaktiven Funktionalitäten bzw. das Entschützen der Schutzgruppen, um eine funktionalisierte erste Generation eines verzweigten Dendrimeren zu generieren,
das Umsetzen der funktionalisierten ersten Generation des verzweigten Dendrimeren mit weiteren Komponenten, die jeweils verschiedene Funktionalitäten mit unterschiedlichen Reaktivitäten bzw. Schutzgruppen aufweisen, wobei diese Mehrkomponentenreaktion über reaktive Verzweigungen der 3 bis 7 Komponenten zu einer nächsten verzweigten Verbindung führt, und
das iterative Wiederholen der vorgenannten Schritte, um peptid-peptoidische Dendrimere höherer Generationen zu erhalten,
wobei die multiple iterative Mehrkomponentenreaktion eine UGI- bzw. PASSERINI-Mehrkomponentenreaktion ist, die polyfunktionalisierte Kerneinheit UGI-reaktiven Funktionalitäten aufweist und mit bifunktionellen Komponenten, die jeweils eine erste UGI-reaktive Funktionalität und eine zweite terminale UGI-reaktive Funktionalität, welche in geschützter Form vorliegt (PURG, engl. protected UGI reactive group), aufweisen, in einer UGI- bzw. PASSERINI-Mehrkomponentenreaktion umgesetzt wird, wobei sich die zweite geschützte UGI-reaktive Funktionalität nach erfolgter Umsetzung durch Entschützen der PURG's wieder zu UGI-reaktiven Gruppen aktivieren lässt.

2. Verfahren nach Anspruch 1, wobei mittels entsprechender UGI-(4CR)-Vierkomponentenreaktion ein Aminderivat, eine Carbonylkomponente, ausgewählt aus Aldehyd oder Keton, eine Carbonsäure und ein Isocyanidderivat (Isonitril) zu einem α-Aminoacylamid-Derivat umgesetzt werden.

3. Verfahren nach Anspruch 1 oder 2, wobei bifunktionelle Bausteine eingesetzt werden, die eine nicht verzweigende Einheit (NBU) umfassen, wodurch sich 1→2-Verzweigungen oder lineare Verlängerungen in jeder Generation beliebig generieren lassen.

4. Verfahren nach einem der Ansprüche 1 bis 3, wobei die polyfunktionelle Kerneinheit über eine oder mehrere UGI- bzw. PASSERINI-Mehrkomponentenreaktionen synthetisiert wird, wobei di-, tri- oder tetrafunktionalisierte, vorzugsweise tri- oder tetrafunktionalisierte Kerneinheiten oder Verzweigungsstellen entstehen.

5. Verfahren nach einem der Ansprüche 1 bis 4, wobei mittels entsprechender Schutzgruppenchemie gezielt geschützte Funktionen selektiv in jeder Generation des divergent aufgebauten Dendrimers oder in der Kerneinheit aktiviert werden, wobei die Schutzgruppen und Reaktionsbedingungen so gewählt werden, dass andere geschützte Funktionen unverändert bleiben und keine Nebenreaktionen eingehen.

6. Verfahren nach einem der Ansprüche 1 bis 5, wobei durch Verwendung konvertierbarer Isonitrile die entstehende C-terminale Amidgruppe der Ugi-Einheit in eine Carbonsäure überführt und somit zur Beteiligung an der nächsten Generation aktiviert ist.

7. Verfahren nach einem der Ansprüche 1 bis 5, worin die entstehende N-terminale Amidgruppe der Ugi-Einheit selektiv gespalten und in eine primäre Aminogruppe überführt wird und somit zur Beteiligung and der nächsten Generation aktiviert ist.

8. Verfahren nach einem der Ansprüche 2 bis 7, weiter umfassend das Bilden von stereogenen Zentren in dem α-Aminoacylamid-Derivat.

9. Verfahren nach einem der Ansprüche 1 bis 8, weiter umfassend das Aktivieren der Oberfläche der erhaltenen peptid-peptoidischen Dendrimere höherer Generation in einer weiteren UGI-4CR, wobei peptid-peptoidische Dendrimere generiert werden, die unterschiedliche Funktionalitäten an ihrer Oberfläche aufweisen.

## Claims

1. A process for preparing chimeric peptide-peptoidic dendrimers by multiple iterative multicomponent reactions, comprising
reacting a polyfunctionalized core unit with two to six further components which each have different functionalities with different reactivities or protective groups, wherein this multicomponent reaction leads to a branched compound by reactively branching the 3 to 7 components,
activating the less reactive functionalities and/or deprotecting the protective groups to generate a functionalized first generation of a branched dendrimer,
reacting the functionalized first generation of the branched dendrimer with further components which each have different functionalities with different reactivities or protective groups, wherein this multicomponent reaction leads to a subsequent branched compound by reactively branching the 3 to 7 components, and
iteratively repeating the aforementioned steps to obtain peptide-peptoidic dendrimers of higher generations,
wherein the multiple iterative multicomponent reaction is a UGI or PASSERINI multicomponent reaction, the polyfunctionalized core unit has UGI-reactive functionalities and is reacted with bifunctional components which each have a first UGI-reactive functionality and a second terminal UGI-reactive functionality which is in protected form (PURG, protected UGI-reactive group) in a UGI or PASSERINI multicomponent reaction, wherein the second protected UGI-reactive functionality is activable after the reaction by deprotecting the PURGs back to UGI-reactive groups.

2. The process as claimed in claim 1, wherein an appropriate UGI (4CR) four-component reaction is used to react an amine derivative, a carbonyl component selected from aldehyde or ketone, a carboxylic acid and an isocyanide derivative (isonitrile) to form an α-aminoacylamide derivative.

3. The process as claimed in claim 1 or 2, wherein bifunctional synthons used comprise a nonbranching unit (NBU) whereby 1→2 branching or linear prolongation is freely generatable in every generation.

4. The process as claimed in any of claims 1 to 3, wherein the polyfunctional core unit is synthesized via one or more UGI or PASSERINI multicomponent reactions to produce di-, tri- or tetrafunctionalized, preferably tri- or tetrafunctionalized, core units or branching sites.

5. The process as claimed in any of claims 1 to 4, wherein appropriate protective-group chemistry is used to specifically activate protected functions selectively in each generation of the divergently constructed dendrimer or in the core unit, wherein the protective groups and reaction conditions are chosen such that other protected functions remain unchanged and do not undergo any secondary reactions.

6. The process as claimed in any of claims 1 to 5, wherein convertible isonitriles are used such that the resulting C-terminal amide group of the Ugi unit is converted into a carboxylic acid and thus is in an activated state for participation in relation to the next generation.

7. The process as claimed in any of claims 1 to 5, wherein the resulting N-terminal amide group of the Ugi unit is selectively cleaved and converted into a primary amino group and thus is in an activated state for participation in relation to the next generation.

8. The process as claimed in any of claims 2 to 7, further comprising forming stereogenic sites in the α-aminoacylamide derivative.

9. The process as claimed in any of claims 1 to 8, further comprising activating the surface of the resultant higher generation peptide-peptoidic dendrimers in a further UGI-4CR, wherein peptide-peptoidic dendrimers generated have different functionalities at their surface.

## Revendications

1. Procédé de préparation de dendrimères peptido-peptoïdiques chimériques par plusieurs réactions multicomposants itératives comprenant
la réaction d'une unité de noyau polyfonctionnelle avec deux à six autres composants, qui présentent respectivement diverses fonctions avec différents degrés de réactivité ou groupements protecteurs, cette réaction multicomposants conduisant par le biais de ramifications réactives des 3 à 7 composants à un composé ramifié,
l'activation des fonctions moins réactives ou la déprotection des groupements protecteurs, pour générer une première génération fonctionnelle d'un dendrimère ramifié,
la réaction de la première génération fonctionnelle du dendrimère ramifié avec d'autres composants, qui présentent respectivement diverses fonctions avec différents degrés de réactivité ou groupements protecteurs, cette réaction multicomposants conduisant par le biais de ramifications réactives des 3 à 7 composants à un prochain composé ramifié, et
la répétition itérative de l'étape susmentionnée, pour obtenir des dendrimères peptido-peptoïdiques de génération supérieure,
la réaction multicomposants itérative étant une réaction multicomposants de UGI ou de PASSERINI, l'unité de noyau polyfonctionnelle présentant des fonctions réactives de UGI et étant transformée par des composants bifonctionnels, qui présentent respectivement une première fonction réactive de UGI et une deuxième fonction terminale réactive de UGI, laquelle existe sous forme protégée (*Protected UGI Reactive Group -* PURG), en une réaction multicomposants de UGI ou de PASSERINI, la deuxième fonction réactive protégée de UGI étant activée, après l'achèvement de la réaction, par la déprotection du PURG à nouveau en groupements réactifs de UGI.

2. Procédé selon la revendication 1, au moyen d'une réaction à quatre composants UGI-(4CR) correspondante un dérivé d'amine, un composant carbonyle, choisi parmi les aldéhydes ou cétones, un acide carboxylique et un dérivé d'isocyanate (isonitrile) étant transformés en un dérivé d'α-aminoacyle amide.

3. Procédé selon la revendication 1 ou 2, des composants bifonctionnels. étant utilisés, qui comprennent une unité non ramifiée (NBU), permettant de générer arbitrairement 1 à 2 ramifications ou extensions linéaires à chaque génération.

4. Procédé selon l'une quelconque des revendications 1 à 3, l'unité de noyau polyfonctionnelle étant synthétisée par le biais d'une ou plusieurs réactions multicomposants de UGI ou de PASSERINI, des unités de noyau di-, tri- ou tétrafonctionnelles, de préférence tri- ou tétrafonctionnelles ou des points de ramifications apparaissant.

5. Procédé selon l'une quelconque des revendications 1 à 4, au moyen de groupements protecteurs correspondants des fonctions protégées et ciblées étant activées sélectivement à chaque génération de dendrimère constitué de manière divergente ou dans l'unité de noyau, les groupements protecteurs et conditions réactionnelles étant choisis de telle sorte que d'autres fonctions protégées restent inchangées et qu'aucune réaction secondaire ne se produit.

6. Procédé selon l'une quelconque des revendications 1 à 5, par l'utilisation d'isonitrile convertible le groupement amide C-terminal résultant de l'unité de Ugi étant converti en un acide carboxylique et étant donc activé pour participer à la prochaine génération.

7. Procédé selon l'une quelconque des revendications 1 à 5, dans lequel le groupement amide N-terminal résultant de l'unité de Ugi est divisé sélectivement et transformé en un groupement amine primaire et est donc activé pour participer à la prochaine génération.

8. Procédé selon l'une quelconque des revendications 2 à 7, comprenant en outre la formation de centres stéréogènes dans le dérivé d'α-aminoacyle amide.

9. Procédé selon l'une quelconque des revendications 1 à 8, comprenant en outre l'activation de la surface des dendrimères peptido-peptoïdiques obtenus de génération supérieure dans une autre Ugi-4CR, des dendrimères peptido-peptoïdiques qui présentent différentes fonctions à leur surface étant générés.
